# EUROPEAN PATENT APPLICATION

(11) **EP 1 008 589 A1**
(43) Date of publication of application: **14.06.2000**
(21) Application number: 00106172.0
(22) Date of filing: 14.05.1993
(51) Int. Cl.: C07D 233/86, A01N 43/50, A01N 43/52, C07D 233/72, C07D 233/70, C07D 235/02, C07D 409/04, C07D 495/10, C07D 491/107, C07D 405/04, C07D 401/04, C07F 9/572

(54) **Fungicidal imidazolinones**

(30) Priority: 22.05.1992 US 887528
(62) Divisional of application: 93911196.9
(71) Applicant: E.I. DU PONT DE NEMOURS & COMPANY INCORPORATED, Wilmington Delaware 19898 (US)
(72) Inventor: The designation of the inventor has not yet been filed
(74) Representative: Beacham, Annabel Rose

(57) **Abstract**

Imidazolinone compounds of Formula I 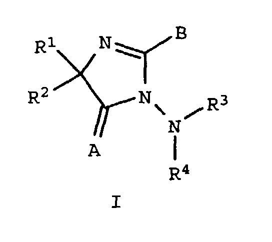 wherein:
- A: is O; S or N-J;
- J: is R¹⁵; C(=O)R¹⁶; C(=O)OR¹⁷; C(=O)SR¹⁸; C(=O)NR¹⁹R²⁰; P(=O)(C₁-C₄ alkyl)₂; or OG;
- G: is H; C₁-C₆ alkyl; benzyl optinally substituted with R³⁴ on the phenyl ring; C(=O)(C₁-C₄ alkyl); C(=O)(C₁-C₄ alkoxy); or C(=O)NHR³⁶;
- B: is H; halogen; cyano; NC; S=C=N; O=C=N; nitro; R²¹; OR²⁹; NR⁴⁹R⁶³; N=CR⁴⁵R⁴⁶; SR⁴⁷; S(O)ₙR⁴⁸; or SO₂NR⁴⁹R⁶⁰;
- n: is 1 or 2;
- R¹, R², R³ and R⁴: are various groups
are disclosed with compositions containing them and methods of their use.

## Description

This invention relates to particular imidazolinone compounds useful as fungicides, agriculturally suitable compositions containing such compounds, and methods of use of such compounds or compositions as fungicides in crop plants.

WO90/12791 is drawn to the use of fungicidal compounds of Formula i 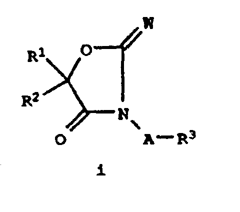 wherein:
- A: is O or NR⁴; and
- W: is O or S.

WO90/12791 also relates to processes for the preparation of compounds of Formula i and to certain novel compounds.

The compounds of WO90/12791 are distinct from those of the present invention in that oxygen is incorporated into the central heterocyclic ring of compounds of Formula i and the bonds forming this five-membered ring are all single bonds.

### SUMMARY OF THE INVENTION

This invention comprises compounds of Formula I including all geometric and stereoisomers thereof, agricultural compositions containing one or more such compounds, and methods of use of such compounds or compositions as fungicides.

The compounds of the present invention have the following structure: 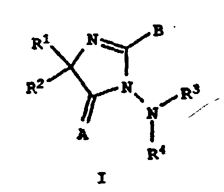 wherein:
- A: is O; S or N-J;
- J: is R¹⁵; C(=O)R¹⁶; C(=O)OR¹⁷; C(=O)SR¹⁸; C(=O)NR¹⁹R²⁰; P(=O) (C₁-C₄ alkyl)₂; or OG;
- G: is H; C₁-C₆ alkyl; benzyl optionally substituted with R³⁴ on the phenyl ring; C(=O)(C₁-C₄ alkyl); C(=O) (C₁-C₄ alkoxy); or C(=O)NHR³⁶;
- B: is H; halogen; cyano; NC; S=C=N; O=C=N; nitro; R²¹; OR²⁹; NR⁴⁹R⁶³; N=CR⁴⁵R⁴⁶; SR⁴⁷; S(O)ₙR⁴⁸; or SO₂NR⁴⁹R⁶⁰;
- n: is 1 or 2;
- R¹: is C₁-C₄ alkyl; C₁-C₄ haloalkyl; C₃-C₆ cycloalkyl; C₂-C₄ alkenyl; C₂-C₄ alkoxycarbonyl; or phenylmethyl optionally substituted with R⁶ on the phenyl ring and with R⁸ on the benzylic carbon;
- R²: is C₁-C₂₀ alkyl optionally substituted with R²²; C₂-C₂₀ alkoxyalkyl optionally substituted with R³⁵; C₂-C₂₀ alkenyl optionally substituted with R⁴²; C₂-C₂₀ alkynyl optionally substituted with R⁴¹; (CH₂CH₂OCH₂CH₂)CH-; (CH₂CH₂SCH₂CH₂)CH-; (CH₂CH₂SO₂CH₂CH₂)CH-; C₅-C₇ cycloalkyl; C₅-C₇ cycloalkenyl; phenyl optionally substituted with R⁵ and R⁷; 2-naphthalenyl; thienyl optionally substituted with R⁵ and R⁷; furyl optionally substituted with R⁷; or pyridyl optionally substituted with R⁵ and R⁷; or
- R¹ and R²: can be taken together to form a structure selected from the group consisting of -CH₂(CH₂)₂CH₂-, -CH₂(CH₂)₃CH₂-, -CH₂(CH₂)₄CH₂-, -CH₂CH₂OCH₂CH₂-, -CH₂CH₂SCH₂CH₂-, 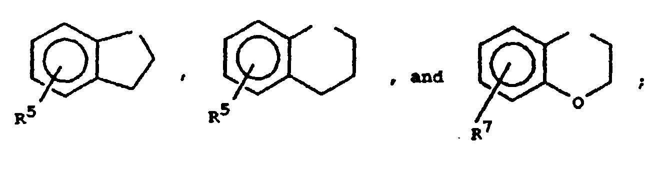
- R³: is phenyl, pyridyl, or pyrimidinyl each optionally substituted with R¹⁰; or phenylmethyl;
- R⁴: is H or methyl;
- R⁵: is halogen: nitro; cyano; C₁-C₆ alkyl; C₅-C₆ cycloalkyl; C₁-C₆ haloalkyl; C₁-C₆ alkylthio; C₁-C₆ haloalkylthio; C₁-C₆ alkoxy; C₁-C₆ haloalkoxy; C₅-C₆ cycloalkyloxy; C₂₋C₆ alkoxyalkyl; C₂-C₆ alkoxyalkoxy; C₃-C₆ alkenyl; C₃-C₆ haloalkenyl; C₃-C₆ alkenyloxy; C₃-C₆ alkynyl; C₃-C₆ haloalkynyl; C₃-C₆ alkynyloxy; C₁-C₆ alkylsulfonyl; C₁-C₆ haloalkylsulfonyl; phenyl or phenylthio each optionally substituted with R²⁴; phenylmethyl, phenoxymethyl, phenethyl, or styryl each optionally substituted with R²⁴ on the phenyl ring; phenoxy optionally substituted with R²⁷; benzyloxy optionally substituted with R³⁰ on the phenyl ring; -OC(=O)NHR²⁸; -C(=O)OR²⁸; or -OC(=O)R²⁸;
- R⁶, R⁷, R¹², R¹³, R²⁴, R²⁶ and R³⁴: are independently 1-2 halogen; nitro; C₁-C₄ alkyl; trifluoromethyl; methylthio; or C₁-C₄ alkoxy;
- R⁸, R¹⁴, R²⁰, R³⁸ and R⁴⁰: are independently H or C₁-C₄ alkyl;
- R⁹: is C₁-C₁₈ alkyl; or phenyl optionally substituted with R⁷;
- R¹⁰, R²⁵ and R³³: are each independently 1-2 substituents selected from the group consisting of halogen, nitro, cyano, C₁-C₄ alkyl, trifluoromethyl, C₁-C₄ alkylthio, C₁-C₄ alkoxy and trifluoromethoxy;
- R¹¹ and R³⁶: are independently C₁-C₆ alkyl; or phenyl optionally substituted with R¹²;
- R¹⁵: is H; C₁-C₈ alkyl optionally substituted with C₁-C₂ alkoxy; C₃-C₆ cycloalkyl; C₃-C₈ alkenyl; C₃-C₈ alkynyl; phenyl optionally substituted with R¹³; benzyl optionally substituted with R¹³ on the phenyl ring and with R²⁰ on the benzylic carbon; or pyridyl optionally substituted with R¹³;
- R¹⁶: is H; C₁-C₁₇ alkyl optionally substituted with R³¹; C₂-C₁₇ alkenyl optionally substituted with R³²; C₂-C₇ alkynyl; C₃-C₈ cycloalkyl; C₅-C₆ cycloalkenyl; C₆-C₇ alkylcycloalkyl; C₄-C₈ cycloalkylalkyl; phenyl optionally substituted with R³³; naphthalenyl, furanyl, thienyl, benzoyl, or pyridyl each optionally substituted with R³⁴; or C₂-C₅ alkoxycarbonyl;
- R¹⁷ and R¹⁸: are independently C₁-C₁₈ alkyl optionally substituted with R²³; C₂-C₁₀ alkenyl optionally substituted with R³²; C₃-C₈ alkynyl; C₃-C₁₂ cycloalkyl; C₅-C₆ cycloalkenyl; C₆-C₇ alkylcycloalkyl; C₆-C₇ cycloalkylalkyl; or phenyl, naphthalenyl, or thienyl each optionally substituted with R³⁴;
- R¹⁹: is H; C₁-C₁₀ alkyl; C₅-C₆ cycloalkyl; or phenyl optionally substituted with R³⁴; or
- R¹⁹ and R²⁰: can be taken together to form a structure selected from the group consisting of -CH₂(CH₂)₂CH₂-, -CH₂(CH₂)₃CH₂-, -CH₂(CH₂)₄CH₂-, -CH₂CH₂OCH₂CH₂-, -CH₂CH(Me)CH₂CH(Me)CH₂-, and -CH₂CH(Me)OCH(Me)CH₂-;
- R²¹: is C₁-C₈ alkyl optionally Substituted with R⁵¹; C₂-C₈ alkenyl or C₂-C₈ alkynyl each optionally substituted with R⁶⁹; C₃-C₆ cycloalkyl optionally substituted with 1-3 halogen; C(=N-V-R⁵³)R⁵²; C(=O)OR⁵³; C(=O)SR⁵³; C(=NR⁵⁵)OR⁵³; C(=S)SR⁵³; C(=O)NR⁵³R⁵⁶; or C(=NR⁵⁵)NR⁵³R⁵⁶;
- V: is O; NR⁵⁵; or a direct bond;
- R²²: is cyano; nitro; C₁-C₁₉ alkylthio; C₁-C₁₉ alkylsulfinyl; C₁-C₁₉ haloalkoxy; C₅-C₆ cycloalkyloxy; C₃-C₁₉ alkenyloxy; C₃-C₁₉ alkynyloxy; C₁-C₁₉ alkylsulfonyl; C₂-C₁₉ alkoxycarbonyl; hydroxyl; hydroxycarbonyl; R²⁸C(=O)O; R²⁸OC(=O)O; R²⁸R⁴⁰NC(=O)O; R³⁹R⁴⁰N; (C₁-C₄ alkoxy)₂P(=E)O; R¹¹SO₃; R⁴⁰R¹⁴R³⁸N⁺; phenyl, phenylthio, phenoxy, phenylsulfonyl, phenylsulfinyl, pyridyl or pyridyloxy each optionally substituted with R³⁰; thienyl, pyrimidinyl, furanyl, naphthalenyl, pyrimidinyloxy, naphthalenyloxy each optionally substituted with R⁷; tetrahydropyranyl; C₃-C₆ cycloalkyl; 2-tetrahydropyranyloxy; or C(=Q)R⁴⁰;
- E: is O or S;
- Q: is O or N-T-W;
- T: is O; NR³⁷; or a direct bond;
- W: is H; C₁-C₈ alkyl, C₃-C₈ alkenyl; phenylmethyl optionally substituted with R⁷ on the phenyl ring and R¹⁴ on the benzylic carbon; phenyl or pyridyl each optionally substituted with R⁷; C(=O)R²⁸; C(=O)OR²⁸; or C(=O)NR²⁸R¹⁴;
- R²³: is 1-3 halogen; C₁-C₁₂ alkoxy; C₁-C₁₂ alkylthio; phenyl or naphthalenyl each optionally substituted with R³⁴; or phenoxymethyl optionally substituted with R³⁴ on the phenyl ring;
- R²⁷: is 1-2 halogen; nitro; cyano; C₁-C₆ alkyl; C₁-C₆ haloalkyl; C₁-C₆ alkoxy; C₁-C₆ haloalkoxy; C₁-C₄ alkylsulfonyl; C₂-C₆ alkoxyalkyl; C₁-C₄ alkylthio; C₅-C₆ cycloalkyl; C₅-C₆ cycloalkyloxy; C₂-C₆ alkenyl; C₂-C₆ haloalkenyl; C₂-C₆ alkynyl; hydroxycarbonyl; C₂-C₄ alkoxycarbonyl; or phenoxy optionally substituted with R²⁴;
- R²⁸: is C₁-C₈ alkyl; or phenyl or pyridyl each optionally substituted with R³⁰;
- R²⁹: is C₁-C₈ alkyl optionally substituted with R⁴⁴; C₃-C₆ alkenyl or C₃-C₆ alkynyl each optionally substituted with R⁶⁹; C₃-C₆ cycloalkyl optionally substituted with 1-3 halogen; phenyl optionally substituted with R⁵⁷ and R⁵⁹; C(=O)R⁵²; C(=NR⁵⁵)R⁵²; C(=O)OR⁵³; C(=N(C₁-C₄ alkyl)]OR⁵³; C(=O)NR⁵³R⁵⁶; N=CR⁶⁸R⁶⁷; or SO₂R⁵²;
- R³⁰: is 1-2 substituents selected from the group consisting of halogen, nitro, cyano, C₁-C₄ alkyl, trifluoromethyl, C₁-C₄ alkoxy and trifluoromethoxy; or phenoxy optionally substituted with R²⁶;
- R³¹: is 1-3 halogen; C₁-C₁₈ alkoxy; allyloxy; C₁-C₁₈ alkylthio; phenyl, phenoxy, benzyloxy, or phenylthio each optionally substituted with R³⁴ on the phenyl ring; acetyl; or C₂-C₅ alkoxycarbonyl;
- R³²: is 1-3 halogen; or C₁-C₄ alkoxy;
- R³⁵: is cyano; nitro; C₁-C₁₇ alkylthio; C₁-C₁₇ alkylsulfinyl; C₁-C₁₇ haloalkoxy; C₅-C₆ cycloalkyloxy; C₂-C₁₇ haloalkenyl; C₃-C₁₇ alkenyloxy; C₃-C₁₇ haloalkynyl; C₃-C₁₇ alkynyloxy; C₁-C₁₇ alkylsulfonyl; C₂-C₁₇ alkoxycarbonyl; hydroxyl; hydroxycarbonyl; R²⁸C(=O)O; R²⁸OC(=O)O; R²⁸R⁴⁰NC(=O)O; R⁴⁰R³⁹N; (C₁-C₄ alkoxy)₂P(=E)O; R¹¹SO₃; R⁴⁰R¹⁴R³⁸N⁺; phenyl, phenoxy, phenylthio, phenylsulfonyl, phenylsulfinyl, pyridyl or pyridyloxy each optionally substituted with R³⁰; thienyl, pyrimidinyl, furanyl, naphthalenyl, pyrimidinyloxy, naphthalenyloxy each optionally substituted with R⁷; tetrahydropyranyl; 2-tetrahydropyranyloxy; C₁-C₁₇ alkoxy; C₂-C₁₇ alkoxyalkoxy; C₃-C₁₇ alkynyl; C₃-C₆ cycloalkyl; or C₂-C₁₇ haloalkoxyalkoxy;
- R³⁷: is H; C₁-C₆ alkyl; or phenyl optionally substituted with R⁷;
- R³⁹: is C₁-C₁₉ alkyl; C₂-C₁₉ alkylcarbonyl; C₂-C₁₉ alkoxycarbonyl; (R⁹R⁴⁰N)C=O; phenyl optionally substituted with R²⁵; or phenoxycarbonyl optionally substituted with R⁷;
- R⁴¹: is cyano; nitro; C₁-C₁₇ alkylthio; C₁-C₁₇ alkylsulfinyl; C₁-C₁₇ haloalkoxy; C₅-C₆ cycloalkyloxy; C₃-C₁₇ alkenyloxy; C₃-C₁₇ alkynyloxy; C₁-C₁₇ alkylsulfonyl; C₂-C₁₇ alkoxycarbonyl; hydroxyl; hydroxycarbonyl; R²⁸C(=O)O; R²⁸OC(=O)O; R²⁸R⁴⁰NC(=O)O; R⁴⁰R³⁹N; (C₁-C₄ alkoxy)₂P(=E)O; R¹¹SO₃; R⁴⁰R¹⁴R³⁸N⁺; phenyl, phenoxy, phenylsulfonyl, phenylsulfinyl, pyridyl or pyridyloxy each optionally substituted with R³⁰; thienyl, pyrimidinyl, furanyl, naphthalenyl, pyrimidinyloxy, naphthalenyloxy each optionally substituted with R⁷; tetrahydropyranyl; 2-tetrahydropyranyloxy; C₁-C₁₇ alkoxy; 1-3 halogen; C₂-C₁₇ alkoxyalkoxy; or C₃-C₆ cycloalkyl;
- R⁴²: is cyano; nitro; C₁-C₁₇ alkylthio; C₁-C₁₇ alkylsulfinyl; C₁-C₁₇ haloalkoxy; C₅-C₆ cycloalkyloxy; C₃-C₁₇ alkenyloxy; C₃-C₁₇ haloalkynyl; C₃-C₁₇ alkynyloxy; C₁-C₁₇ alkylsulfonyl; C₂-C₁₇ alkoxycarbonyl; hydroxyl; hydroxycarbonyl; R²⁸C(=O)O; R²⁸OC(=O)O; R²⁸R⁴⁰NC(=O)O; R⁴⁰R³⁹N; (C₁-C₄ alkoxy)₂P(=E)O; R¹¹SO₃; R⁴⁰R¹⁴R³⁸N⁺; phenyl, phenoxy, phenylthio, phenylsulfonyl, phenylsulfinyl, pyridyl or pyridyloxy each optionally substituted with R³⁰; thienyl, pyrimidinyl, furanyl, naphthalenyl, pyrimidinyloxy, naphthalenyloxy each optionally substituted with R⁷; tetrahydropyranyl; 2-tetrahydropyranyloxy; C₁-C₁₇ alkoxy; 1-3 halogen; C₂-C₁₇ alkoxyalkoxy; C₃-C₁₇ alkynyl; or C₃-C₆ cycloalkyl;
- R⁴⁴: is 1-3 halogen; cyano; nitro; C₁-C₆ alkoxy; C₁-C₆ haloalkoxy; C₂-C₆ alkoxyalkoxy; C₁-C₆ alkylthio; C₁-C₆ alkylsulfonyl; phenyl or phenoxy each optionally substituted with R⁵⁷ and R⁵⁹; NR⁴⁹R⁵⁰; or R⁶²;
- R⁴⁵: is H; C₁-C₄ alkyl; C₁-C₄ haloalkyl; C₂-C₄ alkenyl; C₂-C₆ haloalkenyl; NR⁵⁴R⁵⁵; or SR⁵⁴;
- R⁴⁶: is H; C₁-C₄ alkyl; C₁-C₄ haloalkyl; C₂-C₄ alkenyl; C₂-C₆ haloalkenyl; phenyl optionally substituted with R⁵⁷; NR⁵⁶R⁶⁴; OR⁶⁵; or SR⁶⁵;
- R⁴⁷: is C₁-C₈ alkyl optionally substituted with R⁴⁴; C₃-C₆ alkenyl or C₃-C₆ alkynyl each optionally substituted with R⁶⁹; C₃-C₆ cycloalkyl optionally substituted with 1-3 halogen; phenyl optionally substituted with R⁵⁷ and R⁵⁹; C(=O)R⁵²; C(=NR⁵⁵)R⁵²; C(=O)OR⁵³; C[=N(C₁-C₄ alkyl)]OR⁵³; or C(=O)NR⁵³R⁵⁶;
- R⁴⁸: is C₁-C₆ alkyl; C₂-C₆ alkenyl; C₂-C₆ haloalkenyl; C₂-C₆ alkynyl; C₂-C₆ alkoxyalkyl; phenyl optionally substituted with R⁵⁸; or phenylmethyl optionally substituted with R⁵⁸ on the phenyl ring;
- R⁴⁹: is H; C₁-C₄ alkyl; C₃-C₄ alkenyl; or cyclopropyl;
- R⁵⁰: is H; C₁-C₆ alkyl; C₃-C₆ alkenyl; C₃-C₆ alkynyl; C₂-C₆ alkoxyalkyl; C₃-C₆ haloalkenyl; phenyl optionally substituted with R⁵⁸ and R⁵⁹; or phenylmethyl optionally substituted with R⁵⁸ and R⁵⁹ on the phenyl ring; or
- R⁴⁹ and R⁵⁰: can be taken together to form -(CH₂)₄-; -(CH₂)₅- or -CH₂CH₂OCH₂CH₂-;
- R⁵¹: is 1-3 halogen; C₁-C₆ alkoxy; C₂-C₆ haloalkoxy; C₂-C₆ alkoxyalkoxy; C₁-C₆ alkylthio; C₁-C₆ haloalkylthio; C₃-C₆ alkenyloxy; C₃-C₆ alkynyloxy; C₁-C₆ alkylsulfonyl; C₁-C₆ haloalkylsulfonyl; phenylsulfonyl optionally substituted with R⁵⁷; phenyl or phenoxy each optionally substituted with R⁵⁸ and R⁵⁹; OH; SH; nitro; cyano; O=C=N; S=C=N; NR⁴⁹R⁵⁰; or R⁶²;
- R⁵²: is H; C₁-C₄ alkyl; C₁-C₄ haloalkyl; C₂-C₄ alkenyl; C₂-C₆ haloalkenyl; or phenyl optionally substituted with R⁵⁷;
- R⁵³: is H; C₁-C₆ alkyl; C₃-C₆ alkenyl; C₃-C₆ haloalkenyl; C₃-C₆ alkynyl; C₂-C₆ alkoxyalkyl; phenyl optionally substituted with R⁵⁸ and R⁵⁹; or phenylmethyl optionally substituted with R⁵⁸ and R⁵⁹ on the phenyl ring;
- R⁵⁴, R⁵⁵ and R⁵⁶: are each independently H or C₁-C₄ alkyl;
- R⁵⁷: is 1-2 halogen; nitro; CF₃; methoxy; methyl; or cyano;
- R⁵⁸: is halogen; nitro; CF₃; OCF₃; methoxy; methyl; ethyl; methylthio; cyano; or methoxycarbonyl;
- R⁵⁹: is halogen or C₁-C₄ alkyl;
- R⁶⁰: is C₁-C₄ alkyl; C₁-C₄ haloalkyl; C₂-C₄ alkenyl; C₂-C₆ haloalkenyl; phenyl optionally substituted with R⁵⁷; or C(=O)R⁶¹;
- R⁶¹: is H; C₁-C₄ alkyl; C₁-C₄ haloalkyl; C₂-C₄ alkenyl; C₂-C₆ haloalkenyl; or phenyl optionally substituted with R⁵⁷;
- R⁶²: is C(=N-V-R⁵³)R⁵²; C(=O)OR⁵³; C(=O)NR⁵³R⁵⁶; C(=NR⁵⁵)OR⁵³; C(=NR⁵⁵)NR⁵³R⁵⁶; OC(=O)R⁵²; SC(=O)R⁵²; N(R⁵⁶)C(=O)R⁵²; OC(=NR⁵⁵)R⁵²; N(R⁵⁶)C(=NR⁵⁵)R⁵²; OC(=O)OR⁵³; OC(=O)NR⁵³R⁵⁶; OC(=S)SR⁵³; SC(=O)OR⁵³; N(R⁵⁶)C(=O)OR⁵³; or N(R⁵⁶)C(=NR⁵⁵)NR⁵³R⁵⁴;
- R⁶³: is H; C₁-C₆ alkyl; C₃-C₆ alkenyl; C₃-C₆ alkynyl; C₂-C₆ alkoxyalkyl; C₃-C₆ haloalkenyl; phenyl optionally substituted with R⁵⁸ and R⁵⁹; or phenylmethyl optionally substituted with R⁵⁸ and R⁵⁹ on the phenyl ring; C(=O)R⁵²; C(=NR⁵⁵)R⁵²; C(=O)=R⁵³; C(=O)NR⁵³R⁵⁶; OR⁵³; or SO₂R⁵²;
- R⁶⁴: is C₁-C₄ alkyl; C₃-C₆ alkenyl; or phenyl optionally substituted with R⁵⁷ and R⁵⁹;
- R⁶⁵ and R⁶⁶: are each independently C₁-C₄ alkyl; C₃-C₄ haloalkyl; C₃-C₆ alkenyl; or phenyl optionally substituted with R⁵⁷ and R⁵⁹;
- R⁶⁷: is H; C₁-C₄ alkyl; C₁-C₄ haloalkyl; or C₂-C₄ alkenyl; C₂-C₆ haloalkenyl; phenyl optionally substituted with R⁵⁷; OR⁶⁶; SR⁶⁶; or NR⁵⁴R⁶⁶;
- R⁶⁸: is H; C₁-C₄ alkyl; C₁-C₄ haloalkyl; or C₂-C₄ alkenyl; and
- R⁶⁹: is 1-3 halogen; cyano; nitro; or C(=O)OR⁵⁴; provided that the total number of carbons in R², R¹⁶, R¹⁷ and R¹⁸ is each less than or equal to 20.

### DETAILED DESCRIPTION OF THE INVENTION

In the above recitations, the term "alkyl", used either alone or in compound words such as "alkylthio" or "haloalkyl" denotes straight-chain or branched alkyl; e.g., methyl, ethyl, *n*-propyl, *i*-propyl, or the different butyl, pentyl or hexyl isomers.

"Alkenyl" denotes straight-chain or branched alkenes; e.g., 1-propenyl, 2-propenyl, 3-propenyl and the different butenyl, pentenyl and hexenyl isomers. "Alkenyl" also denotes polyenes such as 1,3-hexadiene and 2,4,6-heptatriene.

"Alkenyloxy" denotes straight-chain or branched alkenyloxy moieties. Examples of alkenyloxy include H₂C=CHCH₂O, (CH₃)₂C=CHCH₂O, (CH₃)CH=CHCH₂O, (CH₃)CH=C(CH₃)CH₂O and CH₂=CHCH₂CH₂O.

"Alkynyl" denotes straight-chain or branched alkynes; e.g., ethynyl, 1-propynyl, 3-propynyl and the different butynyl, pentynyl and hexynyl isomers. "Alkynyl" can also denote moieties comprised of multiple triple bonds; e.g., 2,7-octadiyne and 2,5,8-decatriyne.

"Alkynyloxy" denotes straight-chain or branched alkynyloxy moieties. Examples include HC≡CCH₂O, CH₃C≡CCH₂O and CH₃C≡CCH₂CH₂O.

"Alkylthio" denotes branched or straight-chain alkylthio moieties; e.g., methylthio, ethylthio, and the different propylthio, butylthio, pentylthio and hexylthio isomers.

Examples of "alkylsulfonyl" include CH₃SO₂, CH₃CH₂SO₂, CH₃CH₂CH₂SO₂, (CH₃)₂CHSO₂ and the different butylsulfonyl, pentylsulfonyl and hexylsulfonyl isomers.

"Alkylsulfinyl" denotes both enantiomers of an alkylsulfinyl group. For example, CH₃SO, CH₃CH₂SO, CH₃CH₂CH₂SO, (CH₃)₂CHSO and the different butylsulfinyl, pentylsulfinyl and hexylsufinyl isomers.

"Alkoxy" denotes, for example, methoxy, ethoxy, *n*-propyloxy, isopropyloxy and the different butoxy, pentoxy and hexyloxy isomers.

Examples of "alkoxyalkoxyalkoxy" include CH₃OCH₂CH₂OCH₂O, CH₃CH₂OCH₂CH₂OCH₂O, and (CH₃)₂CHOCH₂CH₂OCH₂O.

"Cycloalkyl" denotes, for example, cyclopropyl, cyclobutyl, cyclopentyl, and cyclohexyl. The term "cycloalkyloxy" denotes the same groups linked through an oxygen atom such as cyclopentyloxy and cyclohexyloxy. "Cycloalkenyl" denotes groups such as cyclopentenyl and cyclohexenyl.

Examples of "cycloalkylalkyl" include cyclopropylmethyl, cyclohexylethyl, and other cycloalkyl moieties bonded to straight-chain or branched alkyl groups. "Alkylcycloalkyl" denotes alkyl substitution on a cycloalkyl moiety. Examples include 4-methylcyclohexyl and 3-isopropylcyclopentyl.

The term "halogen", either alone or in compound words such as "haloalkyl", denotes fluorine, chlorine, bromine or iodine. Further, when used in compound words such as "haloalkyl", said alkyl may be partially or fully substituted with halogen atoms which may be the same or different. Examples of "haloalkyl" include F₃C, ClCH₂, CF₃CH₂ and CF₃CF₂. Examples of "haloalkenyl" include (Cl)₂C=CHCH₂ and CF₃CH₂CH=CHCH₂. Examples of "haloalkynyl" include HC≡CCHCl, CF₃C≡C, CCl₃C≡C and FCH₂C≡CCH₂. Examples of "haloalkoxy" include CF₃O, CCl₃CH₂O, CF₂HCH₂CH₂O and CF₃CH₂O. Examples of "haloalkylthio" include CCl₃S, CF₃S, CCl₃CH₂S and CH₂ClCH₂CH₂S. Examples of "haloalkylsulfonyl" include CF₃SO₂, CCl₃SO₂, CF₃CH₂SO₂ and CF₃CF₂SO₂. Examples of haloalkoxyalkoxy" include CF₃OCH₂O, ClCH₂CH₂OCH₂CH₂O, Cl₃CH₂OCH₂O as well as branched alkyl derivatives.

The total number of carbon atoms in a substituent group is indicated by the "Cᵢ-Cⱼ" prefix where i and j are numbers from 1 to 20. For example, C₁-C₃ alkylsulfonyl designates methylsulfonyl through propylsulfonyl; C₂ alkoxyalkoxy designates CH₃OCH₂O; C₃ alkoxyalkoxy designates, for example, CH₃OCH₂CH₂O or CH₃CH₂OCH₂O; and C₄ alkoxyalkoxy designates the various isomers of an alkoxy group substituted with a second alkoxy group containing a total of 4 carbon atoms, examples including CH₃CH₂CH₂OCH₂O, and CH₃CH₂OCH₂CH₂O. Examples of "alkoxyalkyl" include CH₃OCH₂, CH₃OCH₂CH₂, CH₃CH₂OCH₂, CH₃CH₂CH₂CH₂OCH₂ and CH₃CH₂OCH₂CH₂. Examples of "alkoxycarbonyl" include CH₃OC(=O), CH₃CH₂OC(=O), CH₃CH₂CH₂OC(=O), (CH₃)₂CHOC(=O) and the different butoxy-, pentoxy- or hexyloxycarbonyl isomers.

In the above recitations, when a compound of Formula I is comprised of one or more pyridyl or pyrimidinyl rings, all bonds to these heterocycles are made through the carbon atom(s) of the moieties. When a substituent for a compound of Formula I is defined to include 1-2 halogen or 1-3 halogen, this denotes the occurrence of the same or different halogens at that substituent position one, two, or three times.

When R¹ and R² of the compounds of Formula I are different, then the compounds of Formula I possess a chiral center. This invention, therefore, comprises racemic mixtures as well as pure enantiomers. Compounds of Formula I can also exist as (E)- or (Z)-isomers, or as a mixture of (E)- and (Z)-isomers when compounds of Formula I contain a C=C bond or a C=N bond. This invention, therefore, also comprises mixtures of geometric isomers as well as the individual isomers.

Preferred compounds of Formula I (denoted as Preferred 1) are those wherein:
- R¹: is C₁-C₄ alkyl; C₁-C₄ haloalkyl; C₃-C₄ cycloalkyl; or C₂-C₄ alkenyl;
- R²¹: is C₁-C₄ alkyl optionally substituted with R⁵¹; C₂-C₄ alkenyl, C₂-C₈ alkynyl, or cyclopropyl each optionally substituted with 1-3 halogen; C(=N-V-R⁵³)H; C(=N-V-R⁵³) (C₁-C₄ alkyl); C(=O)OR⁵³; C(=O)SR⁵³; C(=S)SR⁵³; or C(=O)NR⁵³R⁵⁶;
- R²⁹: is C₁-C₄ alkyl optionally substituted with R⁴⁴; C₃-C₆ alkenyl, C₃-C₆ alkynyl, or cyclopropyl each optionally substituted with 1-3 halogen; C(=O)R⁵²; C(=NR⁵⁵)R⁵²; C(=O)OR⁵³; C(=O)NR⁵³R⁵⁶; N=CR⁶⁸R⁶⁷; or SO₂R⁵²;
- R⁴⁴: is 1-3 halogen; cyano; nitro; C₁-C₄ alkoxy; C₁-C₄ haloalkoxy; C₂-C₄ alkoxyalkoxy; C₁-C₄ alkylthio; or R⁶²;
- R⁴⁵: is H; C₁-C₄ alkyl; C₁-C₄ haloalkyl; C₂-C₄ alkenyl; or C₂-C₄ haloalkenyl;
- R⁴⁶: is H; C₁-C₄ alkyl; C₁-C₄ haloalkyl; C₂-C₄ alkenyl; C₂-C₆ haloalkenyl; NR⁵⁶R⁶⁴; OR⁶⁵; or
- R⁴⁷: is C₁-C₄ alkyl optionally substituted with R⁴⁴; C₃-C₄ alkenyl or C₃-C₄ alkynyl each optionally substituted with R⁶⁹; C₃-C₆ cycloalkyl optionally substituted with 1-3 halogen; C(=O)R⁵²; C(=O)OR⁵³; or C(=O)NR⁵³R⁵⁶;
- R⁴⁸: is C₁-C₄ alkyl; C₂-C₄ alkenyl; C₂-C₄ haloalkenyl; C₂-C₄ alkynyl; or C₂-C₆ alkoxyalkyl;
- R⁴⁹: is H; C₁-C₄ alkyl; C₃-C₄ alkenyl; or cyclopropyl;
- R⁵⁰: is H; C₁-C₄ alkyl; C₃-C₄ alkenyl; or C₃-C₄ alkynyl; or
- R⁴⁹ and R⁵⁰: can be taken together to form -(CH₂)₄-; -(CH₂)₅- or -CH₂CH₂OCH₂CH₂-;
- R⁵¹: is 1-3 halogen; C₁-C₄ alkoxy; C₂-C₄ haloalkoxy; C₂-C₄ alkoxyalkoxy; C₁-C₄ alkylthio; C₃-C₄ alkenyloxy; C₃-C₄ alkynyloxy; OH; SH; nitro; cyano; O=C=N; S=C=N; NR⁴⁹R⁵⁰; or R⁶²;
- R⁵²: is H; C₁-C₄ alkyl; C₁-C₄ haloalkyl; C₂-C₄ alkenyl; or C₂-C₄ haloalkenyl;
- R⁵³: is H; C₁-C₄ alkyl; C₃-C₄ alkenyl; or C₃-C₄ haloalkenyl;
- R⁶⁰: is C₁-C₄ alkyl; C₁-C₄ haloalkyl; C₂-C₄ alkenyl; C₂-C₄ haloalkenyl; C(=O) (C₁-C₄ alkyl); or C(=O)H;
- R⁶²: is C(=N-V-R⁵³)R⁵²; C(=O)OR⁵³; C(=O)NR⁵³R⁵⁶; OC(=O)R⁵²; SC(=O)R⁵²; N(R⁵⁶)C(=O)R⁵²; OC(=O)OR⁵³; OC(=O)NR⁵³R⁵⁶; or N(R⁵⁶)C(=O)OR⁵³;
- R⁶³: is H; C₁-C₄ alkyl; C₃-C₄ alkenyl; C₃-C₄ alkynyl; C₃-C₄ haloalkenyl; C(=O)R⁵²; C(=NR⁵⁵)R⁵²; C(=O)OR⁵³; C(=O)NR⁵³R⁵⁶; or OR⁵³;
- R⁶⁴: is C₁-C₄ alkyl; or C₃-C₄ alkenyl;
- R⁶⁵: is C₁-C₄ alkyl; C₃-C₄ haloalkyl; or C₃-C₄ alkenyl; and
- R⁶⁷: is H or C₁-C₄ alkyl;
provided that when R³ is a phenyl or heterocyclic ring disubstituted with two alkyl or alkoxy groups, or one alkyl and one alkoxy group, then at least one of the alkyl and alkoxy groups is methyl or methoxy.

More preferred compounds of Formula I (denoted as Preferred 2) are the compounds of Preferred 1 wherein:
- A: is O or NH;
- B: is halogen; cyano; R²¹; OR²⁹; NR⁴⁹R⁶³; N=CR⁴⁵R⁴⁶; SR⁴⁷; or S(O)₂R⁴⁸;
- R¹: is C₁-C₄ alkyl; C₁-C₄ haloalkyl; or vinyl;
- R²: is C₂-C₂₀ alkyl; C₂-C₂₀ alkoxyalkyl; C₂-C₂₀ haloalkyl; C₃-C₈ alkyl substituted with phenoxy or phenylthio each optionally substituted with R³⁰; C₅-C₇ cycloalkyl; C₂-C₂₀ alkenyl; C₅-C₇ cycloalkenyl; phenyl optionally substituted with R⁵ and R⁷; 2-naphthalenyl; thienyl optionally substituted with R⁵ and R⁷; furyl optionally substituted with R⁷; or pyridyl optionally substituted with R⁵ and R⁷;
provided that when R² is phenyl and R⁵ is other than F, then R⁵ is attached to the para-position relative to the imidazolinone ring;
- R³: is phenyl optionally substituted with R¹⁰;
- R⁴⁴: is 1-3 halogen; C₁-C₄ alkoxy or C₁-C₄ haloalkoxy;
- R⁴⁵: is H or C₁-C₄ alkyl;
- R⁴⁶: is H; C₁-C₄ alkyl; OR⁶⁵; or SR⁶⁵;
- R⁴⁷: is C₁-C₄ alkyl; C₃-C₄ alkenyl; C(=O)R⁵²; or C(=O)OR⁵⁵;
- R⁴⁸: is C₁-C₄ alkyl;
- R⁵¹: is 1-3 halogen or C₂-C₃ haloalkoxy;
- R⁵² and R⁵³: are each independently H; C₁-C₄ alkyl; or C₃-C₄ alkenyl;
- R⁶⁰ and R⁶³: are each independently C₁-C₄ alkyl; and
- R⁶⁵: is C₁-C₄ alkyl or C₃-C₄ alkenyl.

More preferred compounds of Formula I (denoted as Preferred 3) are the compounds of Preferred 2 wherein:
- B: is halogen; cyano; C₁-C₄ alkyl, C₁-C₄ alkylthio, or C₁-C₄ alkoxy each optionally substituted with halogen; N=CR⁴⁵R⁴⁶; NR⁴⁹R⁶³; or S(O)₂(C₁-C₄ alkyl);
- R¹: is methyl or halomethyl;
- R²: is C₂-C₁₂ alkyl; C₃-C₈ alkyl substituted with phenoxy optionally substituted with R³⁰; phenyl optionally substituted with R⁵ and R⁷; thienyl optionally substituted with R⁷; or pyridyl optionally substituted with R⁵ and R⁷;
- R³: is phenyl optionally substituted with F; Cl; or methyl;
- R⁴: is H;
- R⁵: is halogen; nitro; C₁-C₆ alkyl; C₁-C₃ haloalkyl; methylthio; C₁-C₆ alkoxy; C₁-C₂ haloalkoxy; C₅-C₆ cycloalkyloxy; phenoxy optionally substituted with R²⁷; phenylthio substituted with R²⁴; phenoxymethyl optionally substituted with R²⁴ on the phenyl ring; benzyloxy optionally substituted with R³⁰ on the phenyl ring; or -OC(=O)R²⁸;
- R⁷ and R²⁴: are independently F; C₁-C₂ alkyl; methylthio; or C₁-C₂ alkoxy;
- R¹⁶: is C₁-C₆ alkyl;
- R¹⁹: is phenyl optionally substituted with R³⁴;
- R²⁷: is 1-2 halogen; cyano; C₁-C₄ alkyl; trifluoromethyl; C₁-C₄ alkoxy; C₁-C₄ haloalkoxy; C₁-C₄ alkylthio; C₅-C₆ cycloalkyloxy; or allyl;
- R³⁰: is 1-2 halogen; cyano; C₁-C₄ alkyl; trifluoromethyl; C₁-C₄ alkoxy; or trifluoromethoxy;
- R³⁴: is 1-2 halogen; nitro; C₁-C₂ alkyl; or C₁-C₂ alkoxy;
- R⁴⁶: is H or C₁-C₄ alkyl;
- R⁴⁹ and R⁶³: are each independently C₁-C₂ alkyl

Especially preferred compounds of Formula I (denoted as Preferred 4) are the compounds of Preferred 3 wherein:
- B: is F; Cl; cyano; C₁-C₂ alkyl; C₁-C₂ alkylthio; C₁-C₂ alkoxy; N=CR⁴⁵R⁴⁶; NMe₂; or S(O)₂(C₁-C₂ alkyl);
- R¹: is methyl;
- R²: is C₁-C₁₂ alkyl; phenyl optionally substituted with R⁵ and R⁷; or thienyl optionally substituted with R⁵ and R⁷; and
- R⁵: is F; Cl; Br; C₁-C₆ alkyl; trifluoromethyl; C₁-C₆ alkoxy; trifluoromethoxy; 2,2,2-trifluoroethoxy; C₅-C₆ cycloalkyloxy; methylthio; phenoxy optionally substituted with R²⁷; phenylthio optionally substituted with R²⁴; benzyloxy optionally substituted with R³⁰ on the phenyl ring; or -OC(=O)R²⁸.

Specifically preferred compounds of Formula I are the compounds of Preferred 4 which are:
3,5-dihydro-2-methoxy-5-methyl-5-phenyl-3-(phenylamino)-4H-imidazole-4-one; and
3,5-dihydro-5-methyl-2-(methylthio)-5-phenyl-3-(phenylamino)-4H-imidazol-4-one.

It is recognized that some reagents and reaction conditions described below for preparing compounds of Formula I may not be compatible with some functionalities claimed for R¹, R², R³, R⁴, A and B. In these cases, the incorporation of protection/deprotection sequences into the synthesis may be necessary in order to obtain the desired products. The cases in which protecting groups are necessary, and which protecting group to use, will be apparent to one skilled in chemical synthesis.

In the following description of the preparation of compounds of Formula I, compounds denoted as Formula Ia through Formula Ix are various subsets of the compounds of Formula I, and all substituents for Formula Ia through Ix are as defined above for Formula I.

The compounds of Formula I can be prepared as described below in the following Schemes. The 4(H)-imidazol-4-ones of Formula Ia can be prepared by one or all of the methods illustrated in Scheme I.

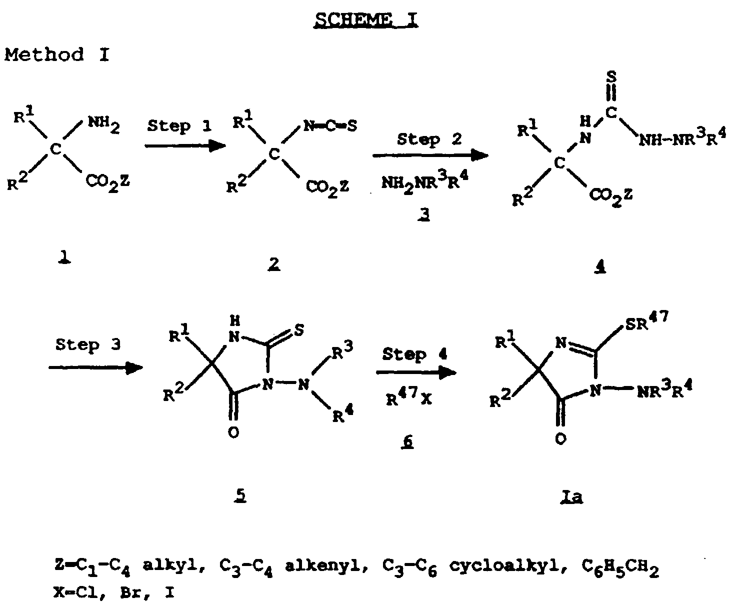

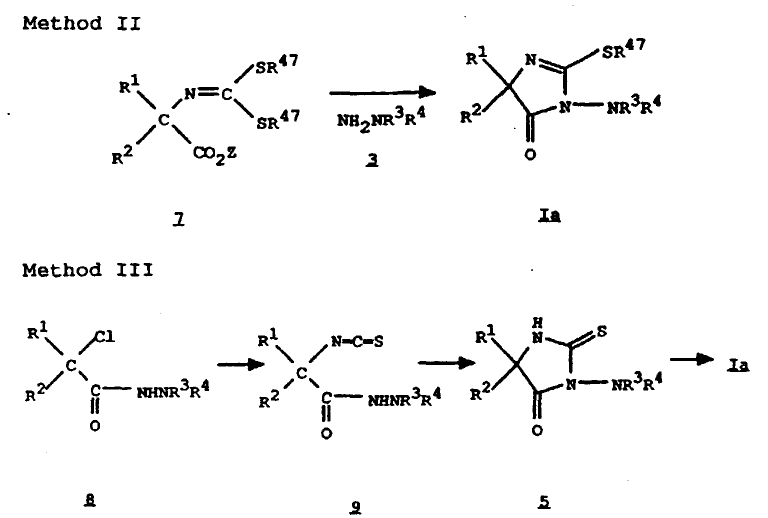

The esters of α-amino-acids of Formula 1 or their salts are known in the literature and can be prepared by literature methods [J. P. Greenstein, M. Winitz, "Chemistry of the Amino Acids" (S. Patai, Ed.), p 697, John Wiley and Sons, Ltd., London (1961)]. The group Z in compounds of Formula 1 can be C₁-C₄ alkyl; C₃-C₄ alkenyl; C₃-C₆ cycloalkyl; or C₆H₅CH₂. Preferred for ease of synthesis and lower expense are Z=C₁-C₄ alkyl. The preparation of the isothiocyanates of Formula 2 from the α-amino esters or their salts of Formula 1 (Step 1) can also be accomplished by literature methods (H. A. Staab and G. Walther, *Liebigs Ann. Chem*., **1962**, *657*, 98; M. L. Moore, F. S. Crossley, *Org. Synth*., **1941**, *21*, 81).

Compounds of Formula 4 can be prepared by treatment of an isothiocyanates of Formula 2 with hydrazines of Formula 3 (Step 2). The preparation of substituted hydrazines of Formula 3 can be accomplished by literature methods (J. Timerblake, J. Stowell; "The Chemistry of the Hydrazo, Azo and Azoxy Groups" (S. Patai, Ed.) p 69, John Wiley and Sons, Ltd., London (1975); J. P. Demers, D. J. Klaubert, *Tetrahedron Lett*., **1987**, 4933). To prepare compounds of Formula 4, the compounds of Formula 2 are dissolved in an inert solvent such as 1-chlorobutane or dichloromethane, and a hydrazine of Formula 3 is added, at a temperature from -50° to 50°C. When the reaction is complete, the resulting mixture is poured into a water-immiscible solvent and washed successively with dilute aqueous mineral acid, water, and brine. The organic phase of this mixture is separated, dried, and the solvent is evaporated to provide the products of Formula 4.

As shown in Step 3, the 2-thioxo-4-imidazolidinone compounds of Formula 5 can be prepared by dissolving compounds of Formula 4 in an inert solvent, such as benzene or toluene, and heating at a temperature from 50° to 200°C. When the reaction is complete, the solvent is evaporated and the resulting mixture is purified to yield products of Formula 5.

In some cases, the isolation of compounds of Formula 4 is unnecessary. For example, compounds of Formula 4 can be converted *in situ* to compounds of Formula 5 by warming the reaction mixture to 50°-200°C. When the reaction is complete, the solvent is removed to provide compounds of Formula 5.

The 4(H)-imidazol-4-ones of Formula Ia can be prepared by dissolving compounds of Formula 5 in an inert solvent, such as chloroform or tetrahydrofuran, and treating the solution with an electrophilic substrate R⁴⁷X wherein X is a leaving group such as a chlorine, bromine, or iodine; followed by a base such as 1,8-diaza-bicyclo[5.4.0]-undec-7-ene, at a temperature from 0° to 100°C (Step 4). When the reaction is complete, the solvent is evaporated and the resulting mixture is purified to yield products of Formula Ia.

An alternative process for preparing compounds of Formula Ia is illustrated in Method II, Scheme I. The α-bis(thio)methyleneamino acid esters of Formula 7 can be prepared by literature methods (D. Hoppe, *Angew. Chem., Int. Ed. Eng.*, **1975**, *14*, 426). Compounds of Formula Ia can be prepared by dissolving compounds of Formula 7 in a protic acid, such as acetic acid, with or without an inert diluent, such as dichloromethane, in the presence of a hydrazine of Formula 3, at a temperature from 20° to 100°C. When the reaction is complete, the reaction mixture is poured into water and extracted with a water-immiscible organic solvent such as ethyl acetate. The combined organic extracts are washed first with aqueous base, such as aqueous sodium bicarbonate, and then with water. The organic layer is then dried and evaporated to yield compounds of Formula Ia.

Another route to synthesize compounds of Formula Ia is illustrated in Method III, Scheme I. This method is described in the literature for when R¹ is H and neither R³ nor R⁴ are H (H. Boehme, F. Martin, J. Strahl; *Arch. Pharm.* **1980**, *313*, 10).

As shown in Step 1, a α-chlorohydrazide, prepared by literature methods (H. Böhme, F. Martin, *Acad. Pharm*., **1974**, *307*, 277), is treated with a salt of a thiocyanate, such as potassium thiocyanate, in an inert solvent, such as acetonitrile at a temperature from 0° to 50°C. When the reaction is complete, the reaction mixture is poured into water and extracted with a water-immiscible solvent such as chloroform. The organic extracts are combined and washed with water. The solvent is removed to give a residue which is redissolved and refluxed in an inert organic solvent such as acetonitrile. When the reaction is complete, the solvent is removed and the resulting mixture is purified to give a compound of Formula 5. The conversion of compounds of Formula 5 to compounds of Formula Ia is described in Step 4, Method I.

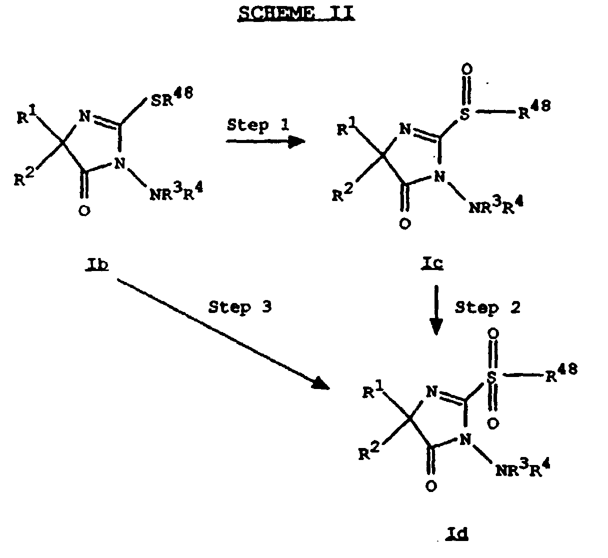

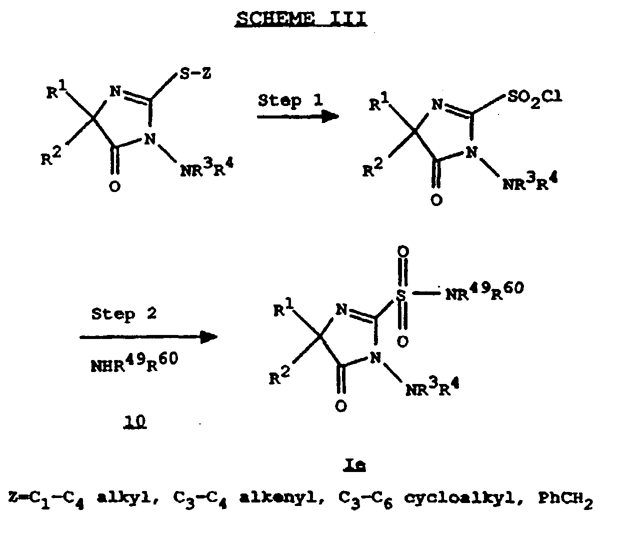

The sulfoxides and sulfones of Formula Ic and Id, respectively, can be prepared by the methods illustrated in Scheme II. The oxidation of sulfides to sulfoxides and sulfones is described in the literature (D. S. Tarbell, C. Weaver, *J. Am. Chem. Soc*., **1941**, *63*, 2939; I. Sircar et al., *J. Med. Chem*., **1985**, *28*, 1405). Compounds of Formula Ib are dissolved in an inert solvent such as dichloromethane or benzene and an oxidant such as *meta*-chloroperoxybenzoic acid or monoperphthalic acid is added (Step 1). The reaction can be conducted at a temperature from 0° to 100°C. When the reaction is complete, the reaction mixture is added to a water-immiscible solvent, washed sequentially with aqueous sodium thiosulfate solution, aqueous sodium bicarbonate solution, and water. The organic layer is dried and the solvent is evaporated to yield compounds of Formula Ic.

Following the same procedure, the preparation of sulfones of Formula Id can can be achieved either from sulfoxides of Formula Ic (Step 2), or from compounds of Formula Ib with the use of excess oxidant (Step 3).

Compounds of Formula Ie can be obtained as described in Scheme III. The oxidation of sulfides to the corresponding sulfonyl chlorides (Step 1) and reaction of the sulfonyl chlorides with an amine (Step 2) provides the sulfonamides. For example, to a solution of the sulfide compounds in a solvent such as acetic acid containing a small amount of water, a source of chlorine, such as chlorine gas is added, at a temperature from 0° to 50°C. When the reaction is complete, the sulfonyl chloride is collected, stirred in an inert solvent such as acetone, at a temperature from -10° to 25°C, and an amine of Formula 10 is added. When the reaction is complete, the solvent is removed to provide compounds of Formula Ie after purification. This sequence is well documented in the literature (R. H. Baker et al., *J. Am. Chem. Soc*., **1946**, *68*, 2636; R. F. Langler, *Can. J. Chem*., **1976**, *54*, 498; J. S. Grossert, R. F. Langler, *Can. J. Chem*., **1977**, *55*, 407, 421). The amines of Formula 10 can be prepared by literature methods [C. A. Buehler, D. E. Pearson, Survey of Organic Syntheses, Vol. **2**, p 391, John Wiley and Sons (1977)].

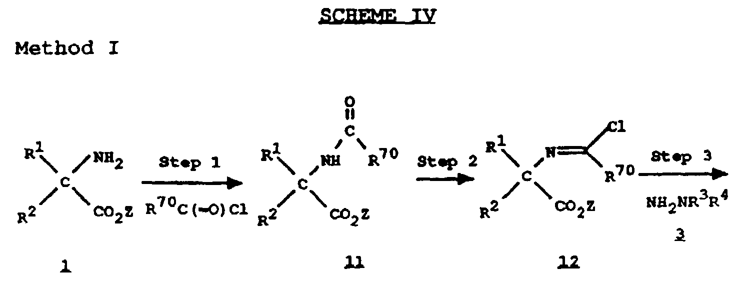

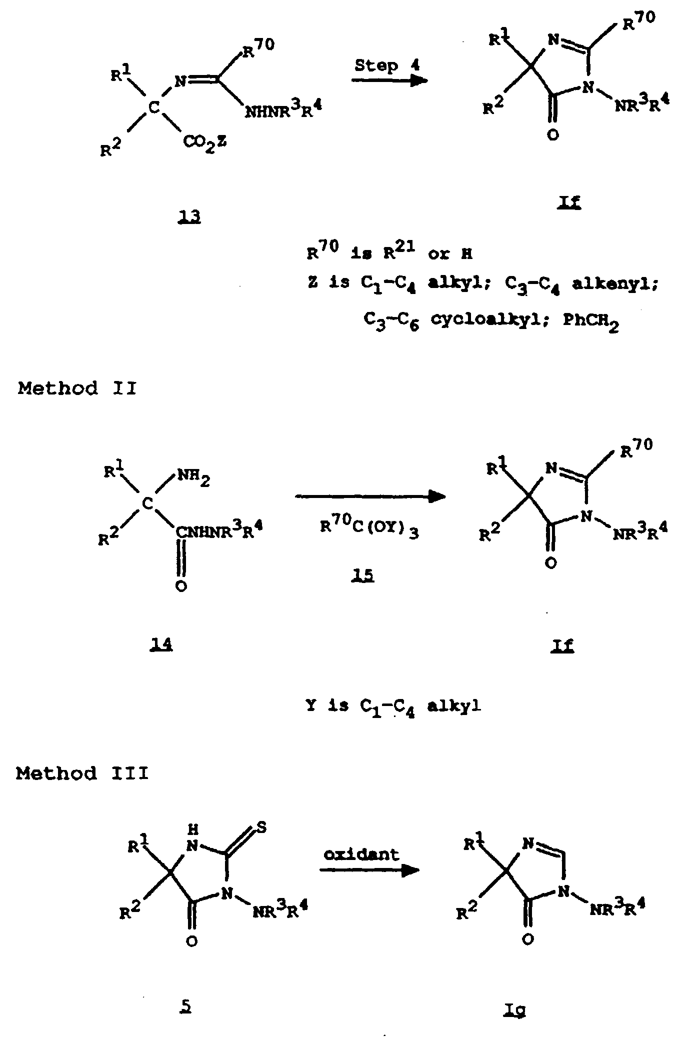

The 4H-imidazol-4-one compounds of Formula If can be prepared by one or more of the methods shown in Scheme IV. The preparation of compounds of Formula 11 from the amino acids of Formula 1 or their salts (Step 1, Method I) can be accomplished using known procedures in the literature [C. A. Buehler, D. E. Pearson, Survey of Organic Syntheses, Vol. **2**, p 813, John Wiley and Sons (1977); V. Prelog, P. Wieland, *Helv. Chim. Acta*, **1946**, *29*, 1128]. The preparation of the iminochlorides of Formula 12 (Step 2) from the asides of Formula 11 can be accomplished by literature methods (J. W. Williams et al., *Org. Synth*., **1946**, *26*, 97; C. C. Price, B. H. Velzar, *J. Org. Chem*., **1947**, *12*, 386). The displacement of the chloride in the iminochlorides with the hydrazines of Formula 3 (Step 3) can also be achieved by procedures known in the literature (H. Priewe, A. Polzak, *Ber. Deutsch. Chem. Ges*., **1955**, *88*, 1932; J. Berger et al., *Monatsch. Chem*., **1981**, *112*, 959). The cyclization of compounds of Formula 13 to heterocycles of Formula If (Step 4) can be accomplished by literature procedures known for the cyclization of *N*-(aminomethylidene)-α-amino acids to 4H-imidazolin-4-ones (Y. Ito et al., *Synth. Commun*., **1974**, *4*, 289; J. R. Ross et al., *J. Heterocycl. Chem*., **1987**, *24*, 661).

Alternatively, the compounds of Formula If can be prepared by the procedure illustrated in Method II, Scheme IV. The hydrazides of Formula 14 can be prepared by procedures known in the literature (M. Brenner, W. Hofer, *Helv. Chim. Acta*, **1961**, *44*, 1798; Unit Ika KK, JP 246,362). The condensation of compounds of Formula 14 with ortho-esters of Formula 15 provides compounds of Formula If. For example, an amino-hydrazide of Formula 14 is mixed with an orthoester of Formula 15, in the presence of a catalytic amount of acid, such as acetic acid, at a temperature from 50° to 200°C. When the reaction is complete, the mixture is purified to provide the product of Formula If. The following literature procedures describe the preparation of 4H-imidazol-4-ones from α-amino carboxylic acid amides and ortho-esters (S. Ginsburg, *J. Org. Chem*., **1962**, *27*, 4062; J. Brunken, G. Bach, *Ber. Deutsch. Chem. Ges*., **1956**, *89*, 1363). The ortho-esters of Formula 15 can be prepared according to processes described in the literature [C. A. Buehler and D. E. Pearson, Survey of Organic Syntheses, Vol. **2**, p 711, John Wiley and Sons (1977)].

In addition, a method to prepare the compounds of Formula Ig is shown in Method III, Scheme IV. The 2-thioxo-4-imidazolidinone of Formula 5 is dissolved in an inert solvent such as acetone and an oxidizing agent such as Oxone® (potassium peroxymonosulfate) is added at a temperature from 0°-100°C. When the reaction is complete, the reaction mixture is poured into a water-immiscible organic solvent such as ethyl acetate and washed with a basic aqueous solution, such as aqueous sodium bicarbonate, and then with water. The organic layer is dried and the solvent is evaporated to give the products of Formula Ig.

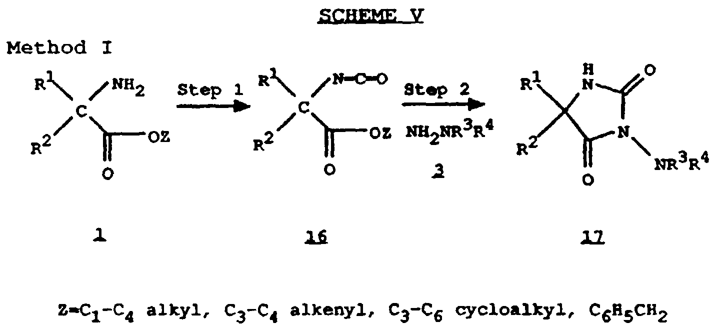

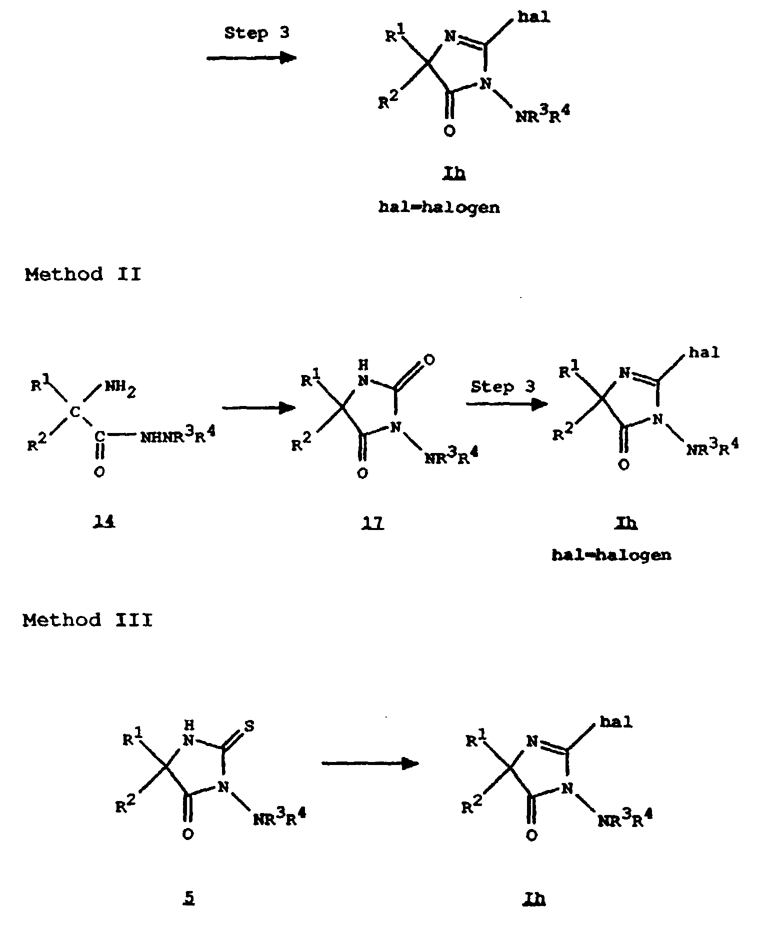

The compounds of Formula Ih can be prepared by one or more of the methods illustrated in Scheme V. The isocyanates of Formula 16 can be prepared (Step 1, Method I) by literature methods, using reagents such as phosgene (L. C. Raitord, H. B. Freyermuth, *J. Org. Chem*., **1943**, *8*, 230; G. Loose, W. Godicke, *Ber*. *Deutsch. Chem. Ges*., **1967**, *100*, 3314), *N,N*'-carbonyldiimidazole (H. A. Staab, W. Benz, *Angew*. *Chem*., **1961**, *73*, 66), or oxalyl chloride (V. Von Gizycki, *Angew. Chem*., **1971**, *83*, 406; M. W. Gittos et al., *J. Chem. Soc., Perkin Trans. 1*, **1976**, 141). The compounds of Formula 17 can be prepared by mixing the isocyanates of Formula 16 and hydrazines of Formula 3 in an inert solvent such as chlorobutane or tetrahydrofuran at a temperature of 0° to 50°C (Step 2). When the reaction is complete, the reaction mixture is poured into an organic solvent and washed sequentially with a dilute aqueous mineral acid solution, a dilute aqueous basic solution, and water. The organic fraction is dried and the solvent is evaporated to give a residue which is redissolved in an inert solvent such as toluene and heated at a temperature of 60° to 200°C. When the cyclization is complete, the solvent is removed to provide compounds of Formula 17. The preparation of 4(H)-imidazol-4-ones of Formula Ih from compounds of Formula 17 (Step 3) can be accomplished using literature procedures known to convert NHC(=O) groups to N=C(halogen) groups (D. Harrison et al., *J. Chem. Soc*., **1963**, 2930; R. Appel at al., *Ber. Deutsch. Chem. Ges*., **1974**, *107*, 698; H. V. Dobeneck, T. Messerschmitt, *Liebigs Ann. Chem*., **1971**, *751*, 32; G. Rio, D. Masure, *Bull. Soc. Chim. Fr*., **1972**, 4604).

Compounds of Formula 17 can also be prepared from compounds of Formula 14 (Method II, Scheme V) using procedures known in the literature for the preparation of hydantoins from α-amino carboxylic acid amides (S. Goldschmidt, M. Wick, *Liebigs Ann. Chem*., **1952**, *575*, 217). Conversion of hydantoins of Formula 17 to heterocycles of Formula Ih is discussed in Step 3 above.

Another method to prepare compounds of Formula Ih is illustrated in Method III, Scheme V. This conversion can be accomplished using literature procedures (C. R. Petrie et al., *J. Med. Chem*., **1985**, *28*, 1010; R. E. Holmes, R. K. Robins, *J. Am. Chem*. *Soc*., **1964**, *86*, 1242; R. T. Koda, J. A. Biles, W. Wolf, *J. Pharm. Sci*., **1968**, *57*, 2056) known for the conversion of NHC(=S) groups to the corresponding N=C(halogen) groups.

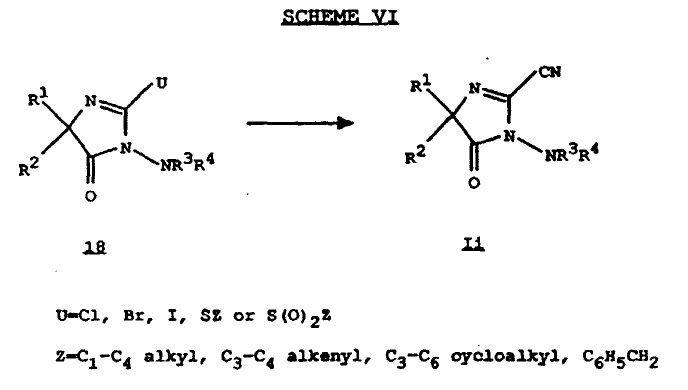

The cyano compounds of Formula Ii can be prepared according to literature procedures for the conversion of -N=C-U functionalities, wherein U=Cl, Br, I, SZ, S(O)₂Z and Z is defined as described above, to -N=C-C≡N moieties, by displacing the group U with cyanide salts. For example, a sulfonyl compound of Formula 18 (Q=S(O)₂Z) is treated with a salt of cyanide, such as potassium cyanide, in an inert solvent, such as dimethysulfoxide, at a temperature from 20° to 200°C. When the reaction is complete, the reaction mixture is poured into water and extracted with an organic solvent. The organic extracts are combined, and washed with water. The solvent is removed to provide a product of Formula Ii after purification. (Scheme VI, N. G. Clark, E. Cawkill, *Tetrahedron Lett*., **1975**, 2717; T. Kato et al., *Chem. Pharm. Bull*., **1986**, *34*, 3635; P. A. Wade et al., *J. Org. Chem*., **1983**, *48*, 1796)

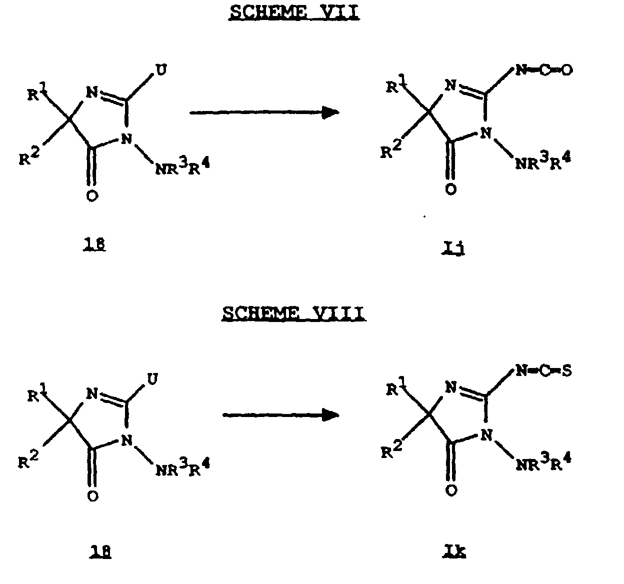

Compounds of Formulae Ij and Ik can be prepared as illustrated in Schemes VII and VIII, respectively. Compounds of Formula Ij can be prepared by displacement of leaving group U with sources of isocyanate, such as the sodium or potassium salt of isocyanate. For example, to a salt of isocyanate, such as potassium isocyanate, in an inert solvent, such as dichloromethane, at a temperature of 20° to 150°C, a compound of Formula 18 is added, with or without the use of a catalytic amount of crown ether, such as 18-crown-6 ether, or a phase transfer reagent, such as tetra-n-butylammonium iodide. When the reaction is complete, the solvent is removed and the residue is purified to yield an isocyanate of Formula Ij. (J. Geordeler, R. Richter, *Synthesis*, **1978**, 760)

Similarly, the isothiocyanate compounds of Formula Ik can be prepared by displacement of the leaving group U with sources of isothiocyanate such as the ammonium or mercuric salts of isothiocyanate. For example, to a stirred solution of a salt of thiocyanate, such as mercuric thiocyanate, in an inert solvent, such as carbon tetrachloride, at a temperature of 0° to 100°C, a compound of Formula 18 is added. When the reaction is complete, the reaction mixture is filtered and solvent is removed to provide, after purification, a compound of Formula Ik. See, for example, G. Barnikow, W. Abraham, *Z. Chem*., **1970**, *10*, 193; J. Goerdeler et al., *Ber. Deutsch. Chem. Ges*., **1975**, *108*, 3071; G. Barnikow et al., *Z. Chem*., **1980**, *20*, 55.

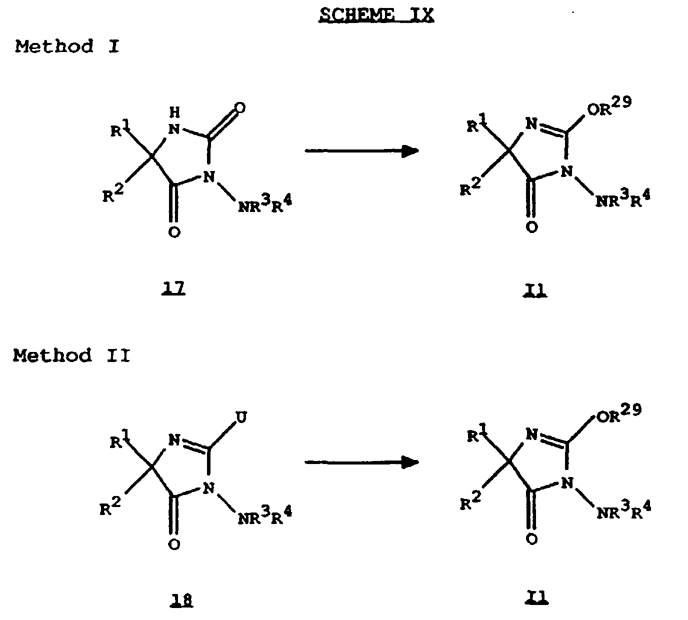

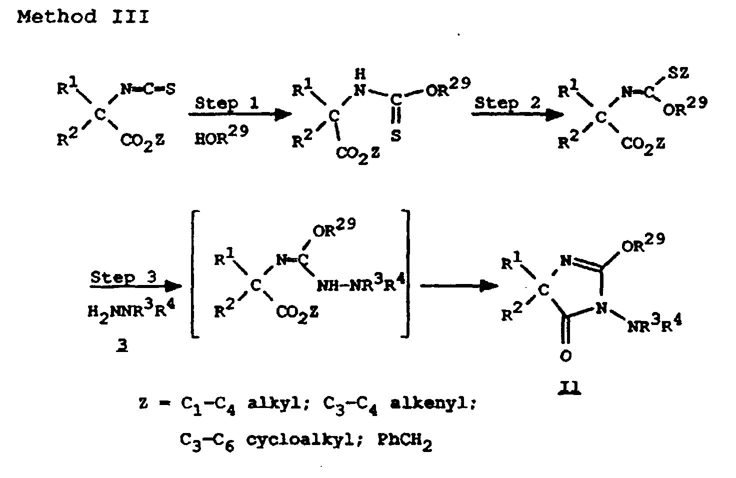

As illustrated in Method I, Scheme IX, 4(H)-imidazol-4-ones of Formula Il can be prepared by reaction of compounds of Formula 17 with reactive electrophiles such as alkyl sulfates (R. Menezes, M. B. Smith, *Synth. Commun*., 1988, *18*, 1625; G. H. Rasmusson et al., *J. Med. Chem*., **1984**, *27*, 1690); alkyl halides (D. Libermann et al., *C. R. Acad. Sci*., **1953**, *237*, 338; S. K. Sihka et al., *Indian J. Chem. B,* **1985**, *24*, 1035; G. Klein et al., *Helv. Chim. Acta,* **1955**, *38*, 1412); alkyl sulfonates (W. Ried, E. Schmidt, *Liebigs Ann. Chem*., **1965**, *676*, 114, J. A. Warshaw et al., *J. Org. Chem*., **1989**, *54*, 1736); activated carbonyl compounds, such as carboxylic acid halides (R. R. Koganty, G A. Digenis, *J. Labelled Compounds*, **1974**, *10*, 419; M. Miyake at al., *Synth. Commun*., **1984**, *14*, 353), isocyanates (W. Logel, K-H. Pook, *Chem. Rev*., **1986**, *119*, 2553); or diazoalkanes (H. Nishiyama et al., *Tetrahedron Lett*., **1979**, 4671).

In addition, compounds of Formula Il can be prepared as illustrated in Method II, Scheme IX by displacing the group U in compounds of Formula 18 with nucleophiles. For example, to a stirred solution of a salt of an alcohol, such as sodium ethoxide, in the corresponding alcohol, in this case, ethanol, or in an inert solvent, such as dimethylformamide, a compound of Formula 18 is added. The reaction is carried out at a temperture of 20° to 200°C. When the reaction is complete, the solvent is removed and the residue purified to provide a compound of Formula Il. (E. C. Tayler, F. Koenzle, *J. Org. Chem*., **1971**, *36*, 233; S. E. Forman et al., *J. Org. Chem*., **1963**, *28*, 2653; P. A. Wade et al., *J. Org. Chem*., **1983**, *48*, 1796; M. Mori, *Synthesis*, **1987**, 278).

In addition, compounds of Formula Il can be prepared by the methods illustrated in Method III, Scheme IX. The isocyanates of Formula 2 can be condensed with alcohols of Formula HOR²⁹ using literature procedures to form the corresponding thionocarbamates (E. Campaign, P. K. Nargund, *J. Org. Chem*., **1964**, *29*, 224; G. S. Skinner, H. C. Vogt, *J. Amer. Chem. Soc*., **1955**, *77*, 5440). The thionocarbamates can be alkylated as illustrated in Step 2 using known methods (R. Gompper, *Ber. Deutsch. Chem. Ges*., **1956**, *89*, 762; M. Kulka, *Can. J. Chem*., **1982**, *47*, 1985). Compounds of Formula Il can be prepared from treatment of the alkylated thionocarbamate with hydrazines of Formula 3 as illustrated in Step 3. In some instances, the intermediate acyclic compound can be isolated and cyclized in a separate step. Example 3 below describes this synthetic sequence in detail.

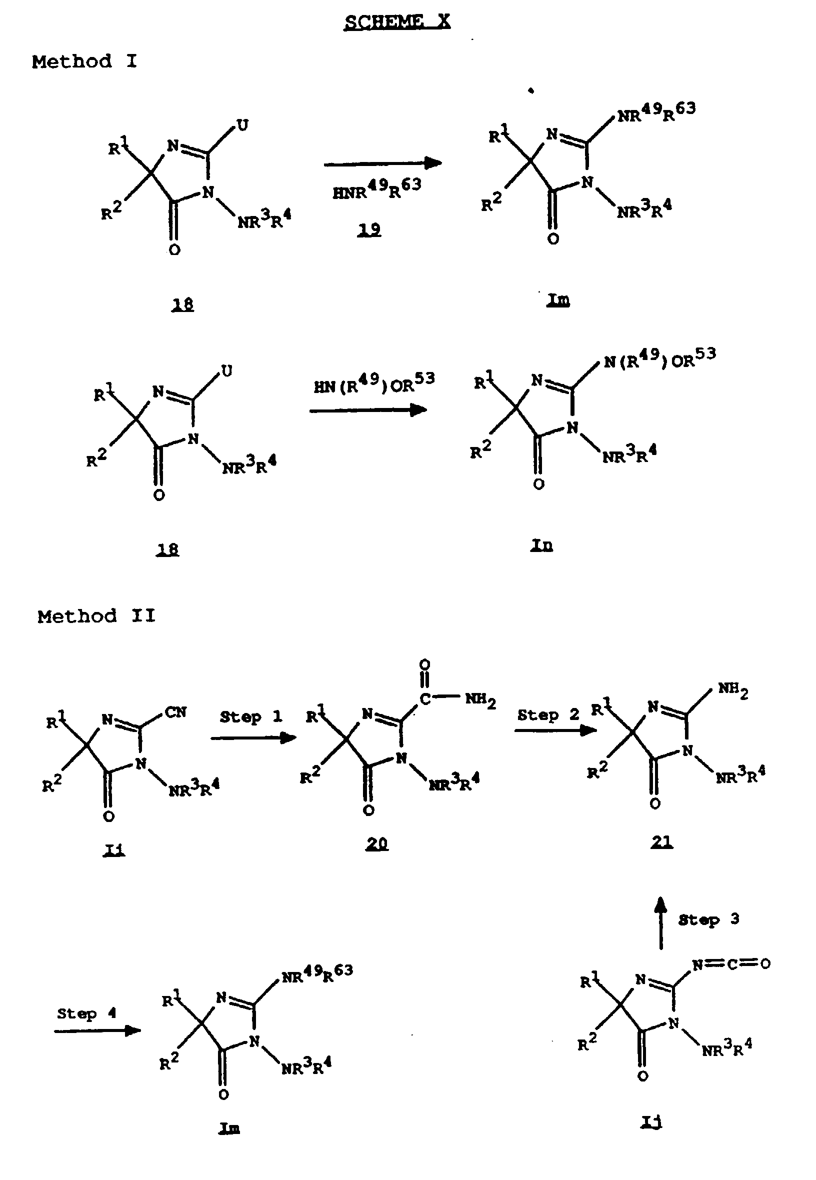

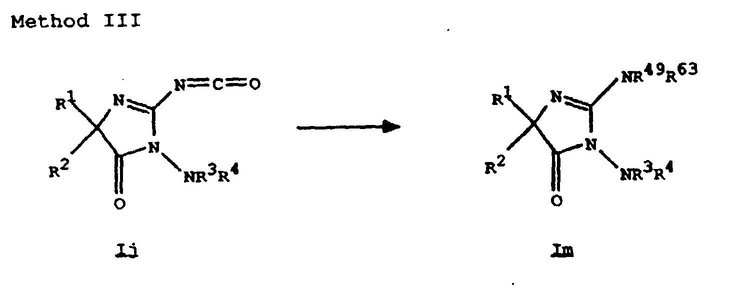

The compounds of Formula Im can be prepared by one or more of the methods illustrated in Scheme X. One method of preparing compounds of Formula Im involves the displacement of group U in compounds of Formula 18 with compounds containing a nitrogen bearing at least one hydrogen (Method I) such as ammonia and its salts (A. V. N. Reddy et al., *Synthesis*, **1986**, 864; A. Yamazaki et al., *J. Org. Chem*., **1967**, *32*, 3032, 3258; T. Ravindranathan et al., *Org. Prep. Proced*. *Int*., **1986**, *18*, 95); primary amines of Formula 19 (M. J. S. Dewar, *J. Chem. Soc*., **1944**, 534; L. Fishbein, J. A. Gallaghan, *J. Am. Chem. Soc*., **1954**, *76*, 1877); and secondary amines of Formula 19 (S. R. Aspinall, E. J. Bianco, *J. Am. Chem. Soc*., **1951**, *73*, 602; ibid., **1957**, *79*, 2199). For example, to a stirred solution of a compound of Formula 18 in an inert solvent such as chloroform, at a temperature of 0° to 150°C, an amino compound of Formula 19 is added. When the reaction is complete, solvent is removed and the residue thus obtained is purified to give a compound of Formula Im. Compounds of Formula In (Method I) can be prepared in a similar manner by treating compounds of Formula 18 with hydroxylamines of the formula HN(R⁴⁹)OR⁵³ or their salts. For example, to a stirred solution of a compound of Formula 18 in an inert solvent such as tetrahydrofuran, at a temperature of 0° to 100°C, a hydroxylamine or its salt is added, followed by a base, such as triethylamine. When the reaction is complete, the reaction mixture is poured into water and extracted with an organic solvent. The combined organic extracts are washed with water and the solvent is removed to yield, after purification, a compound of Formula In. (L. H. Briggs et al., *Aust. J. Chem*., **1976**, *29*, 357; R. A. Long et al., *Biochim. Biophys. Acta*, **1969**, *195*, 584) The primary and secondary amines of Formula 19 can be prepared by literature methods [C. A. Buehler, D. E. Pearson; Survey of Organic Synthesis, Vol. **9**, p 391, John Wiley and Sons (1977)]. The hydroxylamines of the formula HN(R⁴⁹)OR⁵³ or their salts can also be prepared by literature methods [S. Sandler, W. Karo, Org. Functional Group Preparation, Vol. III, p 322, Academic Press (1972)].

Alternatively, compounds of Formula Im can be prepared as illustrated in Method II, Scheme X. The compounds of Formula 21 can be prepared by one or both of the procedures shown. Compounds of Formula Ii (for the preparation of Ii, see Scheme VI) can be hydrolyzed (Step 1) by literature methods to amides of Formula 20 [C. A. Buehler, D. E. Pearson, Survey of Organic Synthesis, Vol. **2**, 813, John Wiley and Sons (1977); H. O. Larson et al., *J. Org. Chem*., **1969**, *34*, 525] which in turn can be converted (Step 2) to compounds of Formula 21 by methods described in the literature (R. C. Jones, *J. Am. Chem. Soc*., **1951**, *73*, 5610; J. Buchi et al., *Helv. Chim. Acta*, **1949**, *32*, 1806; F. J. McCarty et al., *J. Med. Chem*., **1970**, *13*, 814). Another way to prepare compounds of Formula 21 is to hydrolyze (Step 3) compounds of Formula Ij using procedures known in the literature (J. Weinstock, *J. Org. Chem*., **1961**, *26*, 3511; A. Hassner, C. Heatheock, *Tetrahedron*, **1964**, *20*, 1037). Compounds of Formula Im can be prepared from compounds of Formula 21 by derivatization of the amino group at the 2-position of the 4H-imidazol-4-ones of Formula 21 (Step 4) with reactions such as alkylations (A. M. Monro, *Chem. Ind*., **1964**, 1806; P. Nesbitt, P. Sykes, *J. Chem. Soc*., **1954**, 3057; P. Molina at al., *Chem. Ber*., **1988**, *121*, 1495), acylations (M. Van Montagu et al., *Bull. Soc. Chim*. *Belg*., **1968**, *77*, 171; M. S. Newman, V. Lee, *J. Org*. *Chem*., **1975**, *40*, 381), sulfonylations (H. J. Barker, *J. Chem. Soc*., **1943**, 101; N. T. Markiewicz et al., *Nucleos. Nucleot*., 1987, *6*, 769), carbamoylations (G. Schwenker, R. Kolb, *Tetrahedron*, **1969**, *25*, 5437; T. L. Hough et al., J. *Heterocycl. Chem*., **1986**, *23*, 1125), reactions with imidoyl chlorides (F. Findeisen, *Synthesis*, **1972**, 599; O. Tsuge at al., *J. Org. Chem*., **1974**, *39*, 1226), reactions with alkyl chloroformates (W Hunt, E. C. Wagner, *J. Org. Chem*., **1951**, *16*, 1792; D. R. Shridhar at al., *Indian J. Chem*., **1980**, *19*, 699; M. Meldal, J. W. Kindtler, *Acta Chem. Scand. B*, **1986**, *40*, 235); or a combination of these reactions.

In addition, compounds of Formula Im, wherein R⁶³ is C(=O)OR⁵³ and C(=O)NR⁵³R⁵⁶, can be prepared by literature procedures from isocyanates of Formula Ij (see Scheme VII for the preparation of Ij) by treatment with alcohols (S. R. Sandler and W. Karo, Organic Functional Group Preparations, Vol. II, p 223, Academic Press, Inc. (1971)) or amines (S. R. Sandler and W. Karo, Organic Functional Group Preparations, Vol. II, p 135, Academic Press, Inc. (1971)), respectively.

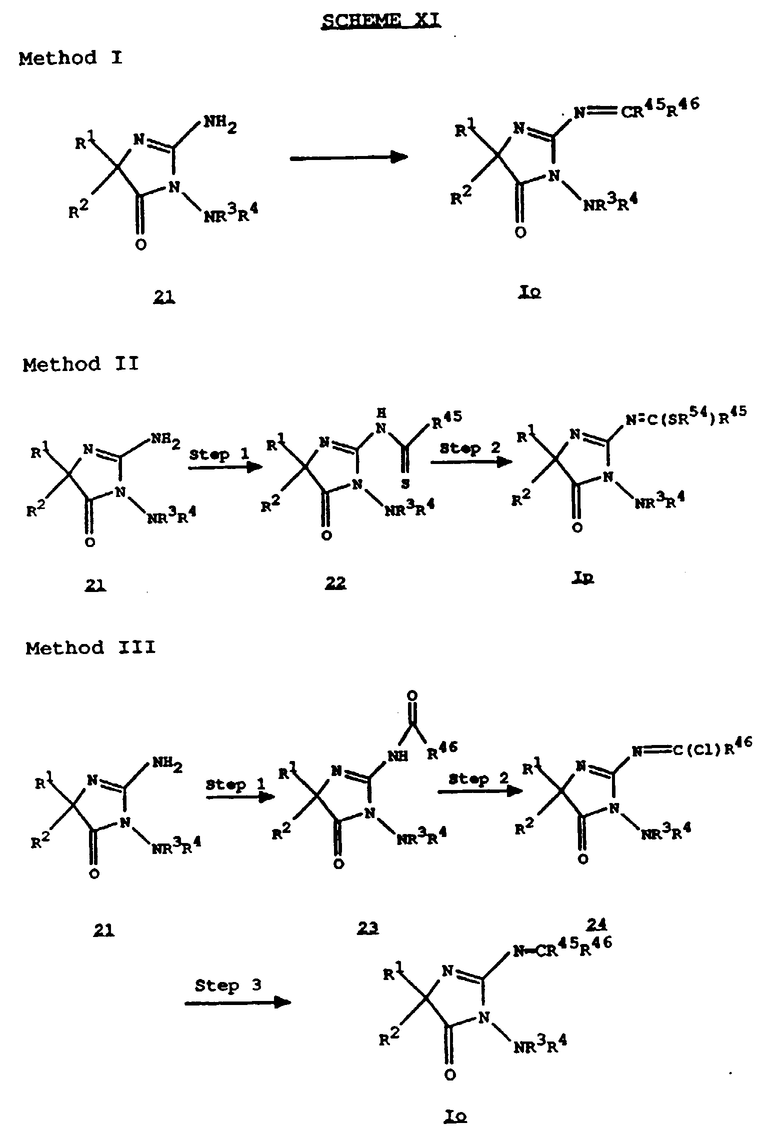

The compounds of Formula Io can be prepared by one or more of the methods illustrated in Scheme XI. As shown in Method I, the compounds of Formula Io can be obtained from amino compounds of Formula 21 by condensation with carbonyl compounds of formula R⁴⁵R⁴⁶C=O (H. Van Der Poel, G. Van Koten, *Synth*. *Commun*., **1978**, *8*, 305; M. Chaykowsky et al., *J. Heterocycl. Chem*., **1977**, *14*, 661), dialkyl acetals of amides of formula R⁴⁵C(OMe)₂NR⁵⁶R⁶⁴ to form compounds of Formula Io wherein R⁴⁶=NR⁵⁶R⁶⁴ (J. J. Fitt, H. W. Gischwend, *J. Org. Chem*., **1977**, *42*, 2639; J. Zemlicka et al., *Collect. Czech, Chem. Commun*., **1966**, *31*, 2198), or ortho-esters of carboxylic acids of formula R⁴⁵C(OR⁶⁵)₃ to form compounds of Formula Io wherein R⁴⁶=OR⁶⁵. For example, an amino compound of Formula 21 is stirred with an ortho-ester, with or without the use of solvent, at a temperature of 30° to 200°C. When the reaction is complete, the reaction mixture is purified to provide a compound of Formula Io. (R. A. Crochet, Jr., C. D. Blanton, Jr., *Synthesis*, **1974**, 55; F. M. F. Chen, N. L. Benoiton, *Can. J. Chem*., **1977**, *55*, 1433). The carbonyl compounds, the ortho-esters, and the dialkyl acetals of amides can be prepared by literature procedures. For carbonyl compounds: C. A. Buehler, D. E. Pearson, Survey of Organic Synthesis, Vol. **2**, pp 480, 532; John Wiley and Sons (1977); for ortho-esters: ibid., p 711; for dialkyl acetals of amides: W. Kantlehner, P. Speh., *Ber. Deutsch. Chem. Ges*., **1972**, *105*, 1340; G. Ege, H. O. Frey, *Tetrahedron Lett*., **1982**, 4217; S. Hanessian, E. Moralioglu, *Can. J. Chem*., **1972**, *50*, 233; T. Oishi et al., *Chem. Pharm. Bull*., **1969**, *17*, 2314).

The compounds of Formula Ip can be prepared using the procedure illustrated in Method II, Scheme XI. The compounds of Formula 22 can be prepared from the amino compounds of Formula 21 by literature methods (Step 1, G. C. Barrett, C. M. O. A. Martins, *J. Chem. Soc. Chem*. *Commun*., **1972**, 698; W. Walter, M. Radke, *Liebigs Ann*. *Chem*., **1970**, *739*, 201; P. Schlack, G. Keil, *Liebigs Ann. Chem*., **1963**, *661*, 164; W. Reid, W. Vond Der Emden, *Liebigs Ann. Chem*., **1961**, *642*, 128). Compounds of Formula Ip can be obtained from compounds of Formula 22 by known methods. For example, to a stirred solution of a compound of Formula 22 in an inert solvent, such as dichloromethane, at a temperature of 20° to 100°C, an alkyl halide, such as iodomethane is added. When the reaction is complete, the solvent is removed to provide, after purification, a compound of Formula Ip. (Step 2, H. Nishiyama et al., *Tetrahedron Lett*., **1979**, 4405; M. Bercot-Vatteroni, *Liebigs Ann. Chem*., **1962**, *7*, 312; A. A. Goldberg, W. Kelly, *J. Chem. Soc*., **1948**, 1919).

Another method for the preparation of compounds of Formula Io is illustrated in Method III, Scheme XI. Following literature methods, the compounds of Formula 23 can be prepared from compounds of Formula 21 (Step 1). For R⁴⁶C(=O)N as amides: S. Sandler, W. Karo, Organic Functional Group Preparations (Second Edition), Vol. **1**, p 316, Academic Press (1983); for R⁴⁶C(=O)N as carbamates: ibid., (First Edition), Vol **2**, p 223, Academic Press (1971); for R⁴⁶C(=O)N as ureas: ibid., (First Edition), Vol. **2**, p 135, Academic Press (1971); for R⁴⁶C(=O)N as thiolcarbamate: A. Hajos, *Ber. Deutsch. Chem. Ges*., **1961**, *94*, 2350; G. Harris, I. C. MacWilliam, *J. Chem. Soc*., **1961**, 2053. Compounds of Formula 24 can be prepared from compounds of Formula 23 (Step 2) by literature methods (R. Appel et al., *Ber. Deutsch. Chem. Ges*., **1974**, *107*, 698; K. Fijimoto et al., *Chem. Ind*., **1971**, 175; W. Reid, R. Christ, *Liebigs Ann. Chem*., **1980**, 693). The compounds of Formula Io can be obtained from compounds of Formula 24 according to literature methods with the use of reagents such as sulfur nucleophiles (P. Wolkoff et al., *Can. J. Chem*., **1974**, *52*, 879; M. Grdinic, V. Hahn, *J. Org. Chem*., **1965**, *30*, 2381; J. H. Davies et al., *J. Chem. Soc., C*, **1968**, 431), nitrogen nucleophiles (I. Matsuda et al., *J. Chem. Soc., Perkin Trans. I*, **1976**, 1528; S. Yanagida et al., *Bull. Chem*. *Soc. Jpn*., **1971**, *44*, 2182; E. Gedlovska et al., *Collect. Czech. Chem. Commun*., **1976**, *41*, 3085), oxygen nucleophiles (S. Knapp, D. V. Patel, *Tetrahedron Lett*., **1982**, *23*, 3539; J. E. Rowe, *Synthesis*, **1980**, 114; L. I. Samarai et al., *Zh. Org. Khim*., **1987**, *23*, 455), or carbon nucleophiles (H. Quast et al., *Liebigs Ann*. *Chem*., **1979**, *83*; N. G. Clark, E. Cawkill, *Tetrahedron Lett*., **1975**, 2717; W. Reid, P. Weidemann, *Ber. Deutsch. Chem. Ges*., **1971**, *104*, 3329).

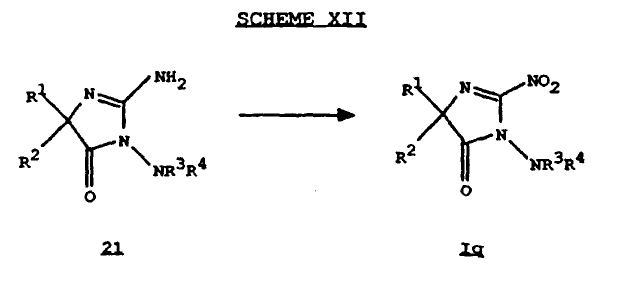

The nitro compounds of Formula Iq can be prepared from compounds of Formula 21 by oxidation methods known in the literature using oxidizing reagents such as percarboxylic acids. For example, to a solution of an oxidant, such as meta-chloroperbenzoic acid in an inert organic solvent, such as chloroform, an amino compound of Formula 21 is added. The reaction is maintained at a temperature of 20° to 100°C. When the reaction is complete, the reaction mixture is sequentially washed with sodium thiosulfate aqueous solution, sodium bicarbonate aqueous solution, and water. The solvent is then removed to provide, after purification, a nitro compound of Formula Iq. (C. H. Robinson, L. Milewich, P. Hoffer, *J. Org. Chem*., **1966**, *31*, 524; E. Keinan, Y. Mazur, *J. Org., Chem*., **1977**, *42*, 844; Y. Yost, *J. Med. Chem*., **1969**, *12*, 961).

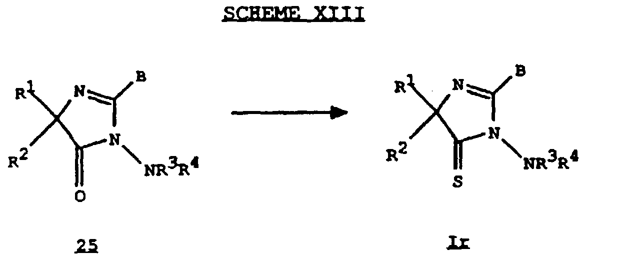

Compounds of Formula Ir can be prepared from compounds of Formula 25, as illustrated in Scheme XIII, following literature methods used to prepare thioamides and thioureas from the corresponding amides and ureas, respectively, using reagents such as sulfur, P₄S₁₀, or Lawesson's reagent. For example, to a solution of a compound of Formula 25 in an inert organic solvent such as benzene or acetonitrile, a solution of a sulfurizing reagent, such as P₄S₁₀ in the same solvent is added, followed by an inorganic salt, such as sodium bicarbonate. The reaction temperature is maintained at 0° to 100°C. When the reaction is complete, an organic solvent, such as diethyl ether is added and the mixture is washed with a basic aqueous solution, such as sodium bicarbonate and then water. The solvent is removed to give, after purification, a compound of Formula Ir. (H. Alper et al., *Angew. Chem., Int. Ed. Eng*., **1978**, *17*, 689; J. Perregaard et al., *Bull. Soc. Chim. Belg.,* **1977**, *86*, 321; J. Voss, *Liebigs Ann. Chem*., **1971**, *746*, 92; T. Nishio et al., *Synthesis*, **1989**, 396; J. M. Kane, *Synthesis*, **1987**, 912).

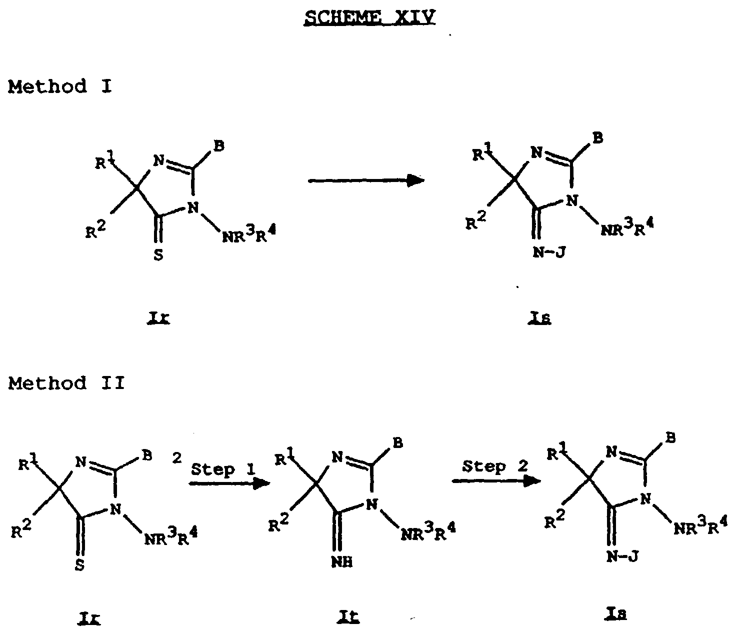

Compounds of Formula Is can be obtained by one or both of the methods illustrated in Scheme XIV. As shown in Method I, compounds of Formula Is can be obtained from compounds of Formula Ir with the use of reagents containing a NH₂ group such as a primary amine or amides (P. N. Bhargava, V. N. Choubey, *Curr. Sci.,* **1968**, *37*, 645; M. E. Baguley, J. A. Elvidge, *J. Chem. Soc*., **1957**, 709; F. Micheel, W. Brunkhorst, *Ber. Deutsch. Chem. Ges*., **1955**, *88*, 481), or hydroxylamines (V. Cerneckij et al., *Collect. Czech. Chem, Commun.,* **1962**, *27*, 87; R. Hazard, *Bull. Soc. Chim. Fr*., **1949**, 228; Sanwa K. K., EP 268,245; J. D. Brion et al., *Synthesis*, **1983**, 220). The amines, amides, and hydroxylamines can be prepared by literature methods. [For amines: G. A. Buehler, D. E. Pearson, Survey of Organic Synthesis, Vol. **2**, p 391, John Wiley and Sons (1977); for amides, ibid., p 813; for hydroxylamines (S. Sandler, W. Karo, Org. Functional Group Preparations, Vol. III, p 322, Academic Press, (1972)].

Compounds of Formula Is can also be prepared as illustrated in Method II, Scheme XIV. Compounds of Formula It can be obtained from compounds of Formula Ir by treatment with sources of ammonia. For example, to a stirred solution of a compound of Formula Ir in an inert solvent such as dichloromethane, a source of ammonia, such as ammonia gas or ammonium chloride, is added at a temperature of -20° to 50°C. When the reaction is complete, the solvent is removed to provide, after purification, a compound of Formula It. (Step 1, G. S. Skinner, H. C. Vogt, *J. Am. Chem. Soc*., **1955**, *77*, 5440; F. H. S. Curd et al., *J. Chem. Soc*., **1948**, 586; J. S. Morley, J. C. E. Simpson, *J. Chem. Soc*., **1952**, 2617) Compounds of Formula It can be converted to compounds of Formula Is by derivatizing the imino nitrogen. For example, an imino compound of Formula It and an electrophilic reagent such as an acyl chloride are combined and stirred in an inert organic solvent such as chloroform, at a temperature of 0° to 100°C, with or without the use of base, such as pyridine. When the reaction is complete, the reaction mixture is poured into water and extracted with an organic solvent. The solvent is removed to provide, after purification, a compound of Formula Is. (Step 2, J. P. Lokensgard et al., *J. Org. Chem*., **1985**, *50*, 5609; R. Kupfer et al., *Chem. Ber*., **1985**, *118*, 3089; R. Allmann et al., *Chem. Ber*., **1986**, *119*, 2444; J. P. Chetia et al., *Synthesis*, **1985**, 83; F. Matsuda et al., *Tetrahdedron Lett*., **1982**, *23*, 4043)

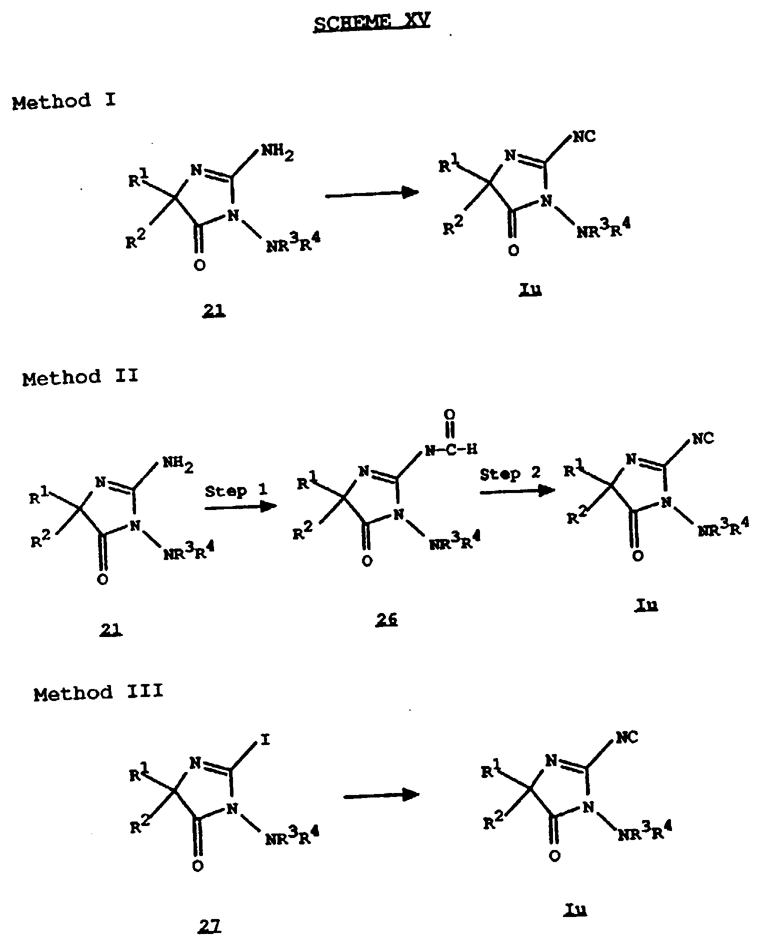

The isonitriles of Formula Iu can be prepared by one or more of the methods illustrated in Scheme XV. Thus, the amino compounds of Formula 21 can be converted to isonitriles of Formula Iu by treatment with dihalocarbenes (Method I). (L. Malatesta, *Gazz. Chim*., **1947**, *77*, 238; S. R. Sandler and W. Karo, Organic Functional Group Preparations, (second edition) Vol. III, p 206, Academic Press, Inc. (1989)). Alternatively, compounds of Formula Iu can be prepared as illustrated in Method II. The formamides of Formula 26 prepared from amino compounds of Formula 21 (Step 1) can be converted to compounds of Formula Iu (Step 2). For example, a compound of Formula 26 and a electrophilic reagent that can serve as a dehydration reagent, such as toluenesulfonyl chloride, and a base, such as pyridine, are mixed at a temperature of 0° to 100°C, with or without the use of an inert organic solvent, such as dichloromethane. When the reaction is complete, the reaction mixture is added to water and extracted with an organic solvent. The combined organic extracts are washed with water. The solvent is removed to provide, after purification, a compound of Formula Iu. This process is well documented in the literature (I. Ugi et al., *Chem. Ber*., **1961**, *94*, 2814; W. R. Hertler and E. J. Corey, *J. Org. Chem*., **1958**, *23*, 1221; R. Obrecht et al., *Synthesis*, **1985**, 400). In addition, compounds of Formula Iu can be prepared from iodo compounds of Formula 27 (Method III) by literature methods with the use of heavy metal cyanides (S. R. Sandler and W. Karo, Organic Functional Group Preparations (second edition), Vol. III, p 206, Academic Press, Inc.; E. G. J. Hartley, *J. Chem. Soc*., **1916**, *109*, 1926). The preparation of the iodo compounds of Formula 17 is described in Scheme V.

### EXAMPLE 1

### Preparation of 3,5-dihydro-5-methyl-2-methylthio-5-phenyl-3-(phenylamino)-4H-imidazol-4-one

To a solution of 5-methyl-5-phenyl-3-phenylamino-2-thioxo-4-imidazolidinone (0.1 g, 0.34 mmol) in dichloromethane (10 mL), iodomethane (0.25 g, 1.75 mmol) and triethylamine (0.18 mL, 1.8 mmol) were added. The mixture was stirred for 48 h, poured into water (10 mL), and extracted with ethyl acetate (three times with 25 mL). The organic extracts were combined, washed with water, dried (MgSO₄), and filtered, and the solvent was removed *in vacuo* to give the title compound as a slightly yellow solid, (95 mg, 90%), m.p. 132-134°C; ¹H NMR (CDCl₃): δ 1.77 (s, 3H), 2.61 (s, 3H), 6.15 (s, 1H), 6.70-7.62 (m, 10H)

### EXAMPLE 2

### Preparation of 3,5-dihydro-5-methyl-5-phenyl-3-(phenylamino)-4H-imidazol-4-one

To a solution of 5-methyl-5-phenyl-2-phenylamino-2-thioxo-4-imidazolidinone (0.2 g, 0.6 mmol) in acetone (6 mL), a solution of Oxone® (49.5% KHSO₅) (0.6 g, 4 mmol in 3.5 mL H₂O) was added. The reaction mixture was stirred vigorously for 4 min. Ethyl acetate (100 mL) and water (20 mL) were added. The mixture was neutralized with aqueous sodium bicarbonate solution. The organic layer was separated, dried (MgSO₄) and filtered, and the solvent was removed *in vacuo* to give an oil which was purified on a silica gel column eluting with ethyl acetate/hexane (1:1) to afford the title compound (0.13 g, 80%), as a white solid, m.p. 120-121°C; IR (CHCl₃): 1733 cm⁻¹; ¹H NMR (CDCl₃): δ 1.77 (s, 3H), 6.20 (s, 1H), 6.67-7.62 (m, 10H), 7.92 (s, 1H).

### EXAMPLE 3

### Preparation of 3,5-dihydro-2-methoxy-5-methyl-5-phenyl-3-(phenylamino)-4H-imidazol-4-one

To a solution of ethyl α-[(methoxythioxomethyl)amino]-α-methyl-benzeneacetate (0.2 g, 0.75 mmol) in dichloromethane (1 mL) was added iodomethane (0.5 g, 3.5 mmol) followed by the dropwise addition of 1,8-diazabicyclo[5.4.0]undec-7-ene (0.25 g, 1.6 mmol). The reaction mixture was stirred for 10 minutes, poured into water (10 mL), and extracted with ethyl acetate (3 times with 10 mL). The organic extracts were combined, washed with water, dried (MgSO₄), and filtered. The solvent was removed in vacuo to give 0.2 g of ethyl α-[[methoxy-(methylthio)methylene]amino]-α-methylbenzeneacetate (95% yield) as an oil. ¹H NMR (CDCl₃): δ 1.25 (t, J=7 Hz, 3H), 1.80 (s, 3H), 2.50 (s, 3H), 4.98 (s, 3H), 4.15 (q, J=7 Hz, 2H), 7.35-7.90 (m, 5H).

To a solution of ethyl α-[[methoxy(methylthio)methylene]amino]-α-methylbenzeneacetate (0.2 g, 0.71 mmol) in glacial acetic acid (1 mL) was added anhydrous phenylhydrazine (0.1 g, 0.93 mmol). The reaction mixture was stirred for 15 minutes, poured into aqueous sodium carbonate, and extracted with ethyl acetate. The ethyl acetate extracts were combined, washed with water, dried (MgSO₄), and filtered. The solvent was removed in vacuo to afford an oil which was purified by silica gel chromatography (ethyl acetate:hexanes = 1:2) to give two fractions. Fraction 1 was ethyl α-[[methoxy(2-phenyl-hydrazino)methylen]amino-α-methylbenzeneacetate (R_{f}=0.33, 40 mg, 16% yield). ¹H NMR (CDCl₃): δ 1.20 (t, J=6.7 Hz, 3H), 2.00 (s, 3H), 3.75 (s, 3H), 4.20 (q, J=6.7 Hz, 2H), 5.50 (bs, 1H), 6.75 (bs, 1H), 6.90-7.70 (m, 10H). Fraction 2 was the title compound (R_{f}=0.07, 10 mg, 4.5% yield). m.p. 110-111°C; ¹H NMR (CDCl₃): δ 1.78 (s, 3H), 4.13 (s, 3H), 6.01 (s, 1H), 6.69-7.67 (m, 10H).

Ethyl α-[[methoxy(2-phenylhydrazino)methylene]amino-α-methylbenzeneacetate was converted into the title compound by heating it for 30 seconds in a round-bottomed flask with a hot-air gun. The compound was purified by silica gel chromatography (ethyl acetate:hexane = 1:2). An additional 23 mg (3.3% yield) was obtained.

### EXAMPLE 4

### Preparation of 3,5-dihydro-5-methyl-2-methyltho-5-heptyl-3-(phenylamino)-4H-imidazol-4-one

To ethyl 2-[[bis(methylthio)methylene]amino]-2-methylnonanoate (4 g, 12.54 mmol) in acetic acid (100 mL) was added phenylhydrazine (1.6 g, 14.8 mmol) and the mixture was stirred for 5 h. The reaction mixture was then slowly poured into enough aqueous sodium carbonate to neutralize the acetic acid. The aqueous mixture was extracted with ethyl acetate. The ethyl acetate extracts were combined, washed with water, dried (MgSO₄), and filtered. The solvent was removed in vacuo to give an oil which was purified by silica gel chromatography (ethyl acetate:hexane = 1:2). The title compound (1.35 g, 32%) was obtained. m.p. 108-110°C, ¹H NMR (CDCl₃): δ 0.86 (t, J=2.4 Hz, 3H), 1.05-1.38 (m, 10H), 1.39 (s, 3H), 1.62 (m, 2H), 2.51 (s, 3H), 6.16 (s, 1H), 6.78-7.25 (m, 5H).

### EXAMPLE 5

### Preparation of 3,5-dihydro-2,5-dimethyl-5-phenyl-3-(phenylamino)-4H-imidazol-4-one

To ethyl α-(acetylamino)-α-methylbenzeneacetate (0.2 g, 0.85 mmol) in carbon tetrachloride (10 mL) was added phosphorus pentachloride (0.26 g, 1.27 mmol). The mixture was stirred and heated at reflux for 5 minutes. The solvent was removed in vacuo. The residue was redissolved in dichloromethane (2 mL) and added dropwise to a solution of phenylhydrazine (0.34 g, 3.15 mmol) in dichloromethane (10 mL). The reaction solution was stirred at room temperature for 24 h, poured into water, and extracted with ethyl acetate. The ethyl acetate extracts were combined, washed with water, dried (MgSO₄), and filtered. The solvent was then removed in vacuo to afford a solid which was recrystallized from 1-chlorobutane to give the title compound (0.15 g, 63.2%). m.p. 110-112°C, ¹H NMR (CDCl₃): δ 1.63 (s, 3H), 2.16 (s, 3H), 6.58-7.50 (m, 10H), 8.69 (s, 1H).

### EXAMPLE 6

### Preparation of 3,5-dihydro-2-methoxy-5-methyl-3-(methyl-phenylamino)-5-methyl-4H-imidazol-4-one

5-Methyl-5-phenyl-3-(phenylamino)-2,4-imidizolidine-dione(2.5 g, 8.88 mmol), iodomethane (14 mL), and silver oxide (2.6 g, 12.59 mmol) were combined and stirred at room temperature for 2 h. The reaction mixture was filtered, and the solvent was removed in vacuo. The residue obtained was stirred with ethyl acetate. The solid which did not dissolve was filtered off and further washed with ethyl acetate. The ethyl acetate filtrate and washes were combined and evaporated in vacuo to give a residue which was purified by silica gel chromatography (ethyl acetate:hexane = 1:2) to afford the title compound (0.2 g, 7.6% yield). m.p. 183-185°C; ¹H NMR (CDCl₃): δ 1.72 (s, 3H), 3.20 (s, 3H), 4.02 (s, 3H), 6.50-7.80 (m, 10H).

Additional compounds were prepared using the procedures exemplified above in Examples 1-6, and are listed in Index Table A hereinafter.

The compounds referred to in the Tables which follow are illustrated below. The groups R¹-R⁶⁹ and A, B, E, V, G, J, n, Q, T, and W are as defined in Formula I in the Summary of the Invention. Additional variables are used in the compounds and Tables and are:
- m,: which designates the size of a portion of some substituted R² alkyl groups, is 0-18;
- p,: which designates the size of a portion of other substituted R² alkyl groups, is 1-19;
- q and r: designate the length of some alkoxyalkyl R² groups. The sum of q and r is 2-20.

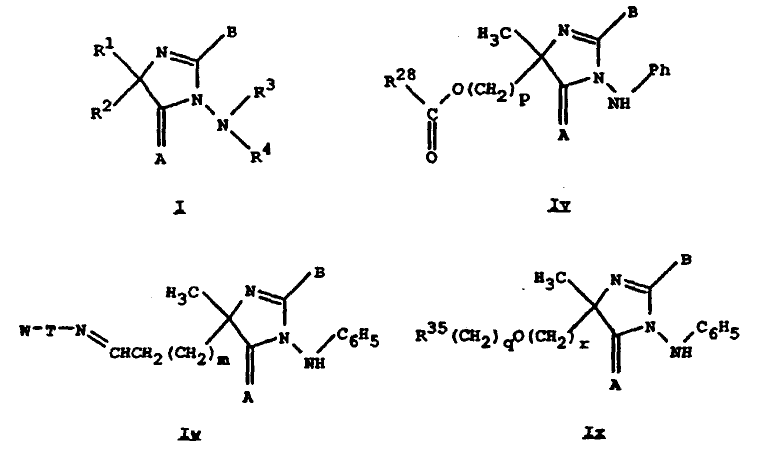

The following abbreviations are used in the Tables which follow. All alkyl groups are the normal isomers unless indicated otherwise.

| | | |
|---|---|---|
| Me = methyl | MeO = methoxy | *t* = tertiary |
| Et = ethyl | EtO = ethoxy | *s* = secondary |
| Ph = phenyl | EtS = ethylthio | *i* = iso |
| Bzl = benzyl | MeS = methylthio | CN = cyano |
| PhCH₂ = benzyl | PbS = phenyithio | *c* = cyclo |

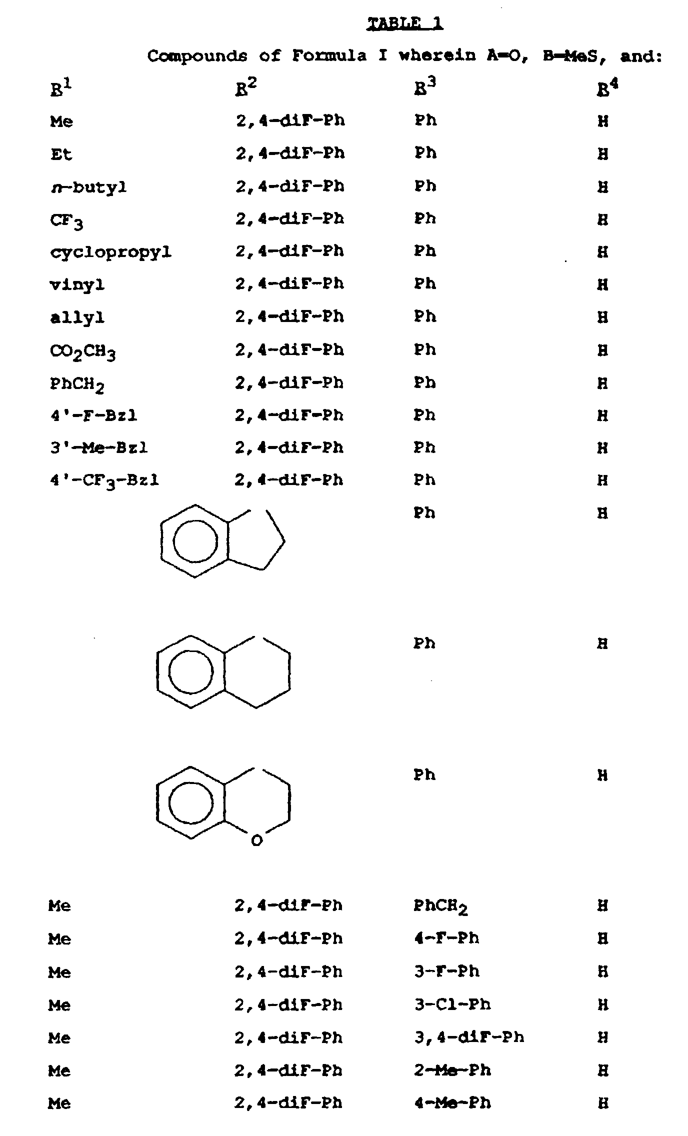

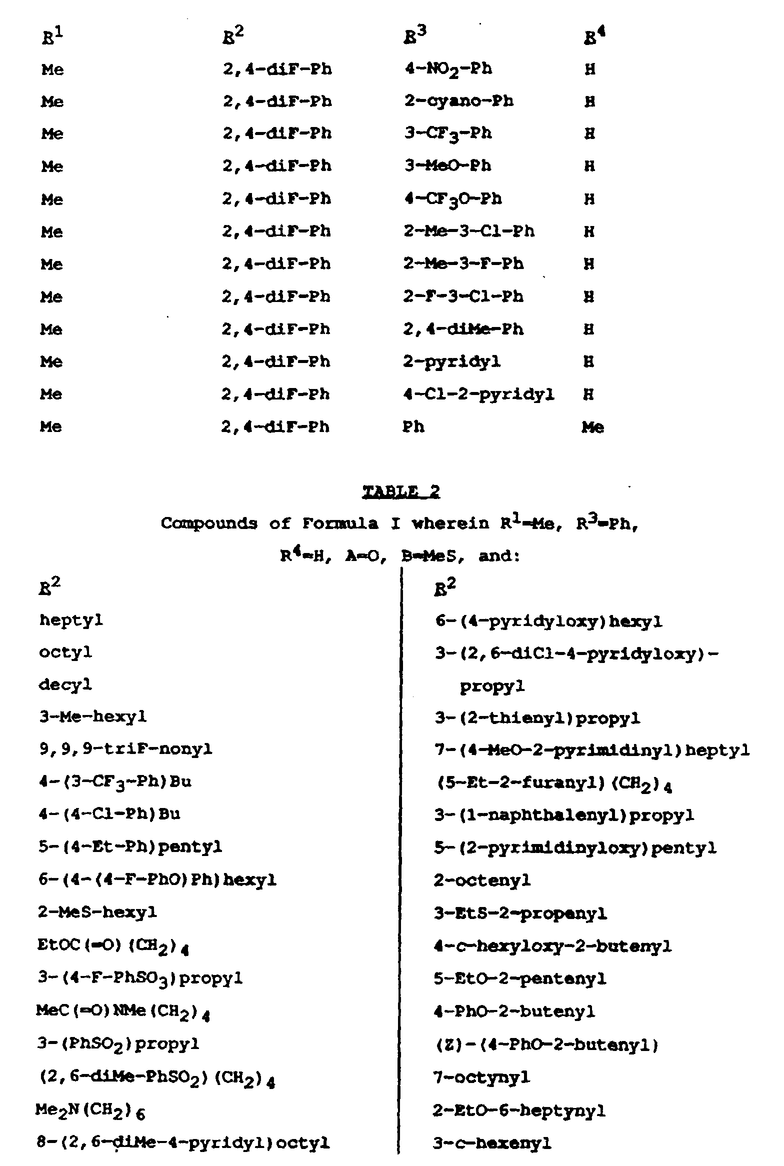

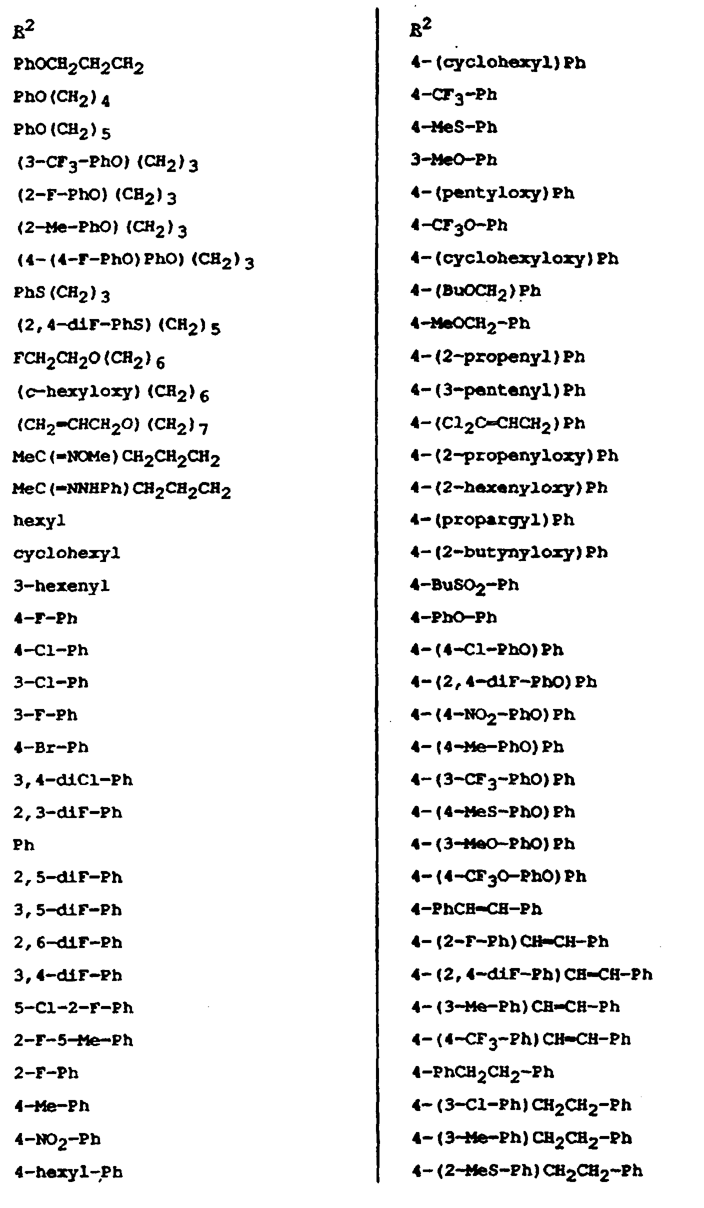

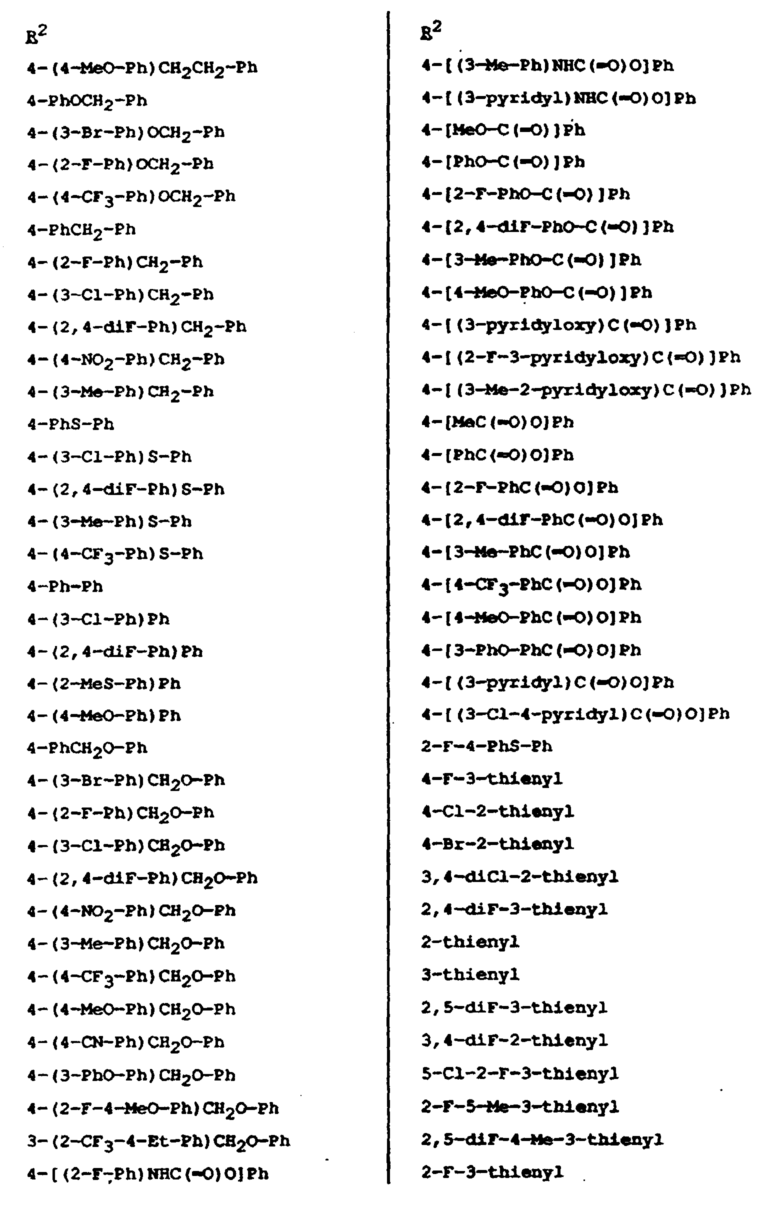

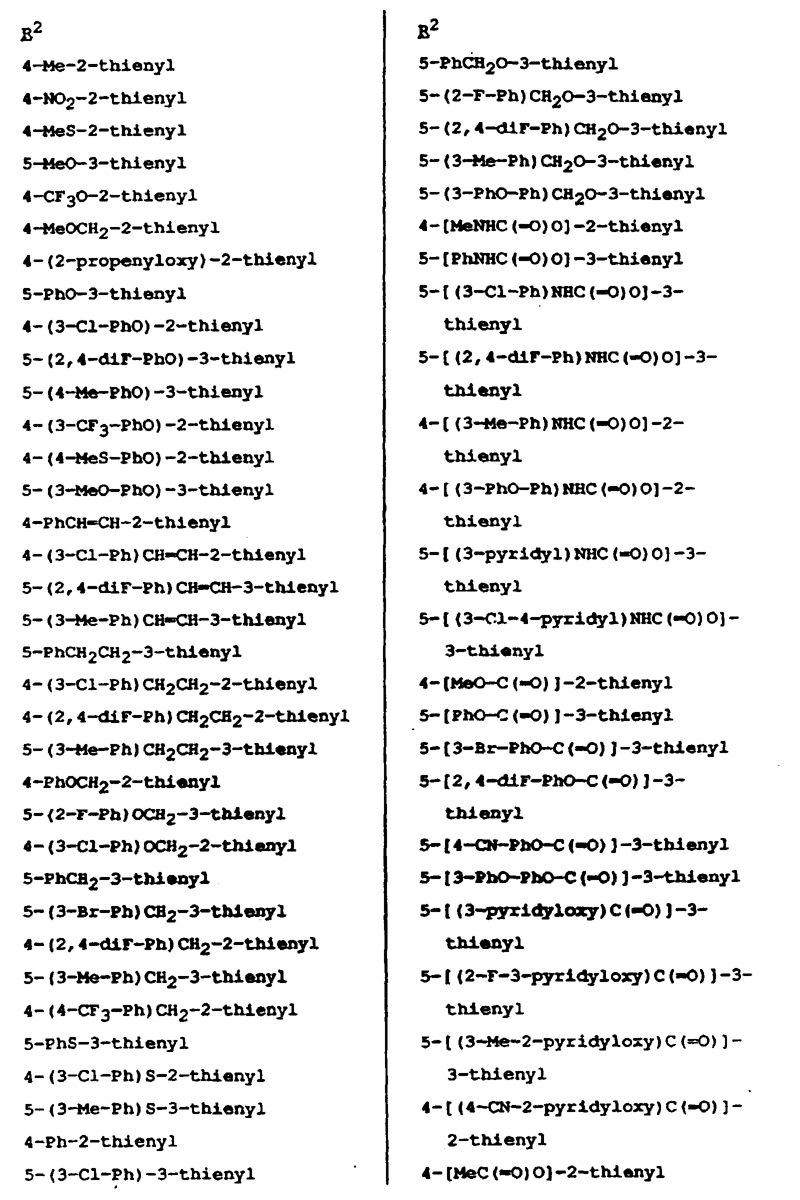

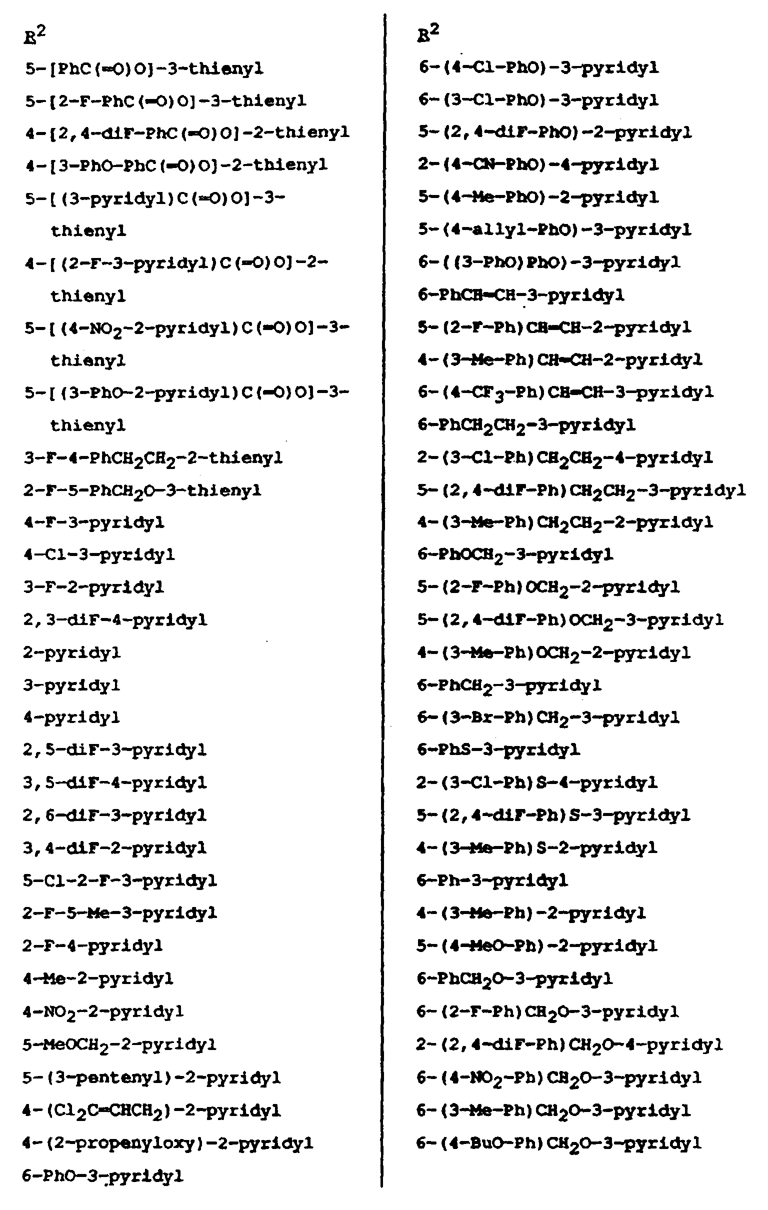

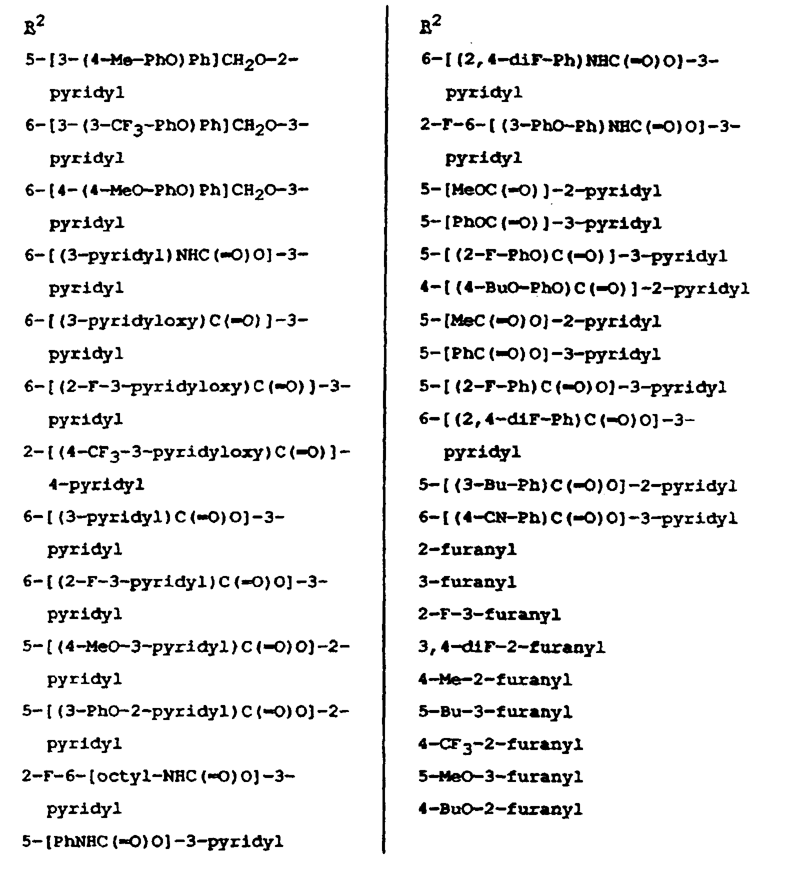

**TABLE 3**

| Compounds of Formula Iv wherein A=O, B=MeS, and: | | | |
|---|---|---|---|
| p | R²⁸ | p | R²⁸ |
| 5 | Me | 6 | 4-F-PhO |
| 6 | Ph | 8 | 4-Et-PhO |
| 8 | 4-CF₃-Ph | 2 | 4-EtO-PhO |
| 5 | 2,4-diCl-Ph | 1 | 4-(4-F-PhO)PhO |
| 1 | 4-F-Ph | 2 | 4-(3-Et-PhO)PhO |
| 2 | 4-Et-Ph | 3 | MeNH |
| 3 | 3-EtO-Ph | 4 | Me₂N |
| 4 | 4-(4-F-PhO)Ph | 1 | PhNH |
| 3 | 4-(3-Et-PhO)Ph | 5 | PhN(Me) |
| 5 | 3-(4-CF₃-PhO)Ph | 6 | 4-CF₃-PhNH |
| 8 | MeO | 2 | 3-F₃CO-PhNH |
| 6 | PhO | 3 | 2,4-diCl-PhNH |
| 2 | 4-F₃CO-PhO | 8 | 4-Et-PhNH |
| 4 | 3,5-diCl-PhO | | |

**TABLE 4**

| Compounds of Formula Iw wherein A=O, B=MeS, and: | | | | | |
|---|---|---|---|---|---|
| m | T | W | m | T | W |
| 1 | O | H | 2 | O | NHMeC(=O) |
| 4 | O | Et | 5 | O | NHPhC(=O) |
| 2 | O | Bzl | 4 | NH | Me |
| 3 | O | 4-Cl-Bzl | 1 | NH | Bzl |
| 5 | O | Ph | 8 | NH | Ph |
| 1 | O | 2,6-diF-Ph | 3 | NH | 3,5-diF-Ph |
| 6 | O | 4-F-Ph | 2 | NH | 4-pyridyl |
| 8 | O | 4-pyridyl | 1 | NH | EtC(=O) |
| 4 | O | MeC(=O) | 4 | NH | PhC(=O) |
| 2 | O | PhC(=O) | 6 | NH | MeOC(=O) |
| 1 | O | 3,5-diF-PhC(=O) | 3 | NH | PhOC(=O) |
| 3 | O | 3,5-MeO-PhC(=O) | 1 | NH | (3-pyridyl)OC(=O) |
| 6 | O | 4-pyridyl-C(=O) | 1 | NH | MeNHC(=O) |
| 4 | O | BuOC(=O) | 2 | N-Me | Me |
| 2 | O | PhOC(=O) | 5 | --- | H |
| 3 | O | (4-EtO-Ph)OC(=O) | 1 | --- | Me |
| 6 | O | (4-pyridyl)OC(=O) | 2 | --- | Ph |
| 3 | N-Me | Ph | | | |

**TABLE 5**

| Compounds of Formula Ix wherein A=O, B=MeS, and: | | | | | |
|---|---|---|---|---|---|
| r | q | R³⁵ | r | q | R³⁵ |
| 2 | 1 | MeS | 2 | 1 | *c*-hexyl |
| 3 | 1 | FCH₂CH₂O | 1 | 2 | *c*-pentyl |
| 1 | 1 | EtOC(=O) | 1 | 2 | EtO |
| 3 | 1 | MeC(=O)O | 1 | 1 | Ph |
| 2 | 2 | PhOC(=O)O | 2 | 2 | PhO |
| 1 | 3 | MeNHC(=O)O | 2 | 6 | H |
| 2 | 1 | PhNH | 2 | 8 | H |
| 2 | 3 | (MeO)₂P(=O)O | 1 | 7 | EtS |
| 1 | 2 | MeSO₃ | 3 | 5 | CF₃O |
| 3 | 2 | 4-pyridyl | 3 | 5 | *c*-hexyl |
| 1 | 3 | 4-pyridyloxy | 1 | 8 | Ph |
| 2 | 2 | 2-pyrimidyl | | | |

**TABLE 6**

| Compounds of Formula I wherein R¹=Me, R²=Ph, R³=Ph, R⁴=H, A=N-R¹⁵, B=MeS, and: | |
|---|---|
| R¹⁵ | R¹⁵ |
| H | H (H₃PO₄ salt) |
| H (HBr salt) | H (camphor sulfonic acid salt) |
| H (HCl salt) | Me |
| H (HI salt) | Me (HBr salt) |
| H (CF₃CO₂H salt) | Et |
| H (MeSO₃H salt) | Bu |
| H (PhSO₃H salt) | octyl |
| H (4-TsOH salt) | MeOCH₂CH₂ |
| H [(4-dodecyl-Ph)SO₃H salt] | cyclopropyl |
| allyl | 4-Br-Bzl |
| 2-octenyl | 4-Me-Bzl |
| propargyl | 4-CF₃-Bzl |
| Ph | 4-MeS-Bzl |
| 4-Cl-Ph | 4-MeO-Bzl |
| 3,5-diCl-Ph | 2-pyridyl |
| 2-F-Ph | 3-pyridyl |
| 3-NO₂-Ph | 5-Cl-2-pyridyl |
| 4-Me-Ph | 6-F-2-pyridyl |
| 4-Me-O-Ph | 6-Me-2-pyridyl |
| Bzl | 2-MeS-3-pyridyl |
| Bzl (HBr salt) | 2-MeO-3-pyridyl |
| 4-Cl-Bzl | |

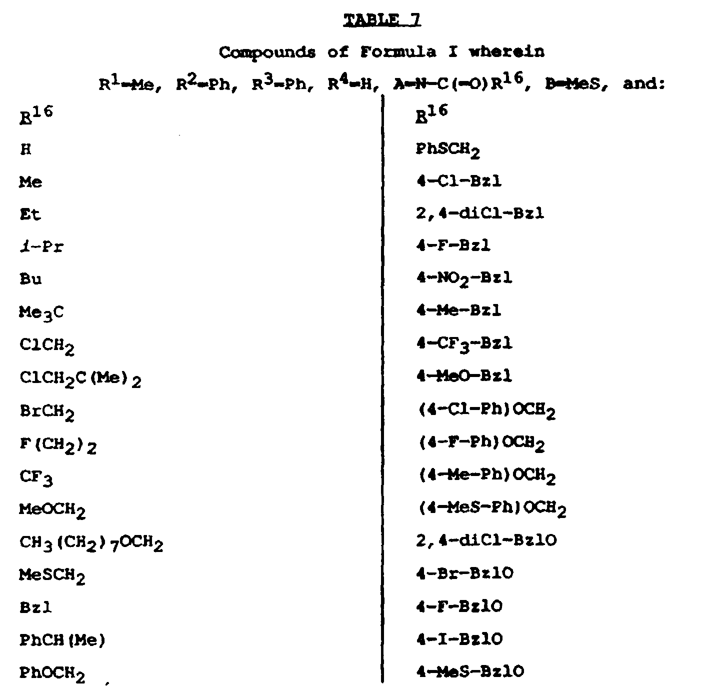

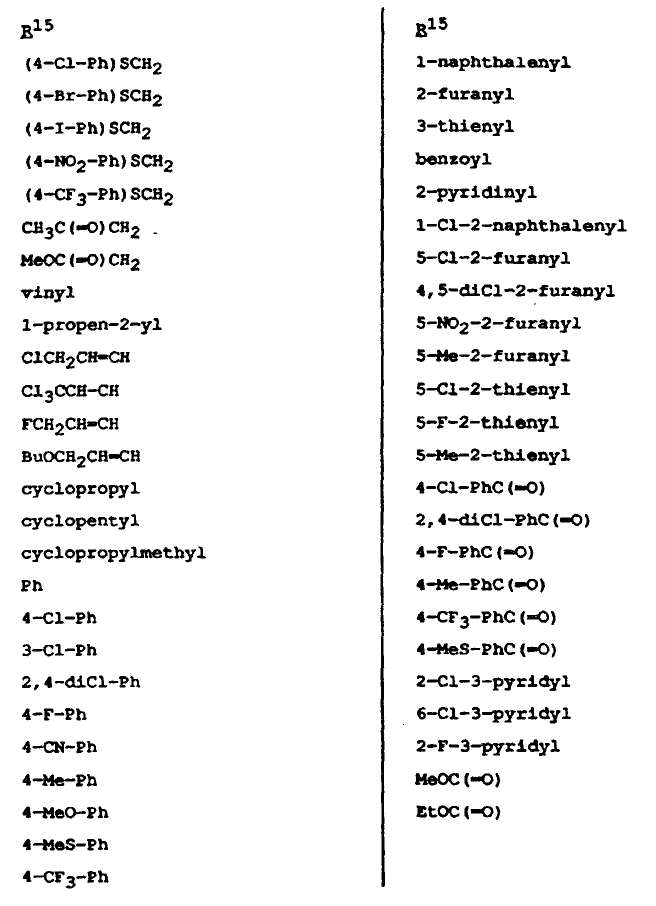

**TABLE 8**

| Compounds of Formula I wherein R¹=Me, R²=Ph, R³=Ph, R⁴=H, A=N-C(=O)OR¹⁷, B=MeS, and: | |
|---|---|
| R¹⁷ | R¹⁷ |
| Me | Cl₃CCH₂ |
| Et | CF₃CH₂ |
| Bu | MeOCH₂CH₂ |
| ClCH₂CH₂ | CH₃(CH₂)₁₁OCH₂CH₂ |
| MeSCH₂CH₂ | MeOCH₂CH=CHCH₂ |
| Bzl | BuOCH₂CH=CHCH₂ |
| 1-naphthalenylmethyl | HC≡CCH₂ |
| 2-naphthalenylmethyl | cyclopropyl |
| PhOCH₂CH₂ | cyclohexyl |
| 4-Cl-Bzl | 1-naphthalenyl |
| 2,4-diCl-Bzl | 2-naphthalenyl |
| 4-F-Bzl | 2-thienyl |
| 4-NO₂-Bzl | 4-Cl-Ph |
| 4-CF₃-Bzl | 2,4-diCl-Ph |
| 1-Cl-2-naphthalenylmethyl | 4-F-Ph |
| (2,4-diCl-Ph)OCH₂CH₂ | 4-NO₂-Ph |
| (4-F-Ph)OCH₂CH₂ | 4-CF₃-Ph |
| (4-Me-Ph)OCH₂CH₂ | 4-MeO-Ph |
| (4-CF₃-Ph)OCH₂CH₂ | 1-F-2-naphthalenyl |
| (4-MeS-Ph)OCH₂CH₂ | 5-Cl-2-thienyl |
| CH₂=CH | 4,5-diCl-thienyl |
| CH₃(CH₂)₆CH=CHCH₂ | 5-Me-2-thienyl |
| CH₂=CHCH₂ | |

**TABLE 9**

| Compounds of Formula I wherein R¹=Me, R²=Ph, R³=Ph, R⁴=H, A=N-C(=O)SR¹⁸, B=MeS, and: | |
|---|---|
| R¹⁸ | R¹⁸ |
| Me | 2-naphthalenylmethyl |
| Et | PhOCH₂CH₂ |
| Bu | 4-Cl-Bzl |
| ClCH₂CH₂ | 4-CF₃-Bzl |
| Cl₃CCH₂ | 1-Cl-2-naphthalenylmethyl |
| CF₃CH₂ | (4-Cl-Ph)OCH₂CH₂ |
| MeOCH₂CH₂ | (2,4-diCl-Ph)OCH₂CH₂ |
| CH₂(CH₂)₁₁OCH₂CH₂ | (4-F-Ph)OCH₂CH₂ |
| EtOCH₂CH₂ | (4-CF₃-Ph)OCH₂CH₂ |
| Bzyl | CH₂=CHCH₂ |
| 1-naphthalenylmethyl | ClCH₂CH=CHCH₂ |
| Cl₃CCH=CHCH₂ | 2,4-diCl-Ph |
| HC≡CCH₂ | 4-F-Ph |
| cyclopropyl | 4-MeS-Ph |
| cyclohexyl | 1-F-2-naphthalenyl |
| 1-naphthalenyl | 5-Cl-2-thienyl |
| 2-naphthalenyl | 4,5-diCl-thienyl |
| 3-thienyl | 5-F-2-thienyl |
| 4-Cl-Ph | 5-Me-2-thienyl |

**TABLE 10**

| Compounds of Formula I wherein R¹=Me, R²=Ph, R³=Ph, R⁴=H, A=N-C(=O)NR¹⁹R²⁰, B=MeS, and: | | | |
|---|---|---|---|
| R¹⁹ | R²⁰ | R¹⁹ | R²⁰ |
| H | H | 4-CF₃-Ph | H |
| Me | H | 4-MeS-Ph | H |
| Et | H | Me | Me |
| i-Pr | H | Me | Et |
| decyl | H | | -CH₂CH₂CH₂CH₂- |
| Ph | H | | -CH₂CH₂CH₂CH₂CH₂- |
| 4-Cl-Ph | H | | -CH₂CH₂OCH₂CH₂- |
| 3,5-diCl-Ph | H | | -CH₂CH(Me)OCH(Me)CH₂- |
| 4-Me-Ph | H | | |

**TABLE 11**

| Compounds of Formula I wherein R¹=Me, R²=Ph, R³=Ph, R⁴=H, A=N-OG, B=MeS, and: | |
|---|---|
| G | G |
| H | 4-F-Bzl |
| H (HBr salt) | 4-NO₂-Bzl |
| Me | 4-Me-Bzl |
| Me (HBr salt) | MeC(=O) |
| hexyl | MeCH₂C(=O) |
| Bzl | MeC(CH₂)₂C(=O) |
| 4-Cl-Bzl | CH₃(CH₂)₃C(=O) |
| 2,4-diCl-Bzl | MeOC(=O) |
| EtOC(=O) | (4-Cl-Ph)NH(C=O) |
| MeNHC(=O) | (4-NO₂-Ph)NHC(=O) |
| BuNHC(=O) | (4-CF₃-Ph)NHC(=O) |
| CH₃(CH₂)₅NHC(=O) | (4-MeS-Ph)NHC(=O) |
| Ph-NH(C=O) | |

**TABLE 12**

| Compounds of Formula I wherein R¹=Me, R²=Ph, R³=Ph, R⁴=H, A=N-J, B=MeS, and: | |
|---|---|
| J | J |
| H | EtC(=O) |
| H (HBr salt) | BuC(=O) |
| Me | MeSC(=O) |
| Bzl | EtSC(=O) |
| HC(=O) | H₂NC(=O) |
| MeC(=O) | MeNHC(=O) |
| CH₂=CH-C(=O) | PhNHC(=O) |
| PhC(=O) | (4-Cl-Ph)NHC(=O) |
| 4-Cl-PhC(=O) | (4-Me-Ph)NHC(=O) |
| 2-naphthoyl | HO |
| 2-furoyl | MeO |
| 2-thienyl | CH₃C(=O)O |
| PhC(=O)C(=O) | MeOC(=O)O |
| 3-pyridinyl-C(=O) | MeNHC(=O)O |
| MeOC(=O)C(=O) | PhNHC(=O)O |
| EtOC(=O)C(=O) | Me₂P(=O) |
| MeOC(=O) | |

**TABLE 13**

| Compounds of Formula I wherein R²=Ph, R³=Ph, R⁴=H, A=N-H (HBr salt), B=MeS, and: | |
|---|---|
| R¹ | R¹ |
| Me | allyl |
| Et | cyclopropyl |
| CF₃ | vinyl |
| CH₃CH=CHCH₂ | 4-Me-Bzl |
| MeO(C=O) | 4-CF₃-Bzl |
| Bzl | 4-MeO-Bzl |
| 4-Cl-Bzl | |

**TABLE 14**

| Compounds of Formula I wherein R¹=Me, R²=Ph, R⁴=H, A=N-H, B=Mes, and: | |
|---|---|
| R³ | R³ |
| 2-pyridyl | 5-CN-2-pyridyl |
| 3-pyridyl | 6-Me-2-pyridyl |
| 2-pyrimidinyl | 5-CF₃-2-pyridyl |
| 4-pyrmidinyl | 5-MeS-2-pyridyl |
| 5-pyrimidinyl | 5-CF₃O-2-pyridyl |
| 4-Cl-Ph | 3-Cl-2-pyrimidinyl |
| 4-Br-Ph | 3,5-diCl-2-pyrimidinyl |
| 4-F-Ph | 3-Br-2-pyrimidinyl |
| 4-NO₂-Ph | 3-F-2-pyrimidinyl |
| 4-Me-Ph | 3-NO₂-2-pyrimidinyl |
| 4-CF₃-Ph | 3-NC-2-pyrimidinyl |
| 4-MeO-Ph | 3-Me-2-pyrimidinyl |
| 4-MeS-Ph | 3-CF₃-2-pyrimidinyl |
| 4-CF₃O-Ph | 3-MeO-2-pyrimidinyl |
| 5-Cl-2-pyridyl | 3-CF₃O-2-pyrimidinyl |
| 5-F-2-pyridyl | Bzl |
| 5-NO₂-2-pyridyl | |

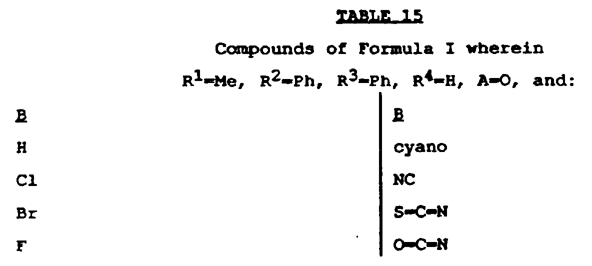

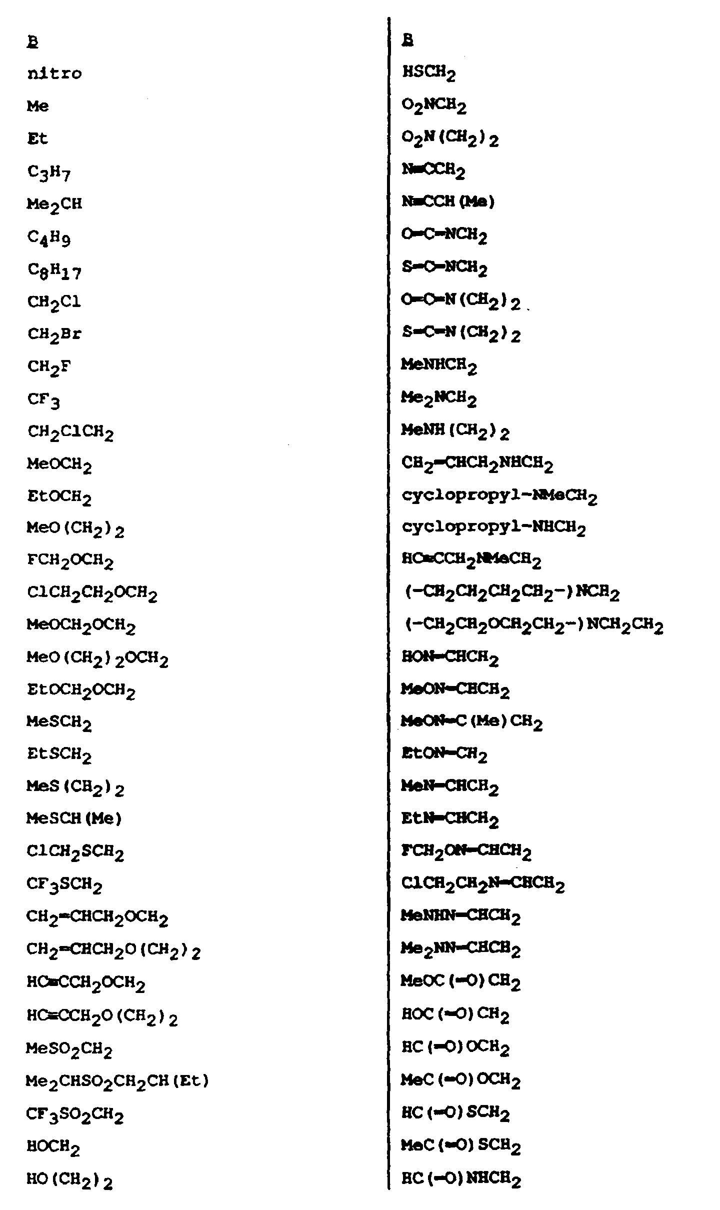

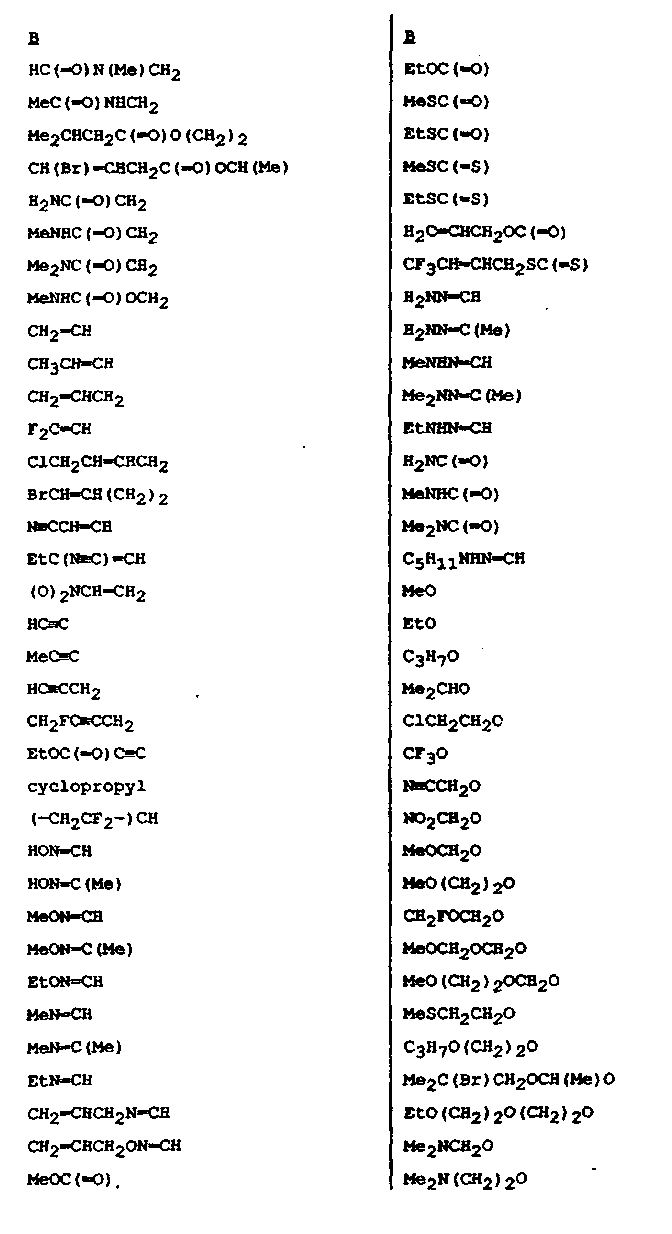

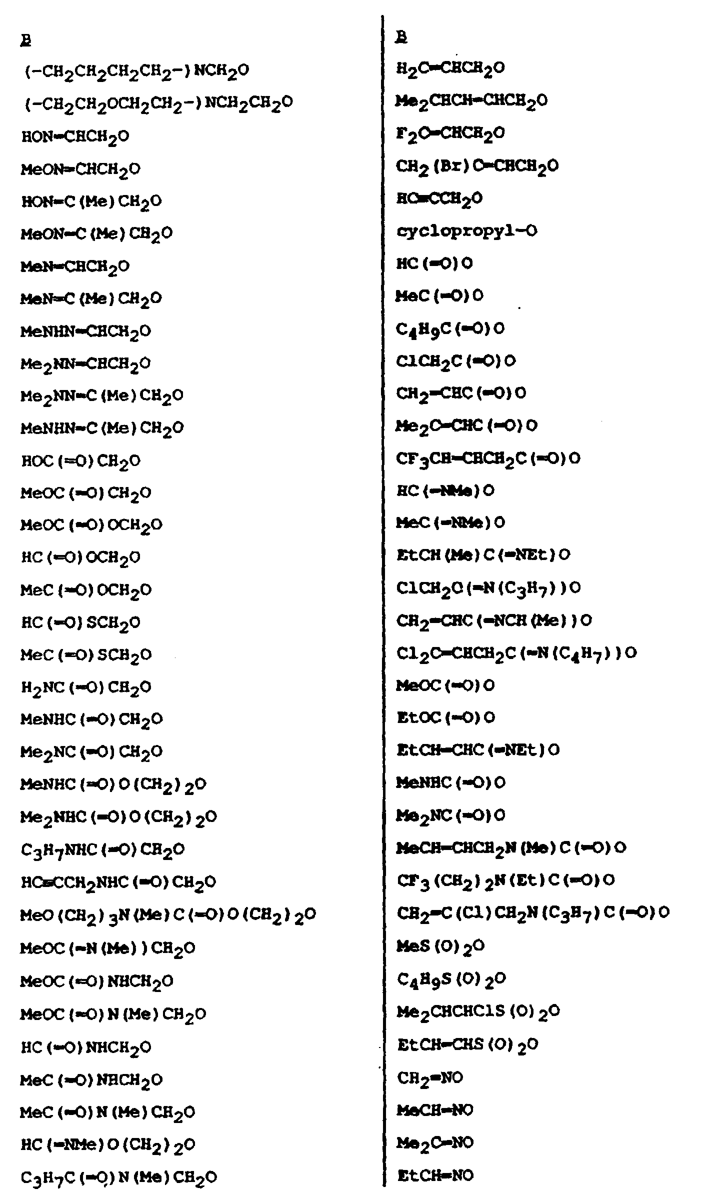

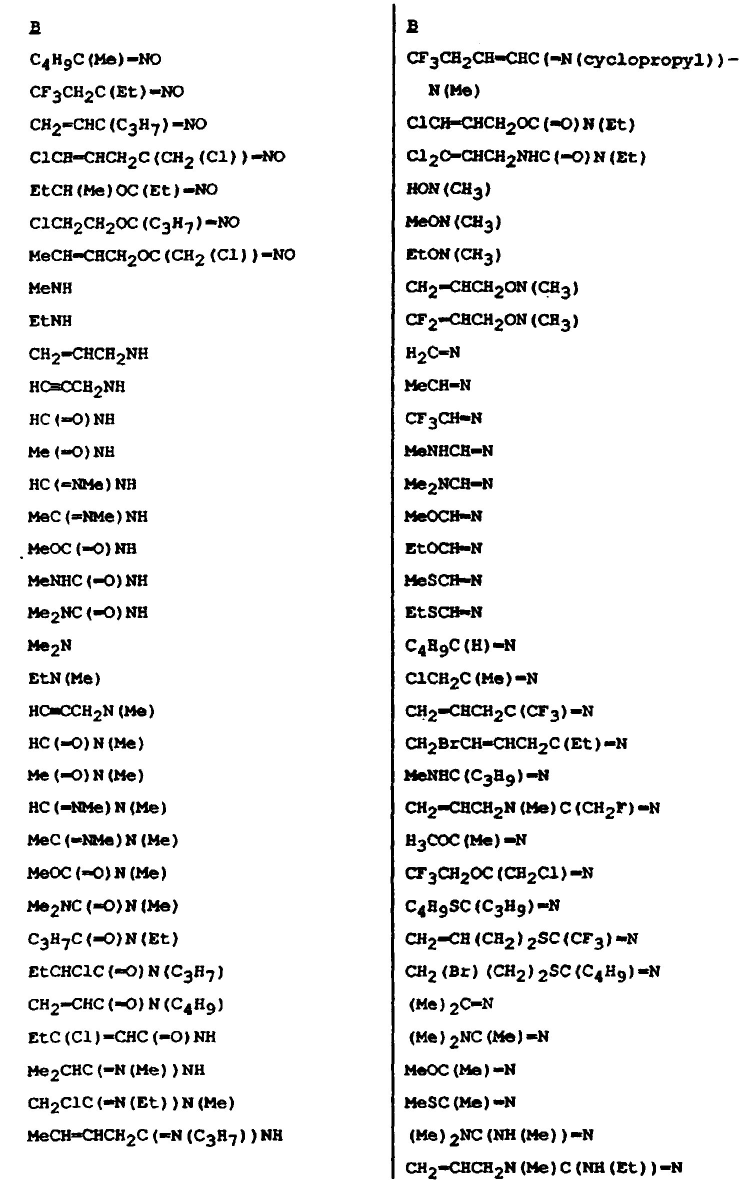

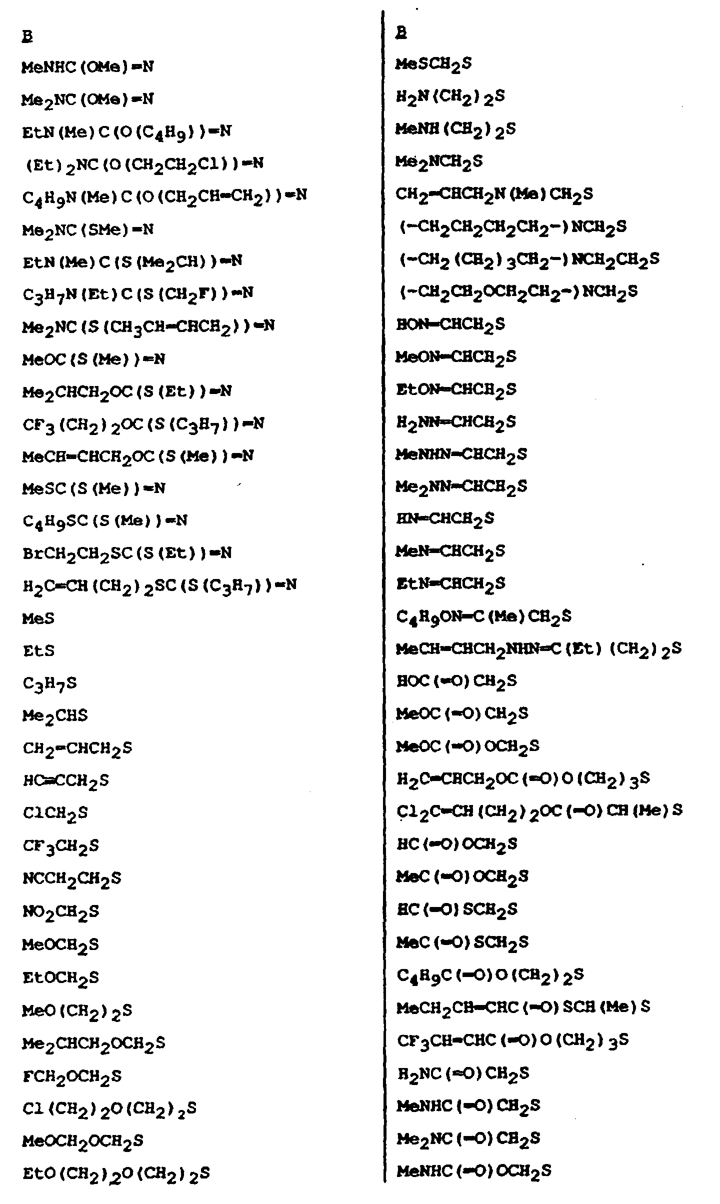

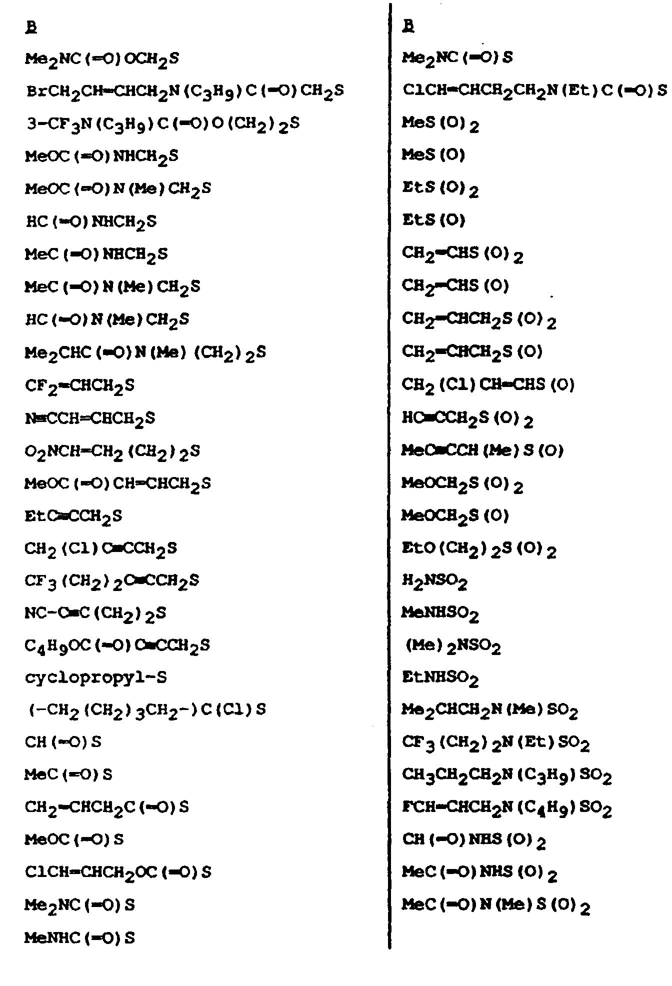

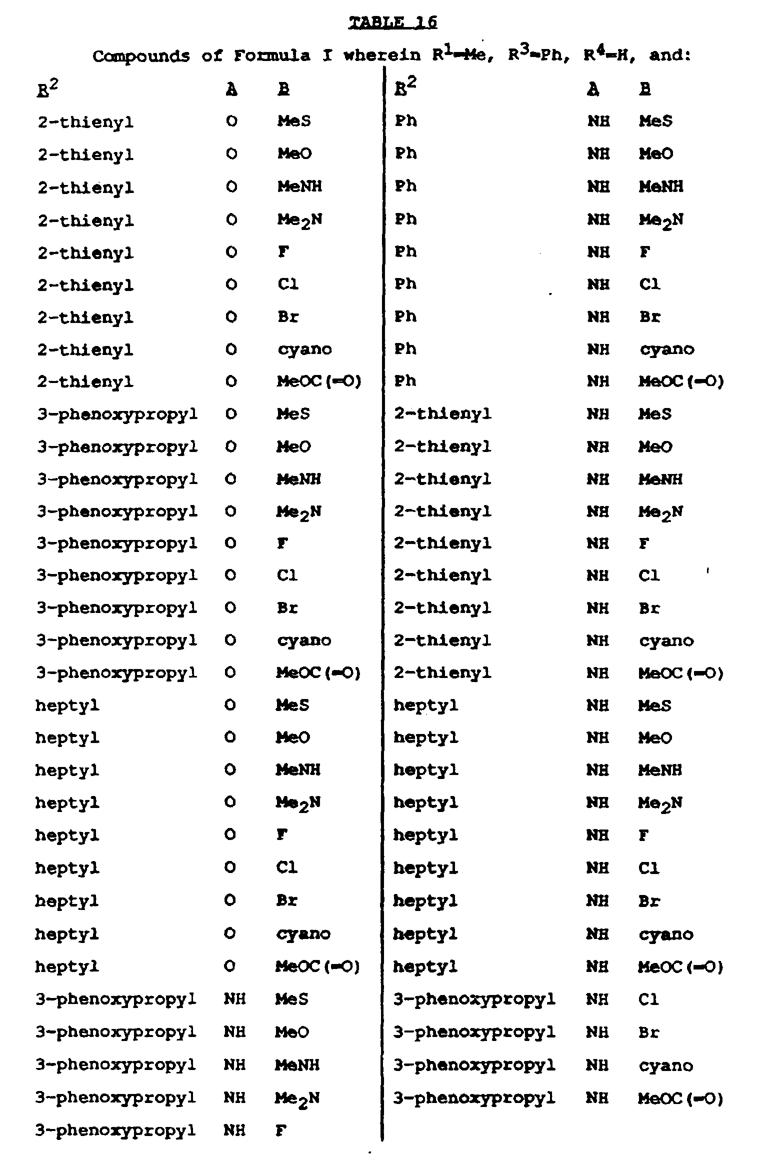

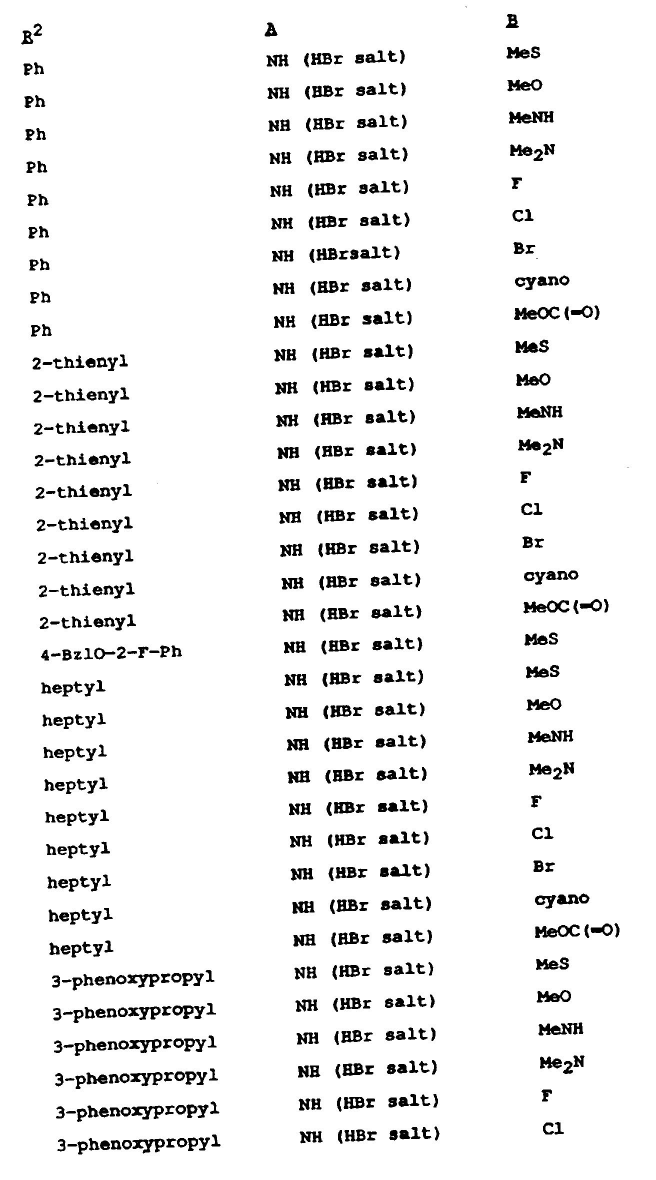

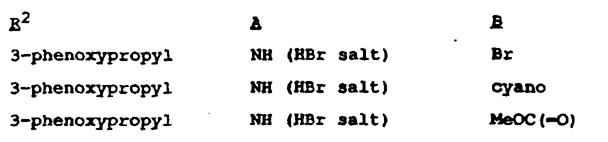

### Formulation/Utility

Compounds of this invention will generally be used in formulation with an agriculturally suitable composition. The fungicidal compositions of the present invention comprise an effective amount of at least one compound of Formula I as defined above and at least one of (a) a surfactant, (b) an organic solvent, and (c) at least one solid or liquid diluent. Useful formulations can be prepared in conventional ways. They include dusts, granules, pellets, solutions, suspensions, emulsions, wettable powders, emulsifiable concentrates, dry flowables and the like. Sprayable formulations can be extended in suitable media and used at spray volumes from about one to several hundred liters per hectare. High strength compositions are primarily used as intermediates for further formulation. The formulations will typically contain effective amounts of active ingredient, diluent and surfactant within the following approximate ranges which add up 100 weight percent.

| | Weight Percent | | |
|---|---|---|---|
| | Active Ingredient | Diluent | Surfactant |
| Wettable Powders | 25-90 | 0-74 | 1-10 |
| Oil Suspensions, Emulsions, Solutions, (including Emulsifiable Concentrates) | 5-50 | 40-95 | 0-15 |
| Dusts | 1-25 | 70-99 | 0-5 |
| Granules, Baits and Pellets | 0.01-99 | 5-99.99 | 0-15 |
| High Strength Compositions | 90-99 | 0-10 | 0-2 |

Typical solid diluents are described in Watkins, et al., *Handbook of Insecticide Dust Diluents and Carriers*, 2nd Ed., Dorland Books, Caldwell, New Jersey. Typical liquid diluents and solvents are described in Marsden, *Solvents Guide*, 2nd Ed., Interscience, New York, 1950. *McCutcheon's Detergents and Emulsifiers Annual*, Allured Publ. Corp., Ridgewood, New Jersey, as well as Sisely and Wood, *Encyclopedia of Surface Active Agents*, Chemical Publ. Co., Inc., New York, 1964, list surfactants and recommended uses. All formulations can contain minor amounts of additives to reduce foam, caking, corrosion, microbiological growth, etc.

Methods for formulating such compositions are well known. Solutions are prepared by simply mixing the ingredients. Fine solid compositions are made by blending and, usually, grinding as in a hammer mill or fluid energy mill, Water-dispersible granules can be produced be agglomerating a fine powder composition; see for example, Cross et al., *Pesticide Formulations*, Washington, D.C., 1988, pp 251-259. Suspensions are prepared by wet-milling; see, for example, U.S. 3,060,084. Granules and pellets can be made by spraying the active material upon preformed granular carriers or by agglomeration techniques. See Browning, "Agglomeration", *Chemical Engineering*, December 4, 1967, pp 147-148, *Perry's Chemical Engineer's Handbook*, 4th Ed., McGraw-Hill, New York, 1963, pp 8-57 and following, and WO 91/13546. Pellets can be prepared as described in U.S. 4,172,714. Water-dispersible and water-soluble granules can be prepared as taught in DE 3,246,493.

For further information regarding the art of formulation, see U.S. 3,235,361, Col. 6, line 16 through Col. 7, line 19 and Examples 10 through 41; U.S. 3,309,192, Col. 5, line 43 through Col. 7, line 62 and Examples 8, 12, 15, 39, 41, 52, 53, 58, 132, 138-140, 162-164, 166, 167 and 169-182; U.S. 2,891,855, Col. 3, line 66 through Col. 5, line 17 and Examples 1-4; Klingman, *Weed Control as a Science*, John Wiley and Sons, Inc., New York, 1961, pp 81-96; and Hance et al., *Weed Control Handbook*, 8th Ed., Blackwell Scientific Publications, Oxford, 1989.

In the following Examples, all percentages are by weight and all formulations are worked up in conventional ways. Compound numbers refer to Index Table A hereinafter.

### Example A

| Wettable Powder | |
|---|---|
| Compound 1 | 65.0% |
| dodecylphenol polyethylene glycol ether | 2.0% |
| sodium ligninsulfonate | 4.0% |
| sodium silicoaluminate | 6.0% |
| montmorillonite (calcined) | 23.0%. |

### Example B

| Granule | |
|---|---|
| Compound 1 | 10.0% |
| attapulgite granules (low volative matter, 0.71/0.30 mm; U.S.S. No. 25-50 sieves) | 90.0%. |

### Example C

| Extruded Pellet | |
|---|---|
| Compound 1 | 25.0% |
| anhydrous sodium sulfate | 10.0% |
| crude calcium ligninsulfonate | 5.0% |
| sodium alkylnaphthalenesulfonate | 1.0% |
| calcium/magnesium bentonite | 59.0%. |

### Example D

| Emulsifiable Concentrate | |
|---|---|
| Compound 1 | 20.0% |
| blend of oil soluble sulfonates and polyoxyethylene ethers | 10.0% |
| isophorone | 70.0%. |

The compounds of this invention are useful as plant disease control agents. The present invention therefore further comprises a method for controlling plant diseases caused by fungal plant pathogens comprising applying to the plant or portion thereof to be protected, or to the plant seed or seedling to be protected, an effective amount of a compound of Formula I or a fungicidal composition containing said compound. The compounds and compositions of this invention provide control of diseases caused by a broad spectrum of fungal plant pathogens in the *Basidiomycete, Ascomycete, Oomycete* and *Deuteromycete* classes. They are effective in controlling a broad spectrum of plant diseases, particularly foliar pathogens of ornamental, vegetable, field, cereal, and fruit crops. These pathogens include *Plasmopara viticola, Phytophthora infestans, Peronospora tabacina,* Ps*eudoperonospora cubensis, Pythium aphanidermatum, Alternaria brassicae, Septeria nodorum, Cercosporidium personatum, Cercospora arachidicola, Pseudocercosporella herpotrichoides, Cercospora beticola, Botrytis cinerea, Monilinia fructicola, Pyricularia oryzae, Podosphaera leucotricha, Venturia inaequalis, Erysiphe graminis, Uncinula necatur, Puccinia recondita, Puccinia graminis, Hemileia vastatrix, Puccinia striiformis, Puccinia arachidis, Rhizoctonia solani, Sphaerotheca fuligines, Fusarium oxysporum, Verticillium dahliae, Pythium aphanidermatum, Phytophthora megasperma* and other generea and species closely related to these pathogens.

Compounds of this invention can also be mixed with one or more other insecticides, fungicides, nematocides, bactericides, acaricides, semiochemicals, repellants, attractants, pheromones, feeding stimulants or other biologically active compounds to form a multi-component pesticide giving an even broader spectrum of agricultural protection. Examples of other agricultural protectants with which compounds of this invention can be formulated are: insecticides such as monocrotophos, carbofuran, tetrachlorvinphos, malathion, parathion-methyl, methomyl, chlordimeform, diazinon, deltamethrin, oxamyl, fenvalerate, esfenvalerate, permethrin, profenofos, sulprofos, triflumuron, diflubenzuron, methoprene, buprofezin, thiodicarb, acephate, azinphosmethyl, chlorpyrifos, dimethoate, fipronil, flufenprox, fonophos, isofenphos, methidathion, methamidophos, phosmet, phosphamidon, phosalone, pirimicarb, phorate, terbufos, trichlorfon, methoxychlor, bifenthrin, biphenate, cyfluthrin, fenpropathrin, fluvalinate, flucythrinate, tralomethrin, metaldehyde and rotenone; fungicides such as carbendazim, thiuram, dodine, maneb, chloroneb, benomyl, cymoxanil, fenpropidine, fenpropimorph, triadimefon, captan, thiophanate-methyl, thiabendazole, phosethyl-Al, chlorothalonil, dichloran, metalaxyl, captafol, iprodione, oxadixyl, vinclozolin, kasugamycin, myclobutanil, tebuconazole, difenoconazole, diniconazole, fluquinconazole, ipconazole, metoonazole, penconazole, propiconazole, uniconzole, flutriafol, procliloraz, pyrifenox, fenarimol, triadimenol, diclobutrazol, copper oxychloride, furalaxyl, folpet, flusilazol, blasticidin S, diclomezine, edifenphos, isoprothiolane, iprobenfos, mepronil, neo-asozin, pencycuron, probenazole, pyroguilon, tricyclazole, validanycin, and flutolanil; nematocides such as aldoxycarb, fenamiphos and fosthietan; bactericides such as oxytetracyline, streptomycin and tribasic copper sulfate; acaricides such as binapacryl, oxythioquinox, chlorobenzilate, dicofol, dienochlor, cyhexatin, hexythiazox, amitraz, propargite, tebufenpyrad and fenbutatin oxide; and biological agents such as Bacillus thuringiensis, baculovirus and avermectin B.

In certain instances, combinations with other fungicides having a similiar spectrum of control but a different mode of action will be particularly advantageous for resistance management.

Plant disease control is ordinarily accomplished by applying an effective amount of a compound of this invention either pre- or post-infection, to the portion of the plant to be protected such as the roots, stems, foliage, fruit, seeds, tubers or bulbs, or to the media (soil or sand) in which the plants to be protected are growing. The compounds can also be applied to the seed to protect the seed and seedling.

Rates of application for these compounds can be influenced by many factors of the environment and should be determined under actual use conditions. Foliage can normally be protected when treated at a rate of from less than 1 g/ha to 5,000 g/ha of active ingredient. Seed and seedlings can normally be protected when seed is treated at a rate of from 0.1 to 10 g per kilogram of seed.

The following Tests demonstrate the control efficacy of compounds of this invention on specific pathogens. The pathogen control protection afforded by the compounds is not limited, however, to these species. See Index Table A for compound descriptions.

Test compounds were first dissolved in acetone in an amount equal to 3% of the final volume and then suspended at a concentration of 200 ppm in purified water containing 250 ppm of the surfactant Trem® 014 (polyhydric alcohol esters). The resulting test suspensions were then used in the following tests.

### TEST A

The test suspension was sprayed to the point of run-off on wheat seedlings. The following day the seedlings were inoculated with a spore dust of *Erysiphe graminis f. sp. tritici*, (the causal agent of wheat powdery mildew) and incubated in a growth chamber at 20°C for 7 days, after which disease ratings were made.

### TEST B

The test suspension was sprayed to the point of run-off on wheat seedlings. The following day the seedlings were inoculated with a spore suspension of *Puccinia recondita* (the causal agent of wheat leaf rust) and incubated in a saturated atmosphere at 20°C for 24 h, and then moved to a growth chamber at 20°C for 6 days, after which disease ratings were made.

### TEST-C

The test suspension was sprayed to the point of run-off on rice seedlings. The following day the seedlings were inoculated with a spore suspension of *Pyricularia oryzae* (the causal agent of rice blast) and incubated in a saturated atmosphere at 27°C for 24 h, and then moved to a growth chamber at 30°C for 5 days, after which disease ratings were made.

### TEST D

The test suspension was sprayed to the point of run-off on tomato seedlings. The following day the seedlings were inoculated with a spore suspension of *Phytophthora infestans* (the causal agent of potato and tomato late blight) and incubated in a saturated atmosphere at 20°C for 24 h, and then moved to a growth chamber at 20°C for 5 days, after which disease ratings were made.

### TEST E

The test suspension was sprayed to the point of run-off on potato seedlings. The following day the seedlings were inoculated with a spore suspension of *Phytophthora infestans* (the causal agent of potato and tomato late blight) and incubated in a saturated atmosphere at 20°C for 24 h, and then moved to a growth chamber at 20°C for 5 days, after which disease ratings were made.

### TEST F

The test suspension was sprayed to the point of run-off on grape seedlings. The following day the seedlings were inoculated with a spore suspension of *Plasmopara viticola* (the causal agent of grape downy mildew) and incubated in a saturated atmosphere at 20°C for 24 h, moved to a growth chamber at 20°C for 6 days,and then incubated in a saturated atmosphere at 20°C for 24 h, after which disease ratings were made.

### TEST G

The test suspension was sprayed to the point of run-off on cucumber seedlings. The following day the seedlings were inoculated with a spore suspension of *Botrytis cinerea* (the causal agent of gray mold on many crops) and incubated in a saturated atmosphere at 20°C for 48 h, and moved to a growth chamber at 20°C for 5 days, after which disease ratings were made.

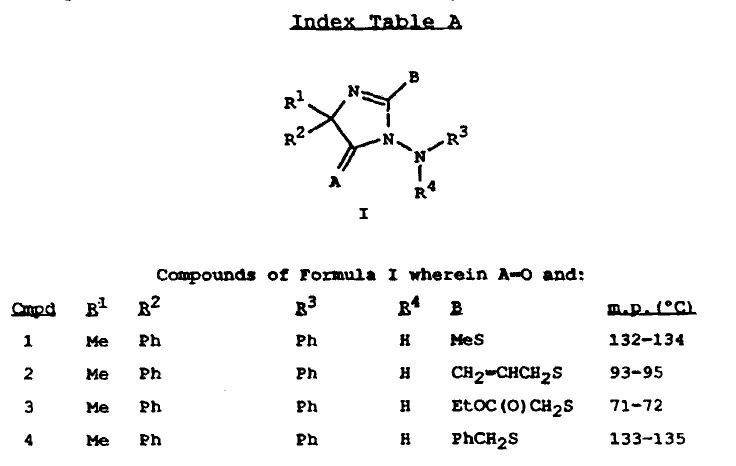

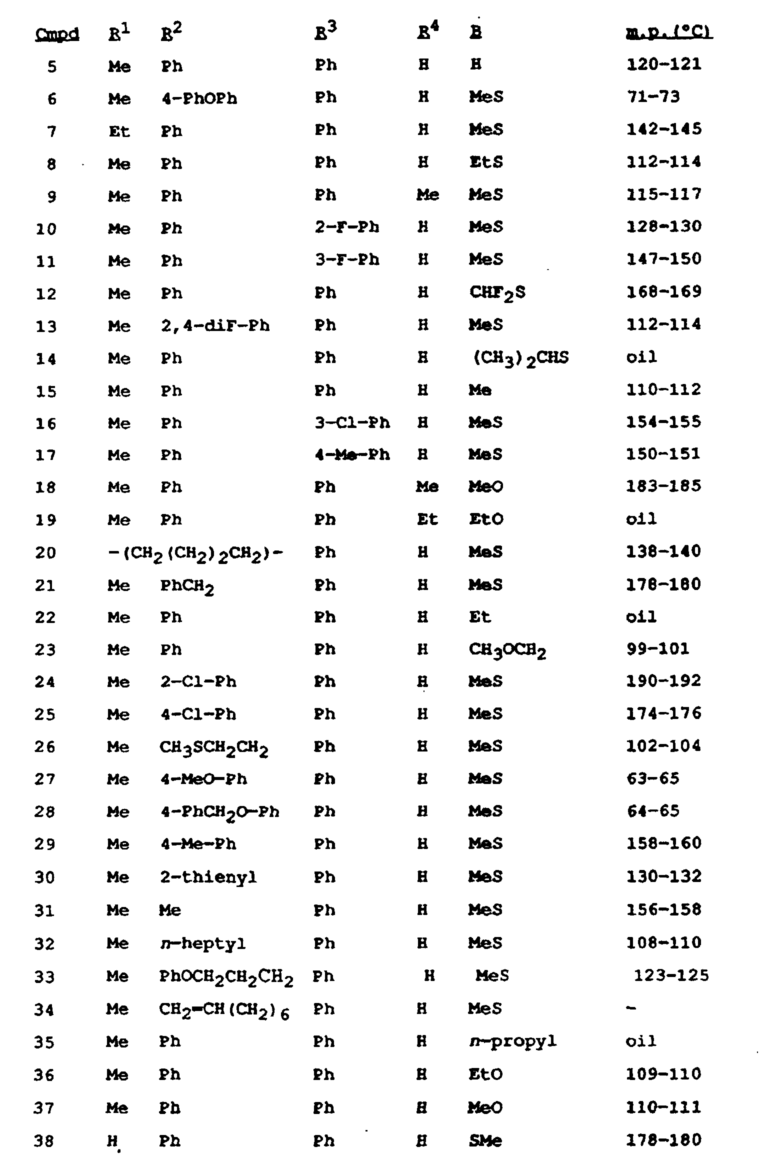

| Index Table B | |
|---|---|
| Cmpd No. | ¹H NMR Data (CDCl₃, δ) |
| 14 | 1.48 (d, J=6.8 Hz, 3H), 1.52 (d, J=6.8 Hz, 3H), 1.76 (s, 3H), 4.03 (m, J=6.8 Hz, 1H), 6.10 (broad s, 1H), 6.93-7.62 (m, 10H) |
| 19 | major isomer: δ 1.27 (t, J=6.5 Hz, 3H), 1.79 (s, 3H), 3.60 (m, 2H), 4.52 (m, 2H), 6.75-7.65 (m, 10H) minor isomer: δ 1.06 (t, J=6.5 Hz, 3H), 1.77 (s, 3H), 3.60 (m, 2H), 4.52 (m, 2H), 6.60-7.65 (m, 10H) |
| 22 | 1.30 (t, J=7.4 Hz, 3H), 1.68 (s, 3H), 2.55 (q, J=7.4 Hz, 2H), 6.70 (s, 1H), 6.52-7.60 (m, 10H) |
| 34 | 0.95-1.30 (m, 8H), 1.23 (s, 3H), 1.50-2.00 (m, 4H), 2.45 (s, 3H), 4.78-5.12 (m, 2H), 5.50-5.92 (m, 1H), 6.40 (s, 1H), 6.61-7.30 (m, 5H) |
| 35 | 1.04 (t, J=7.6 Hz, 3H), 1.73 (s, 3H), 1.84 (m, 2H), 2.77 (q, J=7.6 Hz, 2H), 6.25 (s, 1H), 6.58-7.63 (m, 10H) |

Results for Tests A-G are given in Table C. In the table, a rating of 100 indicates 100% disease control and a rating of 0 indicates no disease control (relative to the controls). NT = not tested.

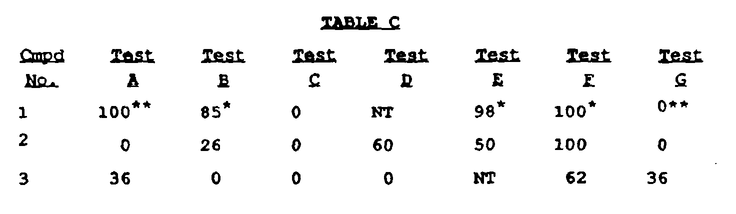

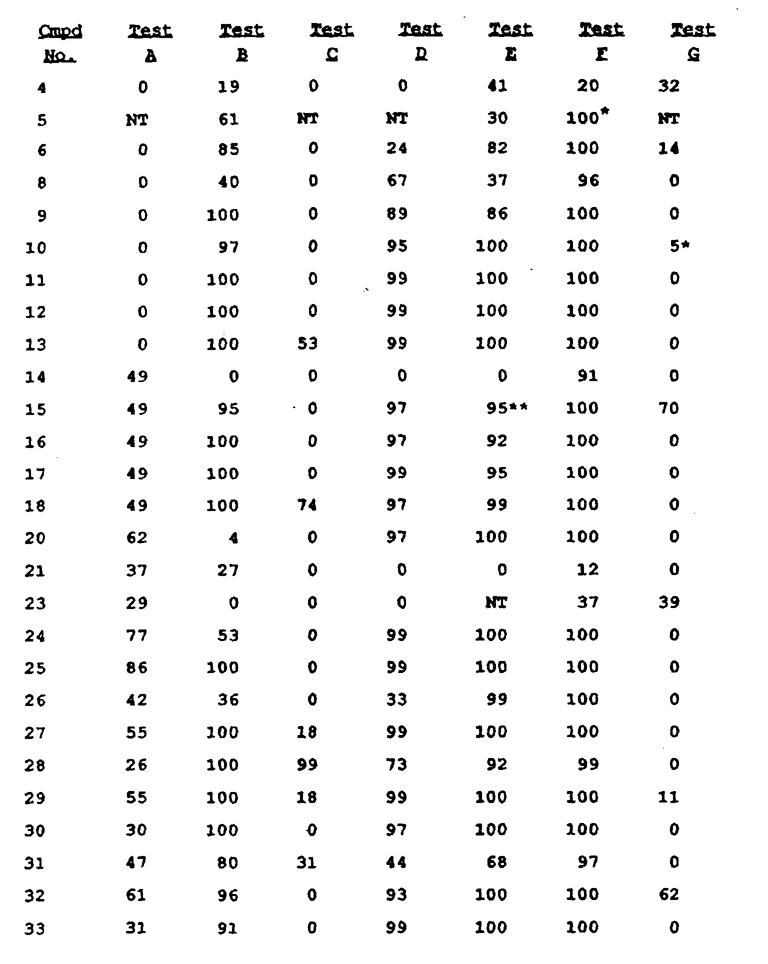

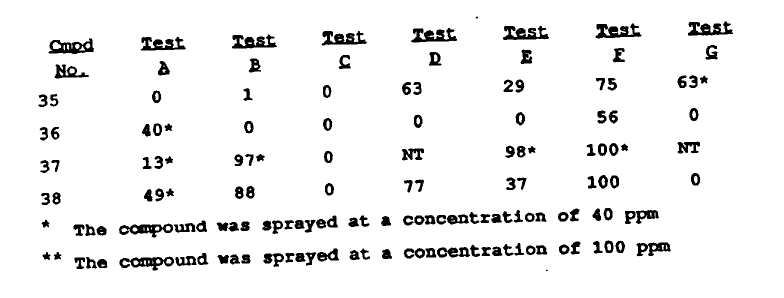

## Claims

1. A compound of Formula I 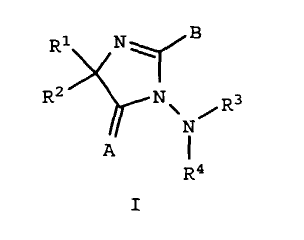 wherein:
A is O; S or N-J;
J is R¹⁵; C(=O)R¹⁶; C(=O)OR¹⁷; C(=O)SR¹⁸; C(=O)NR¹⁹R²⁰; P(=O)(C₁-C₄ alkyl)₂; or OG;
G is H; C₁-C₆ alkyl; benzyl optionally substituted with R³⁴ on the phenyl ring; C(=O)(C₁-C₄ alkyl); C(=O)(C₁-C₄ alkoxy); or C(=O)NHR³⁶;
B is H; halogen; cyano; NC; S=C=N; O=C=N; nitro; R²¹; OR²⁹; NR⁴⁹R⁶³; N=CR⁴⁵R⁴⁶; SR⁴⁷; S(O)ₙR⁴⁸; or SO₂NR⁴⁹R⁶⁰;
n is 1 or 2;
R¹ is C₁-C₄ alkyl; C₁-C₄ haloalkyl; C₃-C₆ cycloalkyl; C₂-C₄ alkenyl; C₂-C₄ alkoxycarbonyl; or phenylmethyl optionally substituted with R⁶ on the phenyl ring and with R⁸ on the benzylic carbon;
R² is C₁-C₂₀ alkyl optionally substituted with R²²; C₂-C₂₀ alkoxyalkyl optionally substituted with R³⁵; C₂-C₂₀ alkenyl optionally substituted with R⁴²; C₂-C₂₀ alkynyl optionally substituted with R⁴¹; (CH₂CH₂OCH₂CH₂)CH-; (CH₂CH₂SCH₂CH₂)CH-; (CH₂CH₂SO₂CH₂CH₂)CH-; C₅-C₇ cycloalkyl; C₅-C₇ cycloalkenyl; phenyl optionally substituted with R⁵ and R⁷; 2-naphthalenyl; thienyl optionally substituted with R⁵ and R⁷; furyl optionally substituted with R⁷; or pyridyl optionally substituted with R⁵ and R⁷; or
R¹ and R² can be taken together to form a structure selected from the group consisting of -CH₂(CH₂)₂CH₂-, -CH₂(CH₂)₃CH₂-, -CH₂(CH₂)₄CH₂-, -CH₂CH₂OCH₂CH₂-, -CH₂CH₂SCH₂CH₂-, 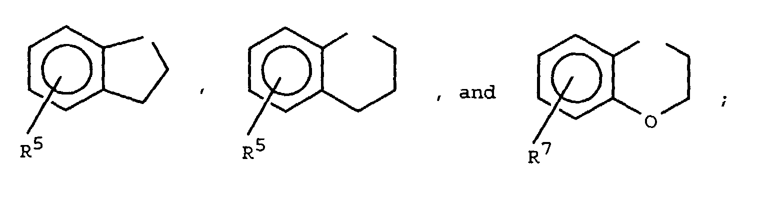
R³ is phenyl, pyridyl, or pyrimidinyl each optionally substituted with R¹⁰; or phenylmethyl;
R⁴ is H or methyl;
R⁵ is halogen; nitro; cyano; C₁-C₆ alkyl; C₅-C₆ cycloalkyl; C₁-C₆ haloalkyl; C₁-C₆ alkylthio; C₁-C₆ haloalkylthio; C₁-C₆ alkoxy; C₁-C₆ haloalkoxy; C₅-C₆ cycloalkyloxy; C₂-C₆ alkoxyalkyl; C₂-C₆ alkoxyalkoxy; C₃-C₆ alkenyl; C₃-C₆ haloalkenyl; C₃-C₆ alkenyloxy; C₃-C₆ alkynyl; C₃-C₆ haloalkynyl; C₃-C₆ alkynyloxy; C₁-C₆ alkylsulfonyl; C₁-C₆ haloalkylsulfonyl; phenyl or phenylthio each optionally substituted with R²⁴; phenylmethyl, phenoxymethyl, phenethyl, or styryl each optionally substituted with R²⁴ on the phenyl ring; phenoxy optionally substituted with R²⁷; benzyloxy optionally substituted with R³⁰ on the phenyl ring; -OC(=O)NHR²⁸; -C(=O)OR²⁸; or -OC(=O)R²⁸;
R⁶, R⁷, R¹², R¹³, R²⁴, R²⁶ and R³⁴ are independently 1-2 halogen; nitro; C₁-C₄ alkyl; trifluoromethyl; methylthio; or C₁-C₄ alkoxy;
R⁸, R¹⁴, R²⁰, R³⁸ and R⁴⁰ are independently H or C₁-C₄ alkyl;
R⁹ is C₁-C₁₈ alkyl; or phenyl optionally substituted with R⁷;
R¹⁰, R²⁵ and R³³ are each independently 1-2 substituents selected from the group consisting of halogen, nitro, cyano, C₁-C₄ alkyl, trifluoromethyl, C₁-C₄ alkylthio, C₁-C₄ alkoxy and trifluoromethoxy;
R¹¹ and R³⁶ are independently C₁-C₆ alkyl; or phenyl optionally substituted with R¹²;
R¹⁵ is H; C₁-C₈ alkyl optionally substituted with C₁-C₂ alkoxy; C₃-C₆ cycloalkyl; C₃-C₈ alkenyl; C₃-C₈ alkynyl; phenyl optionally substituted with R¹³; benzyl optionally substituted with R¹³ on the phenyl ring and with R²⁰ on the benzylic carbon; or pyridyl optionally substituted with R¹³;
R¹⁶ is H; C₁-C₁₇ alkyl optionally substituted with R³¹; C₂-C₁₇ alkenyl optionally substituted with R³²; C₂-C₇ alkynyl; C₃-C₈ cycloalkyl; C₅-C₆ cycloalkenyl; C₆-C₇ alkylcycloalkyl; C₄-C₈ cycloalkylalkyl; phenyl optionally substituted with R³³; naphthalenyl, furanyl, thienyl, benzoyl, or pyridyl each optionally substituted with R³⁴; or C₂-C₅ alkoxycarbonyl;
R¹⁷ and R¹⁸ are independently C₁-C₁₈ alkyl optionally substituted with R²³; C₂-C₁₀ alkenyl optionally substituted with R³²; C₃-C₈ alkynyl; C₃-C₁₂ cycloalkyl; C₅-C₆ cycloalkenyl; C₆-C₇ alkylcycloalkyl; C₆-C₇ cycloalkylalkyl; or phenyl, naphthalenyl, or thienyl each optionally substituted with R³⁴;
R¹⁹ is H; C₁-C₁₀ alkyl; C₅-C₆ cycloalkyl; or phenyl optionally substituted with R³⁴; or
R¹⁹ and R²⁰ can be taken together to form a structure selected from the group consisting of -CH₂(CH₂)₂CH₂-, -CH₂(CH₂)₃CH₂-, -CH₂(CH₂)₄CH₂-, -CH₂CH₂OCH₂CH₂-, -CH₂CH(Me)CH₂CH(Me)CH₂-, and -CH₂CH(Me)OCH(Me)CH₂-;
R²¹ is C₁-C₈ alkyl optionally substituted with R⁵¹; C₂-C₈ alkenyl or C₂-C₈ alkynyl each optionally substituted with R⁶⁹; C₃-C₆ cycloalkyl optionally substituted with 1-3 halogen; C(=N-V-R⁵³)R⁵²; C(=O)OR⁵³; C(=O)SR⁵³; C(=NR⁵⁵)OR⁵³; C(=S)SR⁵³; C(=O)NR⁵³R⁵⁶; or C(=NR⁵⁵)NR⁵³R⁵⁶;
V is O; NR⁵⁵; or a direct bond;
R²² is cyano; nitro; C₁-C₁₉ alkylthio; C₁-C₁₉ alkylsulfinyl; C₁-C₁₉ haloalkoxy; C₅-C₆ cycloalkyloxy; C₃-C₁₉ alkenyloxy; C₃-C₁₉ alkynyloxy; C₁-C₁₉ alkylsulfonyl; C₂-C₁₉ alkoxycarbonyl; hydroxyl; hydroxycarbonyl; R²⁸C(=O)O; R²⁸OC(=O)O; R²⁸R⁴⁰NC(=O)O; R³⁹R⁴⁰N; (C₁-C₄ alkoxy)₂P(=E)O; R¹¹SO₃; R⁴⁰R¹⁴R³⁸N⁺; phenyl, phenylthio, phenoxy, phenylsulfonyl, phenylsulfinyl, pyridyl or pyridyloxy each optionally substituted with R³⁰; thienyl, pyrimidinyl, furanyl, naphthalenyl, pyrimidinyloxy, naphthalenyloxy each optionally substituted with R⁷; tetrahydropyranyl; C₃-C₆ cycloalkyl; 2-tetrahydropyranyloxy; or C(=Q)R⁴⁰;
E is O or S;
Q is O or N-T-W;
T is O; NR³⁷; or a direct bond;
W is H; C₁-C₈ alkyl, C₃-C₈ alkenyl; phenylmethyl optionally substituted with R⁷ on the phenyl ring and R¹⁴ on the benzylic carbon; phenyl or pyridyl each optionally substituted with R⁷; C(=O)R²⁸; C(=O)OR²⁸; or C(=O)NR²⁸R¹⁴;
R²³ is 1-3 halogen; C₁-C₁₂ alkoxy; C₁-C₁₂ alkylthio; phenyl or naphthalenyl each optionally substituted with R³⁴; or phenoxymethyl optionally substituted with R³⁴ on the phenyl ring;
R²⁷ is 1-2 halogen; nitro; cyano; C₁-C₆ alkyl; C₁-C₆ haloalkyl; C₁-C₆ alkoxy; C₁-C₆ haloalkoxy; C₁-C₄ alkylsulfonyl; C₂-C₆ alkoxyalkyl; C₁-C₄ alkylthio; C₅-C₆ cycloalkyl; C₅-C₆ cycloalkyloxy; C₂-C₆ alkenyl; C₂-C₆ haloalkenyl; C₂-C₆ alkynyl; hydroxycarbonyl; C₂-C₄ alkoxycarbonyl; or phenoxy optionally substituted with R²⁴;
R²⁸ is C₁-C₈ alkyl; or phenyl or pyridyl each optionally substituted with R³⁰;
R²⁹ is C₁-C₈ alkyl optionally substituted with R⁴⁴; C₃-C₆ alkenyl or C₃-C₆ alkynyl each optionally substituted with R⁶⁹; C₃-C₆ cycloalkyl optionally substituted with 1-3 halogen; phenyl optionally substituted with R⁵⁷ and R⁵⁹; C(=O)R⁵²; C(=NR⁵⁵)R⁵²; C(=O)OR⁵³; C[=N(C₁-C₄ alkyl)]OR⁵³; C(=O)NR⁵³R⁵⁶; N=CR⁶⁸R⁶⁷; or SO₂R⁵²;
R³⁰ is 1-2 substituents selected from the group consisting of halogen, nitro, cyano, C₁-C₄ alkyl, trifluoromethyl, C₁-C₄ alkoxy and trifluoromethoxy; or phenoxy optionally substituted with R²⁶;
R³¹ is 1-3 halogen; C₁-C₁₈ alkoxy; allyloxy; C₁-C₁₈ alkylthio; phenyl, phenoxy, benzyloxy, or phenylthio each optionally substituted with R³⁴ on the phenyl ring; acetyl; or C₂-C₅ alkoxycarbonyl;
R³² is 1-3 halogen; or C₁-C₄ alkoxy;
R³⁵ is cyano; nitro; C₁-C₁₇ alkylthio; C₁-C₁₇ alkylsulfinyl; C₁-C₁₇ haloalkoxy; C₅-C₆ cycloalkyloxy; C₂-C₁₇ haloalkenyl; C₃-C₁₇ alkenyloxy; C₃-C₁₇ haloalkynyl; C₃-C₁₇ alkynyloxy; C₁-C₁₇ alkylsulfonyl; C₂-C₁₇ alkoxycarbonyl; hydroxyl; hydroxycarbonyl; R²⁸C(=O)O; R²⁸OC(=O)O; R²⁸R⁴⁰NC(=O)O; R⁴⁰R³⁹N; (C₁-C₄ alkoxy)₂P(=E)O; R¹¹SO₃; R⁴⁰R¹⁴R³⁸N⁺; phenyl, phenoxy, phenylthio, phenylsulfonyl, phenylsulfinyl, pyridyl or pyridyloxy each optionally substituted with R³⁰; thienyl, pyrimidinyl, furanyl, naphthalenyl, pyrimidinyloxy, naphthalenyloxy each optionally substituted with R⁷; tetrahydropyranyl; 2-tetrahydropyranyloxy; C₁-C₁₇ alkoxy; C₂-C₁₇ alkoxyalkoxy; C₃-C₁₇ alkynyl; C₃-C₆ cycloalkyl; or C₂-C₁₇ haloalkoxyalkoxy;
R³⁷ is H; C₁-C₆ alkyl; or phenyl optionally substituted with R⁷;
R³⁹ is C₁-C₁₉ alkyl; C₂-C₁₉ alkylcarbonyl; C₂-C₁₉ alkoxycarbonyl; (R⁹R⁴⁰N)C=O; phenyl optionally substituted with R²⁵; or phenoxycarbonyl optionally substituted with R⁷;
R⁴¹ is cyano; nitro; C₁-C₁₇ alkylthio; C₁-C₁₇ alkylsulfinyl; C₁-C₁₇ haloalkoxy; C₅-C₆ cycloalkyloxy; C₃-C₁₇ alkenyloxy; C₃-C₁₇ alkynyloxy; C₁-C₁₇ alkylsulfonyl; C₂-C₁₇ alkoxycarbonyl; hydroxyl; hydroxycarbonyl; R²⁸C(=O)O; R²⁸OC(=O)O; R²⁸R⁴⁰NC(=O)O; R⁴⁰R³⁹N; (C₁-C₄ alkoxy)₂P(=E)O; R¹¹SO₃; R⁴⁰R¹⁴R³⁸N⁺; phenyl, phenoxy, phenylsulfonyl, phenylsulfinyl, pyridyl or pyridyloxy each optionally substituted with R³⁰; thienyl, pyrimidinyl, furanyl, naphthalenyl, pyrimidinyloxy, naphthalenyloxy each optionally substituted with R⁷; tetrahydropyranyl; 2-tetrahydropyranyloxy; C₁-C₁₇ alkoxy; 1-3 halogen; C₂-C₁₇ alkoxyalkoxy; or C₃-C₆ cycloalkyl;
R⁴² is cyano; nitro; C₁-C₁₇ alkylthio; C₁-C₁₇ alkylsulfinyl; C₁-C₁₇ haloalkoxy; C₅-C₆ cycloalkyloxy; C₃-C₁₇ alkenyloxy; C₃-C₁₇ haloalkynyl; C₃-C₁₇ alkynyloxy; C₁-C₁₇ alkylsulfonyl; C₂-C₁₇ alkoxycarbonyl; hydroxyl; hydroxycarbonyl; R²⁸C(=O)O; R²⁸OC(=O)O; R²⁸R⁴⁰NC(=O)O; R⁴⁰R³⁹N; (C₁-C₄ alkoxy)₂P(=E)O; R¹¹SO₃; R⁴⁰R¹⁴R³⁸N⁺; phenyl, phenoxy, phenylthio, phenylsulfonyl, phenylsulfinyl, pyridyl or pyridyloxy each optionally substituted with R³⁰; thienyl, pyrimidinyl, furanyl, naphthalenyl, pyrimidinyloxy, naphthalenyloxy each optionally substituted with R⁷; tetrahydropyranyl; 2-tetrahydropyranyloxy; C₁-C₁₇ alkoxy; 1-3 halogen; C₂-C₁₇ alkoxyalkoxy; C₃-C₁₇ alkynyl; or C₃-C₆ cycloalkyl;
R⁴⁴ is 1-3 halogen; cyano; nitro; C₁-C₆ alkoxy; C₁-C₆ haloalkoxy; C₂-C₆ alkoxyalkoxy; C₁-C₆ alkylthio; C₁-C₆ alkylsulfonyl; phenyl or phenoxy each optionally substituted with R⁵⁷ and R⁵⁹; NR⁴⁹R⁵⁰; or R⁶²;
R⁴⁵ is H; C₁-C₄ alkyl; C₁-C₄ haloalkyl; C₂-C₄ alkenyl; C₂-C₆ haloalkenyl; NR⁵⁴R⁵⁵; or SR⁵⁴;
R⁴⁶ is H; C₁-C₄ alkyl; C₁-C₄ haloalkyl; C₂-C₄ alkenyl; C₂-C₆ haloalkenyl; phenyl optionally substituted with R⁵⁷; NR⁵⁶R⁶⁴; OR⁶⁵; or SR⁶⁵;
R⁴⁷ is C₁-C₈ alkyl optionally substituted with R⁴⁴; C₃-C₆ alkenyl or C₃-C₆ alkynyl each optionally substituted with R⁶⁹; C₃-C₆ cycloalkyl optionally substituted with 1-3 halogen; phenyl optionally substituted with R⁵⁷ and R⁵⁹; C(=O)R⁵²; C(=NR⁵⁵)R⁵²; C(=O)OR⁵³; C[=N(C₁-C₄ alkyl)]OR⁵³; or C(=O)NR⁵³R⁵⁶;
R⁴⁸ is C₁-C₆ alkyl; C₂-C₆ alkenyl; C₂-C₆ haloalkenyl; C₂-C₆ alkynyl; C₂-C₆ alkoxyalkyl; phenyl optionally substituted with R⁵⁸; or phenylmethyl optionally substituted with R⁵⁸ on the phenyl ring;
R⁴⁹ is H; C₁-C₄ alkyl; C₃-C₄ alkenyl; or cyclopropyl;
R⁵⁰ is H; C₁-C₆ alkyl; C₃-C₆ alkenyl; C₃-C₆ alkynyl; C₂-C₆ alkoxyalkyl; C₃-C₆ haloalkenyl; phenyl optionally substituted with R⁵⁸ and R⁵⁹; or phenylmethyl optionally substituted with R⁵⁸ and R⁵⁹ on the phenyl ring; or
R⁴⁹ and R⁵⁰ can be taken together to form -(CH₂)₄-; -(CH₂)₅- or -CH₂CH₂OCH₂CH₂-;
R⁵¹ is 1-3 halogen; C₁-C₆ alkoxy; C₂-C₆ haloalkoxy; C₂-C₆ alkoxyalkoxy; C₁-C₆ alkylthio; C₁-C₆ haloalkylthio; C₃-C₆ alkenyloxy; C₃-C₆ alkynyloxy; C₁-C₆ alkylsulfonyl; C₁-C₆ haloalkylsulfonyl; phenylsulfonyl optionally substituted with R⁵⁷; phenyl or phenoxy each optionally substituted with R⁵⁸ and R⁵⁹; OH; SH; nitro; cyano; O=C=N; S=C=N; NR⁴⁹R⁵⁰; or R⁶²;
R⁵² is H; C₁-C₄ alkyl; C₁-C₄ haloalkyl; C₂-C₄ alkenyl; C₂-C₆ haloalkenyl; or phenyl optionally substituted with R⁵⁷;
R⁵³ is H; C₁-C₆ alkyl; C₃-C₆ alkenyl; C₃-C₆ haloalkenyl; C₃-C₆ alkynyl; C₂-C₆ alkoxyalkyl; phenyl optionally substituted with R⁵⁸ and R⁵⁹; or phenylmethyl optionally substituted with R⁵⁸ and R⁵⁹ on the phenyl ring;
R⁵⁴, R⁵⁵ and R⁵⁶ are each independently H or C₁-C₄ alkyl;
R⁵⁷ is 1-2 halogen; nitro; CF₃; methoxy; methyl; or cyano;
R⁵⁸ is halogen; nitro; CF₃; OCF₃; methoxy; methyl; ethyl; methylthio; cyano; or methoxycarbonyl;
R⁵⁹ is halogen or C₁-C₄ alkyl;
R⁶⁰ is C₁-C₄ alkyl; C₁-C₄ haloalkyl; C₂-C₄ alkenyl; C₂-C₆ haloalkenyl; phenyl optionally substituted with R⁵⁷; or C(=O)R⁶¹;
R⁶¹ is H; C₁-C₄ alkyl; C₁-C₄ haloalkyl; C₂-C₄ alkenyl; C₂-C₆ haloalkenyl; or phenyl optionally substituted with R⁵⁷;
R⁶² is C(=N-V-R⁵³)R⁵²; C(=O)OR⁵³; C(=O)NR⁵³R⁵⁶; C(=NR⁵⁵)OR⁵³; C(=NR⁵⁵)NR⁵³R⁵⁶; OC(=O)R⁵²; SC(=O)R⁵²; N(R⁵⁶)C(=O)R⁵²; OC(=NR⁵⁵)R⁵²; N(R⁵⁶)C(=NR⁵⁵)R⁵²; OC(=O)OR⁵³; OC(=O)NR⁵³R⁵⁶; OC(=S)SR⁵³; SC(=O)OR⁵³; N(R⁵⁶)C(=O)OR⁵³; or N(R⁵⁶)C(=NR⁵⁵)NR⁵³R⁵⁴;
R⁶³ is H; C₁-C₆ alkyl; C₃-C₆ alkenyl; C₃-C₆ alkynyl; C₂-C₆ alkoxyalkyl; C₃-C₆ haloalkenyl; phenyl optionally substituted with R⁵⁸ and R⁵⁹; or phenylmethyl optionally substituted with R⁵⁸ and R⁵⁹ on the phenyl ring; C(=O)R⁵²; C(=NR⁵⁵)R⁵²; C(=O)OR⁵³; C(=O)NR⁵³R⁵⁶; OR⁵³; or SO₂R⁵²;
R⁶⁴ is C₁-C₄ alkyl; C₃-C₆ alkenyl; or phenyl optionally substituted with R⁵⁷ and R⁵⁹;
R⁶⁵ and R⁶⁶ are each independently C₁-C₄ alkyl; C₃-C₄ haloalkyl; C₃-C₆ alkenyl; or phenyl optionally substituted with R⁵⁷ and R⁵⁹;
R⁶⁷ is H; C₁-C₄ alkyl; C₁-C₄ haloalkyl; or C₂-C₄ alkenyl; C₂-C₆ haloalkenyl; phenyl optionally substituted with R⁵⁷; OR⁶⁶; SR⁶⁶; or NR⁵⁴R⁶⁶;
R⁶⁸ is H; C₁-C₄ alkyl; C₁-C₄ haloalkyl; or C₂-C₄ alkenyl; and
R⁶⁹ is 1-3 halogen; cyano; nitro; or C(=O)OR⁵⁴;
provided that the total number of carbons in R², R¹⁶,
R¹⁷ and R¹⁸ is each less than or equal to 20;
and all geometric isomers, enantiomers and mixtures thereof, including racemic mixtures.

2. A compound of Claim 1 wherein:
R¹ is C₁-C₄ alkyl; C₁-C₄ haloalkyl; C₃-C₄ cycloalkyl; or C₂-C₄ alkenyl;
R²¹ is C₁-C₄ alkyl optionally substituted with R⁵¹; C₂-C₄ alkenyl, C₂-C₈ alkynyl, or cyclopropyl each optionally substituted with 1-3 halogen; C(=N-V-R⁵³)H; C(=N-V-R⁵³)(C₁-C₄ alkyl); C(=O)OR⁵³; C(=O)SR⁵³; C(=S)SR⁵³; or C(=O)NR⁵³R⁵⁶;
R²⁹ is C₁-C₄ alkyl optionally substituted with R⁴⁴; C₃-C₆ alkenyl, C₃-C₆ alkynyl, or cyclopropyl each optionally substituted with 1-3 halogen; C(=O)R⁵²; C(=NR⁵⁵)R⁵²; C(=O)OR⁵³; C(=O)NR⁵³R⁵⁶; N=CR⁶⁸R⁶⁷; or SO₂R⁵²;
R⁴⁴ is 1-3 halogen; cyano; nitro; C₁-C₄ alkoxy; C₁-C₄ haloalkoxy; C₂-C₄ alkoxyalkoxy; C₁-C₄ alkylthio; or R⁶²;
R⁴⁵ is H; C₁-C₄ alkyl; C₁-C₄ haloalkyl; C₂-C₄ alkenyl; or C₂-C₄ haloalkenyl;
R⁴⁶ is H; C₁-C₄ alkyl; C₁-C₄ haloalkyl; C₂-C₄ alkenyl; C₂-C₆ haloalkenyl; NR⁵⁶R⁶⁴; OR⁶⁵; or
R⁴⁷ is C₁-C₄ alkyl optionally substituted with R⁴⁴; C₃-C₄ alkenyl or C₃-C₄ alkynyl each optionally substituted with R⁶⁹; C₃-C₆ cycloalkyl optionally substituted with 1-3 halogen; C(=O)R⁵²; C(=O)OR⁵³; or C(=O)NR⁵³R⁵⁶;
R⁴⁸ is C₁-C₄ alkyl; C₂-C₄ alkenyl; C₂-C₄ haloalkenyl; C₂-C₄ alkynyl; or C₂-C₆ alkoxyalkyl;
R⁴⁹ is H; C₁-C₄ alkyl; C₃-C₄ alkenyl; or cyclopropyl;
R⁵⁰ is H; C₁-C₄ alkyl; C₃-C₄ alkenyl; or C₃-C₄ alkynyl; or
R⁴⁹ and R⁵⁰ can be taken together to form -(CH₂)₄-; -(CH₂)₅- or -CH₂CH₂OCH₂CH₂-;
R⁵¹ is 1-3 halogen; C₁-C₄ alkoxy; C₂-C₄ haloalkoxy; C₂-C₄ alkoxyalkoxy; C₁-C₄ alkylthio; C₃-C₄ alkenyloxy; C₃-C₄ alkynyloxy; OH; SH; nitro; cyano; O=C=N; S=C-N; NR⁴⁹R⁵⁰; or R⁶²;
R⁵² is H; C₁-C₄ alkyl; C₁-C₄ haloalkyl; C₂-C₄ alkenyl; or C₂-C₄ haloalkenyl;
R⁵³ is H; C₁-C₄ alkyl; C₃-C₄ alkenyl; or C₃-C₄ haloalkenyl;
R⁶⁰ is C₁-C₄ alkyl; C₁-C₄ haloalkyl; C₂-C₄ alkenyl; C₂-C₄ haloalkenyl; C(=O)(C₁-C₄ alkyl); or C(=O)H;
R⁶² is C(=N-V-R⁵³)R⁵²; C(=O)OR⁵³; C(=O)NR⁵³R⁵⁶; OC(=O)R⁵²; SC(=O)R⁵²; N(R⁵⁶)C(=O)R⁵²; OC(=O)OR⁵³; OC(=O)NR⁵³R⁵⁶; or N(R⁵⁶)C(=O)OR⁵³;
R⁶³ is H; C₁-C₄ alkyl; C₃-C₄ alkenyl; C₃-C₄ alkynyl; C₃-C₄ haloalkenyl; C(=O)R⁵²; C(=NR⁵⁵)R⁵²; C(=O)OR⁵³; C(=O)NR⁵³R⁵⁶; or OR⁵³;
R⁶⁴ is C₁-C₄ alkyl; or C₃-C₄ alkenyl;
R⁶⁵ is C₁-C₄ alkyl; C₃-C₄ haloalkyl; or C₃-C₄ alkenyl; and
R⁶⁷ is H or C₁-C₄ alkyl;
provided that when R³ is a phenyl or heterocyclic ring disubstituted with two alkyl or alkoxy groups, or one alkyl and one alkoxy group, then at least one of the alkyl and alkoxy groups is methyl or methoxy.

3. A compound of Claim 2 wherein:
A is O or NH;
B is halogen; cyano; R²¹; OR²⁹; NR⁴⁹R⁶³; N=CR⁴⁵R⁴⁶; SR⁴⁷; or S(O)₂R⁴⁸;
R¹ is C₁-C₄ alkyl; C₁-C₄ haloalkyl; or vinyl;
R² is C₂-C₂₀ alkyl; C₂-C₂₀ alkoxyalkyl; C₂-C₂₀ haloalkyl; C₃-C₈ alkyl substituted with phenoxy or phenylthio each optionally substituted with R³⁰; C₅-C₇ cycloalkyl; C₂-C₂₀ alkenyl; C₅-C₇ cycloalkenyl; phenyl optionally substituted with R⁵ and R⁷; 2-naphthalenyl; thienyl optionally substituted with R⁵ and R⁷; furyl optionally substituted with R⁷; or pyridyl optionally substituted with R⁵ and R⁷;
provided that when R² is phenyl and R⁵ is other than F, then R⁵ is attached to the para-position relative to the imidazolinone ring;
R³ is phenyl optionally substituted with R¹⁰;
R⁴⁴ is 1-3 halogen; C₁-C₄ alkoxy or C₁-C₄ haloalkoxy;
R⁴⁵ is H or C₁-C₄ alkyl;
R⁴⁶ is H; C₁-C₄ alkyl; OR⁶⁵; or SR⁶⁵;
R⁴⁷ is C₁-C₄ alkyl; C₃-C₄ alkenyl; C(=O)R⁵²; or C(=O)OR⁵⁵;
R⁴⁸ is C₁-C₄ alkyl;
R⁵¹ is 1-3 halogen or C₂-C₃ haloalkoxy;
R⁵² and R⁵³ are each independently H; C₁-C₄ alkyl; or C₃-C₄ alkenyl;
R⁶⁰ and R⁶³ are each independently C₁-C₄ alkyl; and
R⁶⁵ is C₁-C₄ alkyl or C₃-C₄ alkenyl.

4. A compound of Claim 3 wherein:
B is halogen; cyano; C₁-C₄ alkyl, C₁-C₄ alkylthio, or C₁-C₄ alkoxy each optionally substituted with halogen; N=CR⁴⁵R⁴⁶; NR⁴⁹R⁶³; or S(O)₂(C₁-C₄ alkyl);
R¹ is methyl or halomethyl;
R² is C₂-C₁₂ alkyl; C₃-C₈ alkyl substituted with phenoxy optionally substituted with R³⁰; phenyl optionally substituted with R⁵ and R⁷; thienyl optionally substituted with R⁷; or pyridyl optionally substituted with R⁵ and R⁷;
R³ is phenyl optionally substituted with F; Cl; or methyl;
R⁴ is H;
R⁵ is halogen; nitro; C₁-C₆ alkyl; C₁-C₃ haloalkyl; methylthio; C₁-C₆ alkoxy; C₁-C₂ haloalkoxy; C₅-C₆ cycloalkyloxy; phenoxy optionally substituted with R²⁷; phenylthio substituted with R²⁴; phenoxymethyl optionally substituted with R²⁴ on the phenyl ring; benzyloxy optionally substituted with R³⁰ on the phenyl ring; or -OC(=O)R²⁸;
R⁷ and R²⁴ are independently F; C₁-C₂ alkyl; methylthio; or C₁-C₂ alkoxy;
R¹⁶ is C₁-C₆ alkyl;
R¹⁹ is phenyl optionally substituted with R³⁴;
R²⁷ is 1-2 halogen; cyano; C₁-C₄ alkyl; trifluoromethyl; C₁-C₄ alkoxy; C₁-C₄ haloalkoxy; C₁-C₄ alkylthio; C₅-C₆ cycloalkyloxy; or allyl;
R³⁰ is 1-2 halogen; cyano; C₁-C₄ alkyl; trifluoromethyl; C₁-C₄ alkoxy; or trifluoromethoxy;
R³⁴ is 1-2 halogen; nitro; C₁-C₂ alkyl; or C₁-C₂ alkoxy;
R⁴⁶ is H or C₁-C₄ alkyl;
R⁴⁹ and R⁶³ are each independently C₁-C₂ alkyl.

5. A compound of Claim 4 wherein:
B is F; Cl; cyano; C₁-C₂ alkyl; C₁-C₂ alkylthio; C₁-C₂ alkoxy; N=CR⁴⁵R⁴⁶; NMe₂; or S(O)₂(C₁-C₂ alkyl);
R¹ is methyl;
R² is C₁-C₁₂ alkyl; phenyl optionally substituted with R⁵ and R⁷; or thienyl optionally substituted with R⁵ and R⁷; and
R⁵ is F; Cl; Br; C₁-C₆ alkyl; trifluoromethyl; C₁-C₆ alkoxy; trifluoromethoxy; 2,2,2-trifluoroethoxy; C₅-C₆ cycloalkyloxy; methylthio; phenoxy optionally substituted with R²⁷; phenylthio optionally substituted with R²⁴; benzyloxy optionally substituted with R³⁰ on the phenyl ring; or -OC(=O)R²⁸.

6. A compound of Claim 5 which is:
3,5-dihydro-2-methoxy-5-methyl-5-phenyl-3-(phenylamino)-4H-imidazol-4-one; or
3,5-dihydro-5-methyl-2-(methylthio)-5-phenyl-3-(phenylamino)-4H-imidazol-4-one.

7. A fungicidal composition comprising an effective amount of a compound of Formula I as defined in any of claims 1 to 6, and at least one of (a) a surfactant, (b) an organic solvent, and (c) at least one solid or liquid diluent.

8. A fungicidal composition as claimed in Claim 7 further comprising at least one additional fungicidal compound.

9. A composition of Claim 8 wherein the additional fungicidal compound is selected from the group consisting of maneb, cymoxanil, fenpropidine, fenpropimorph, phosethyl-Al, metalaxyl, oxadixyl, tebuconazole, difenoconazole, diniconazole, fluquinoxonazole, ipconazole, metconazole, penconazole, propiconazole, uniconzole, copper oxychioride, furalaxyl, folpet, flusilazol, probenazole, tricyclazole, and flutriafol.

10. A composition of Claim 9 wherein the compound of Formula I is 3,5-dihydro-5-methyl-2-(methylthio)-5-phenyl-3-(phenylamino)-4H-imidazol-4-one and the additional fungicidal compound is cymoxanil.

11. A method for controlling plant diseases comprising applying to the plant or portion thereof to be protected, or to the plant seed or seedling to be protected an effective amount of a compound of Formula I as defined in any of claims 1 to 6 or a composition as defined in any of claims 7 to 10.

12. A process for the preparation of imidazolinones of Formula Ia: 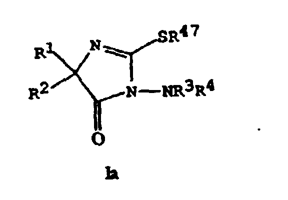
R¹ is C₁-C₄ alkyl; C₁-C₄ haloalkyl; C₃-C₆ cycloalkyl; C₂-C₄ alkenyl; C₂-C₄ alkoxycarbonyl; or phenylmethyl optionally substituted with R⁶ on the phenyl ring and with R⁸ on the benzylic carbon;
R² is C₁-C₂₀ alkyl optionally substituted with R²²; C₂-C₂₀ alkoxyalkyl optionally substituted with R³⁵; C₂-C₂₀ alkenyl optionally substituted with R⁴²; C₂-C₂₀ alkynyl optionally substituted with R⁴¹; (CH₂CH₂OCH₂CH₂)CH-; (CH₂CH₂SCH₂CH₂)CH-; (CH₂CH₂SO₂CH₂CH₂)CH-; C₅-C₇ cycloalkyl; C₅-C₇ cycloalkenyl; phenyl optionally substituted with R⁵ and R⁷; 2-naphthalenyl; thienyl optionally substituted with R⁵ and R⁷; furyl optionally substituted with R⁷; or pyridyl optionally substituted with R⁵ and R⁷; or
R¹ and R² can be taken together to form a structure selected from the group consisting of -CH₂(CH₂)₂CH₂-, -CH₂(CH₂)₃CH₂-, -CH₂(CH₂)₄CH₂-, -CH₂CH₂OCH₂CH₂-, -CH₂CH₂SCH₂CH₂-, 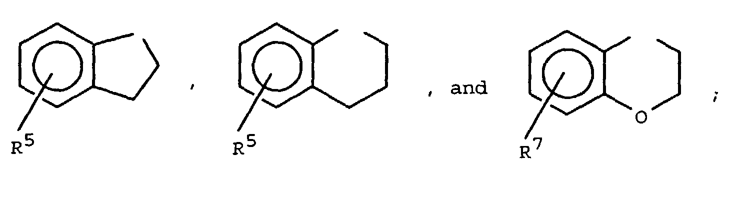
R³ is phenyl, pyridyl, or pyrimidinyl each optionally substituted with R¹⁰; or phenylmethyl;
R⁴ is H or methyl;
R⁴⁷ is C₁-C₈ alkyl optionally substituted with R⁴⁴; C₃-C₆ alkenyl or C₃-C₆ alkynyl each optionally substituted with R⁶⁹; C₃-C₆ cycloalkyl optionally substituted with 1-3 halogen; phenyl optionally substituted with R⁵⁷ and R⁵⁹; C(=O)R⁵²; C(=NR⁵⁵)R⁵²; C(=O)OR⁵³; C[=N(C₁-C₄ alkyl)]OR⁵³; or C(=O)NR⁵³R⁵⁶;
R⁵ is halogen; nitro; cyano; C₁-C₆ alkyl; C₅-C₆ cycloalkyl; C₁-C₆ haloalkyl; C₁-C₆ alkylthio; C₁-C₆ haloalkylthio; C₁-C₆ alkoxy; C₁-C₆ haloalkoxy; C₅-C₆ cycloalkyloxy; C₂-C₆ alkoxyalkyl; C₂-C₆ alkoxyalkoxy; C₃-C₆ alkenyl; C₃-C₆ haloalkenyl; C₃-C₆ alkenyloxy; C₃-C₆ alkynyl; C₃-C₆ haloalkynyl; C₃-C₆ alkynyloxy; C₁-C₆ alkylsulfonyl; C₁-C₆ haloalkylsulfonyl; phenyl or phenylthio each optionally substituted with R²⁴; phenylmethyl, phenoxymethyl, phenethyl, or styryl each optionally substituted with R²⁴ on the phenyl ring; phenoxy optionally substituted with R²⁷; benzyloxy optionally substituted with R³⁰ on the phenyl ring; -OC(=O)NHR²⁸; -C(=O)OR²⁸; or -OC(=O)R²⁸;
R⁶, R⁷, R¹², R¹³, R²⁴ and R²⁶ are independently 1-2 halogen; nitro; C₁-C₄ alkyl; trifluoromethyl; methylthio; or C₁-C₄ alkoxy;
R⁸, R¹⁴, R³⁸ and R⁴⁰ are independently H or C₁-C₄ alkyl;
R¹⁰, and R²⁵ are each independently 1-2 substituents selected from the group consisting of halogen, nitro, cyano, C₁-C₄ alkyl, trifluoromethyl, C₁-C₄ alkylthio, C₁-C₄ alkoxy and trifluoromethoxy;
R¹¹ is independently C₁-C₆ alkyl; or phenyl optionally substituted with R¹²;
R²² is cyano; nitro; C₁-C₁₉ alkylthio; C₁-C₁₉ alkylsulfinyl; C₁-C₁₉ haloalkoxy; C₅-C₆ cycloalkyloxy; C₃-C₁₉ alkenyloxy; C₃-C₁₉ alkynyloxy; C₁-C₁₉ alkylsulfonyl; C₂-C₁₉ alkoxycarbonyl; hydroxyl; hydroxycarbonyl; R²⁸C(=O)O; R²⁸OC(=O)O; R²⁸R⁴⁰NC(=O)O; R³⁹R⁴⁰N; (C₁-C₄ alkoxy)₂P(=E)O; R¹¹SO₃; R⁴⁰R¹⁴R³⁸N⁺; phenyl, phenylthio, phenoxy, phenylsulfonyl, phenylsulfinyl, pyridyl or pyridyloxy each optionally substituted with R³⁰; thienyl, pyrimidinyl, furanyl, naphthalenyl, pyrimidinyloxy, naphthalenyloxy each optionally substituted with R⁷; tetrahydropyranyl; C₃-C₆ cycloalkyl; 2-tetrahydropyranyloxy; or C(=Q)R⁴⁰;
R²⁷ is 1-2 halogen; nitro; cyano; C₁-C₆ alkyl; C₁-C₆ haloalkyl; C₁-C₆ alkoxy; C₁-C₆ haloalkoxy; C₁-C₄ alkylsulfonyl; C₂-C₆ alkoxyalkyl; C₁-C₄ alkylthio; C₅-C₆ cycloalkyl; C₅-C₆ cycloalkyloxy; C₂-C₆ alkenyl; C₂-C₆ haloalkenyl; C₂-C₆ alkynyl; hydroxycarbonyl; C₂-C₄ alkoxycarbonyl; or phenoxy optionally substituted with R²⁴;
R²⁸ is C₁-C₈ alkyl; or phenyl or pyridyl each optionally substituted with R³⁰;
R³⁰ is 1-2 substituents selected from the group consisting of halogen, nitro, cyano, C₁-C₄ alkyl, trifluoromethyl, C₁-C₄ alkoxy and trifluoromethoxy; or phenoxy optionally substituted with R²⁶;
R³⁵ is cyano; nitro; C₁-C₁₇ alkylthio; C₁-C₁₇ alkylsulfinyl; C₁-C₁₇ haloalkoxy; C₅-C₆ cycloalkyloxy; C₂-C₁₇ haloalkenyl; C₃-C₁₇ alkenyloxy; C₃-C₁₇ haloalkynyl; C₃-C₁₇ alkynyloxy; C₁-C₁₇ alkylsulfonyl; C₂-C₁₇ alkoxycarbonyl; hydroxyl; hydroxycarbonyl; R²⁸C(=O)O; R²⁸OC(=O)O; R²⁸R⁴⁰NC(=O)O; R⁴⁰R³⁹N; (C₁-C₄ alkoxy)₂P(=E)O; R¹¹SO₃; R⁴⁰R¹⁴R³⁸N⁺; phenyl, phenoxy, phenylthio, phenylsulfonyl, phenylsulfinyl, pyridyl or pyridyloxy each optionally substituted with R³⁰; thienyl, pyrimidinyl, furanyl, naphthalenyl, pyrimidinyloxy, naphthalenyloxy each optionally substituted with R⁷; tetrahydropyranyl; 2-tetrahydropyranyloxy; C₁-C₁₇ alkoxy; C₂-C₁₇ alkoxyalkoxy; C₃-C₁₇ alkynyl; C₃-C₆ cycloalkyl; or C₂-C₁₇ haloalkoxyalkoxy;
R³⁹ is C₁-C₁₉ alkyl; C₂-C₁₉ alkylcarbonyl; C₂-C₁₉ alkoxycarbonyl; (R⁹R⁴⁰N)C=O; phenyl optionally substituted with R²⁵; or phenoxycarbonyl optionally substituted with R⁷;
R⁴¹ is cyano; nitro; C₁-C₁₇ alkylthio; C₁-C₁₇ alkylsulfinyl; C₁-C₁₇ haloalkoxy; C₅-C₆ cycloalkyloxy; C₃-C₁₇ alkenyloxy; C₃-C₁₇ alkynyloxy; C₁-C₁₇ alkylsulfonyl; C₂-C₁₇ alkoxycarbonyl; hydroxyl; hydroxycarbonyl; R²⁸C(=O)O; R²⁸OC(=O)O; R²⁸R⁴⁰NC(=O)O; R⁴⁰R³⁹N; (C₁-C₄ alkoxy)₂P(=E)O; R¹¹SO₃; R⁴⁰R¹⁴R³⁸N⁺; phenyl, phenoxy, phenylsulfonyl, phenylsulfinyl, pyridyl or pyridyloxy each optionally substituted with R³⁰; thienyl, pyrimidinyl, furanyl, naphthalenyl, pyrimidinyloxy, naphthalenyloxy each optionally substituted with R⁷; tetrahydropyranyl; 2-tetrahydropyranyloxy; C₁-C₁₇ alkoxy; 1-3 halogen; C₂-C₁₇ alkoxyalkoxy; or C₃-C₆ cycloalkyl;
R⁴² is cyano; nitro; C₁-C₁₇ alkylthio; C₁-C₁₇ alkylsulfinyl; C₁-C₁₇ haloalkoxy; C₅-C₆ cycloalkyloxy; C₃-C₁₇ alkenyloxy; C₃-C₁₇ haloalkynyl; C₃-C₁₇, alkynyloxy; C₁-C₁₇ alkylsulfonyl; C₂-C₁₇ alkoxycarbonyl; hydroxyl; hydroxycarbonyl; R²⁸C(=O)O; R²⁸OC(=O)O; R²⁸R⁴⁰NC(=O)O; R⁴⁰R³⁹N; (C₁-C₄ alkoxy)₂P(=E)O; R¹¹SO₃; R⁴⁰R¹⁴R³⁸N⁺; phenyl, phenoxy, phenylthio, phenylsulfonyl, phenylsulfinyl, pyridyl or pyridyloxy each optionally substituted with R³⁰; thienyl, pyrimidinyl, furanyl, naphthalenyl, pyrimidinyloxy, naphthalenyloxy each optionally substituted with R⁷; tetrahydropyranyl; 2-tetrahydropyranyloxy; C₁-C₁₇ alkoxy; 1-3 halogen; C₂-C₁₇ alkoxyalkoxy; C₃-C₁₇ alkynyl; or C₃-C₆ cycloalkyl;
R⁴⁴ is 1-3 halogen; cyano; nitro; C₁-C₆ alkoxy; C₁-C₆ haloalkoxy; C₂-C₆ alkoxyalkoxy; C₁-C₆ alkylthio; C₁-C₆ alkylsulfonyl; phenyl or phenoxy each optionally substituted with R⁵⁷ and R⁵⁹; NR⁴⁹R⁵⁰; or R⁶²;
R⁴⁹ is H; C₁-C₄ alkyl; C₃-C₄ alkenyl; or cyclopropyl;
R⁵⁰ is H; C₁-C₆ alkyl; C₃-C₆ alkenyl; C₃-C₆ alkynyl; C₂-C₆ alkoxyalkyl; C₃-C₆ haloalkenyl; phenyl optionally substituted with R⁵⁸ and R⁵⁹; or phenylmethyl optionally substituted with R⁵⁸ and R⁵⁹ on the phenyl ring; or
R⁴⁹ and R⁵⁰ can be taken together to form -(CH₂)₄-; -(CH₂)₅- or -CH₂CH₂OCH₂CH₂-;
R⁵² is H; C₁-C₄ alkyl; C₁-C₄ haloalkyl; C₂-C₄ alkenyl; C₂-C₆ haloalkenyl; or phenyl optionally substituted with R⁵⁷;
R⁵³ is H; C₁-C₆ alkyl; C₃-C₆ alkenyl; C₃-C₆ haloalkenyl; C₃-C₆ alkynyl; C₂-C₆ alkoxyalkyl; phenyl optionally substituted with R⁵⁸ and R⁵⁹; or phenylmethyl optionally substituted with R⁵⁸ and R⁵⁹ on the phenyl ring;
R⁵⁴, R⁵⁵ and R⁵⁶ are each independently H or C₁-C₄ alkyl;
R⁵⁷ is 1-2 halogen; nitro; CF₃; methoxy; methyl; or cyano;
R⁵⁸ is halogen; nitro; CF₃; OCF₃; methoxy; methyl; ethyl; methylthio; cyano; or methoxycarbonyl;
R⁵⁹ is halogen or C₁-C₄ alkyl;
R⁶² is C(=N-V-R⁵³)R⁵²; C(=O)OR⁵³; C(=O)NR⁵³R⁵⁶; C(=NR⁵⁵)OR⁵³; C(=NR⁵⁵)NR⁵³R⁵⁶; OC(=O)R⁵²; SC(=O)R⁵²; N(R⁵⁶)C(=O)R⁵²; OC(=NR⁵⁵)R⁵²; N(R⁵⁶)C(=NR⁵⁵)R⁵²; OC(=O)OR⁵³; OC(=O)NR⁵³R⁵⁶; OC(=S)SR⁵³; SC(=O)OR⁵³; N(R⁵⁶)C(=O)OR⁵³; or N(R⁵⁶)C(=NR⁵⁵)NR⁵³R⁵⁴;
R⁶⁹ is 1-3 halogen; cyano; nitro; or C(=O)OR⁵⁴;
comprising either
a) reacting an alpha-bis(thio)methyleneamino acid ester of Formula 7 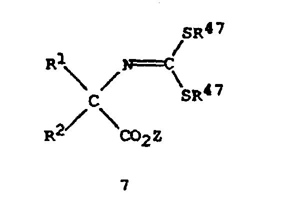 wherein
R¹, R² and R⁴⁷ are as defined above for Formula Ia; and
Z is C₁-C₄ alkyl; C₃-C₄ alkenyl, C₃-C₆ cycloalkyl, or C₆H₅CH₂;
with a hydrazine of Formula 3
NH₂NR³R⁴ 3
wherein R³ and R⁴ are as defined above for Formula Ia; to yield a compound of Formula Ia; or
b) reacting an isothiocyanate of Formula 2 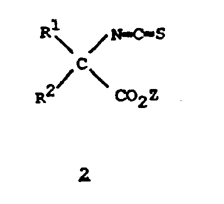 wherein R¹ and R² are as defined above for Formula Ia; and
Z is C₁-C₄ alkyl; C₃-C₄ alkenyl, C₃-C₆ cycloalkyl, or C₆H₅CH₂;
with a hydrazine of Formula 3
NH₂NR³R⁴ 3
wherein R³ and R⁴ are as defined above for Formula Ia;
cyclising the reaction product and
reacting the cyclised product with an electrophilic substrate of Formula 6
R⁴⁷X 6
wherein R⁴⁷ is as defined above for Formula Ia; to yield a compound of Formula Ia.
